# EUROPEAN PATENT SPECIFICATION

(11) **EP 4 658 207 B1**
(45) Date of publication and mention of the grant of the patent: **26.08.2026**
(21) Application number: 24703431.7
(22) Date of filing: 30.01.2024
(51) Int. Cl.: A61F 2/24

(54) **SYSTEMS FOR DELIVERING PROSTHETICS TO TREAT A CARDIAC VALVE**
SYSTEME ZUR FREISETZUNG VON PROTHESEN ZUR BEHANDLUNG EINER HERZKLAPPE
SYSTÈMES DE POSE DE PROTHÈSES POUR TRAITER UNE VALVE CARDIAQUE

(30) Priority: 31.01.2023 US 202363482478 P
(43) Date of publication of application: 10.12.2025
(73) Proprietor: Croivalve Ltd., Dublin 1 D01 YX93 (IE)
(72) Inventor: QUINN, Conor, Dublin, K36V9997 (IE); SOBRINO-SERRANO, Gabriel, Galway (IE); LAUNOIS, Pascal, Dublin, 14 (IE); KENNY, Gavin, Galway (IE)
(74) Representative: Weldon O'Brien Ltd.
(86) International application number: PCT/IB2024/050858
(87) International publication number: WO 2024/161309

(56) References cited:
- WO-A1-2020/197854
- US-A1- 2018 168 803
- US-A1- 2020 297 491
- US-B2- 11 219 525

## Description

### CROSS-REFERENCE TO RELATED APPLICATION

### FIELD OF USE

This technology is directed to apparatus and methods for performing transcatheter or minimally invasive repair of a defective cardiac valve, such as the tricuspid, mitral, pulmonary, and aortic valves.

### BACKGROUND

The human heart has four major valves which moderate and direct blood flow in the cardiovascular system. These valves serve critical functions in assuring a unidirectional flow of an adequate blood supply through the cardiovascular system. The mitral valve and aortic valve control the flow of oxygen-rich blood from the lungs to the body. The mitral valve lies between the left atrium and left ventricle, while the aortic valve is situated between the left ventricle and the aorta. Together, the mitral and aortic valves ensure that oxygen-rich blood received from the lungs is ejected into systemic circulation. The tricuspid and pulmonary valves control the flow of oxygen-depleted blood from the body to the lungs. The tricuspid valve lies between the right atrium and right ventricle, while the pulmonary valve is situated between the right ventricle and the pulmonary artery. Together the tricuspid and pulmonary valves ensure unidirectional flow of oxygen-depleted blood received from the right atrium towards the lungs.

Heart valves are passive structures composed of leaflets that open and close in response to differential pressures on either side of the valve. The aortic, pulmonary, and tricuspid valves have three leaflets, while the mitral valve has only two leaflets. Dysfunction of the cardiac valves is common and can have profound clinical consequences. Regurgitation occurs when the valve leaflets do not meet, or "coapt" correctly, thus causing blood to leak backwards through the valve each time the heart pumps. Failure of the valves to prevent regurgitation leads to an increase in the pressure of blood in the lungs or liver and reduces forward blood flow, causing the heart to pump more blood to compensate for the loss of pressure. Such degradation may result in serious cardiovascular compromise or even death. Valvular dysfunction either results from a defect in the valve leaflet or supporting structure, or dilation of the fibrous ring supporting the valve. These factors lead to poor coaptation of valve leaflets, allowing blood to travel in the wrong direction.

Previously known medical treatments to address diseased valves generally involve either repairing the diseased native valve or replacing the native valve with a mechanical or biological valve prosthesis. Previously-known valve prostheses have some disadvantages, such as the need for long-term maintenance with blood thinners, the risk of clot formation, limited durability, etc. Accordingly, valve repair, when possible, usually is preferable to valve replacement. However, most dysfunctional valves are too diseased to be repaired using previously known methods and apparatus. Accordingly, a need exists for a prosthesis capable of assisting heart valve function that enables treatment of a larger patient population, while reducing the need to fully supplant the native heart valve.

For many years, the standard treatment for such valve dysfunction called for surgical repair or replacement of the valve during open-heart surgery, a procedure conducted under general anesthesia. An incision is made through the patient's sternum (sternotomy), and the heart is accessed and stopped while blood flow is rerouted through a heart-lung bypass machine. When replacing the valve, the native valve is excised and replaced with either a mechanical or biological prosthesis. However, these surgeries are prone to many complications and long hospital stays for recuperation.

More recently, transvascular techniques have been developed for introducing and implanting a replacement valve, using a flexible catheter in a manner less invasive than open-heart surgery. In such techniques, a replacement valve is mounted in a compressed state at the end of a flexible catheter and advanced through the blood vessel of a patient until the prosthetic valve reaches the implantation site. The valve then is expanded to its functional size at the site of the defective native valve, usually by inflating a balloon within where the valve has been mounted. By expanding the prosthetic valve, the native valve leaflets are generally pushed aside and rendered ineffective. Examples of such devices and techniques, wherein the native valve is replaced in its entirety by a substitute tissue valve, are described, for example, in U.S. Patent Nos. 6,582,462 and 6,168,614 to Andersen.

Prostheses have been produced and used for over sixty years to treat cardiac disorders. They have been made from a variety of materials, both biological and artificial. Mechanical or artificial valves generally are made from non-biological materials, such as plastics or metals. Such materials, while durable, are prone to blood clotting and thrombus formation, which in turn increases the risk of embolization and stroke or ischemia. Anticoagulants may be taken to prevent blood clotting that may result in thromboembolic complications and catastrophic heart failure, however, such anti-clotting medication may complicate a patient's health due to the increased risk of hemorrhage.

In contrast, "bio-prosthetic" valves are constructed with prosthetic leaflets made of natural tissue, such as bovine, equine or porcine pericardial tissue, which functions very similarly to the leaflets of the natural human heart valve by imitating the natural action of the heart valve leaflets, coapting between adjacent tissue junctions known as commissures. The main advantage of valves made from tissue is they are not as prone to blood clots and do not absolutely require lifelong systemic anticoagulation.

In recent years, bio-prosthetic valves have been constructed by integrating prosthetic leaflets made from natural tissue into a stent-like supporting frame, which provides a dimensionally stable support structure for the prosthetic leaflets. In more advanced prosthetic heart valve designs, besides providing dimensionally stable support structure for the prosthetic leaflets, the stent-like supporting frame also imparts a certain degree of controlled flexibility, thereby reducing stress on the prosthetic leaflet tissue during valve opening and closure and extending the lifetime of the prosthetic leaflets. In most designs, the stent-like supporting frame is covered with a biocompatible cloth (usually a polyester material such as Dacron^{™} or polytetrafluoroethylene (PTFE)) that provides sewing attachment points for the prosthetic leaflet commissures and prosthetic leaflets themselves. Alternatively, a cloth-covered suture ring may be attached to the stent-like supporting frame, providing a site for sewing the valve structure in position within the patient's heart during a surgical valve replacement procedure.

While iterative improvements have been made on surgical bio-prosthetic valves over the last several decades, existing bio-prosthetic valves still have drawbacks. In most designs, the bio-prosthetic valve is implanted as a replacement for the native valve, filling the entire space the native valve had occupied. One drawback to this procedure is the mismatch in size and mass between opposing surfaces of the stent-like supporting frame. The mismatch is often due to the variability in the shapes and mechanical characteristics of the stent-like supporting frame. For prosthetic valves with balloon-expandable stent-like supporting frames, the recoil of the supporting frames post-balloon-inflation may lead to perivalvular leaks around the circumference of the prosthetic valve and potential slippage and migration of the valve post-implantation. Another risk associated with prosthetic valves having balloon-expandable supporting frames is potential damage to the prosthetic leaflets of the prosthesis during implantation, when the prosthetic leaflets may be compressed between the balloon and the supporting frame. For prosthetic valves with self-expanding stent-like supporting frames, mismatch may arise due to the deformation/movement of the supporting frame, e.g., slight deformation of the frame into a less than circular shape during normal cardiac movement. Such mismatch may lead to instability among components of a prosthetic valve, resulting in perivalvular leaks and uneven stress distribution in the prosthetic leaflets, resulting in accelerated wear of the valve.

Some innovation has addressed these problems by augmenting, rather than replacing, the native valve. The simplest of these devices is a plug suspended across the center of the valve that allows the native leaflets to coapt against the plug body to block regurgitation, as described in U.S. Patent No. 7,854,762 to Speziali. Though the plug design helps to prevent regurgitation, the major drawback is that it also blocks some of the blood flow during diastole. Improved prostheses are described in U.S. Patent Nos. 10,383,729, 10,682,231, 10,987,220 to Quinn, U.S. Patent Nos. 10,952,854, 11,207,182 to Heneghan, and U.S. Patent No. 11,219,525 to Vesely.

Accessing the tricuspid valve with a replacement heart valve via a transfemoral venous access route is a desired option given the familiarity of physicians with this anatomical delivery pathway, and its conventional use in clinical practice. However, from a design and engineering perspective the transfemoral venous access route provides challenges due to some acute bends and angles the heart valve replacement device needs to traverse.

In view of the foregoing drawbacks of previously known systems and methods, there exists a need for improved systems and methods for delivering prosthetics to a defective heart valve.

### SUMMARY

This technology overcomes the drawbacks of previously-known systems and methods by providing systems and methods for implanting a therapeutic heart valve device at a native heart valve of a patient's heart. The system includes a prosthetic device configured to be implanted at the native heart valve, and a support configured to maintain the prosthetic device at the native heart valve. The support includes a first adjustable bend configured to be actuated to adjust a position of the prosthetic device along a first plane, and a second adjustable bend proximal to the first adjustable bend. The second adjustable bend is configured to be actuated to adjust the position of the prosthetic device along a second plane. The support further includes an elongated rail configured to be coupled to the prosthetic device. In addition, the system includes a handle operatively coupled to the support and having one or more actuators configured to be actuated to independently actuate the first adjustable bend and the second adjustable bend. An orientation of the first plane may be dependent on an angle of the second adjustable bend within the second plane. For example, the first and second adjustable bends may be actuated to adjust the position of the prosthetic device along the first and second planes to facilitate navigation of the prosthetic device through the patient's inferior vena cava (IVC) and right atrium to the patient's tricuspid valve.

In some embodiments, the elongated rail may include a first preformed bend disposed at the first adjustable bend. Moreover, the elongated rail may have a first flexibility, and the support further may include a first shaping catheter slidably disposed over the elongated rail such that movement of the first shaping catheter over the first preformed bend of the elongated rail adjusts an angle of the first adjustable bend. For example, the first shaping catheter may have a second flexibility less flexible than the first flexibility. The first shaping catheter may have a distal portion having the second flexibility, and a proximal portion having a third flexibility such that movement of the proximal portion of the first shaping catheter over the second adjustable bend does not adjust an angle of the second adjustable bend.

The elongated rail further may include a second preformed bend disposed at the second adjustable bend. Accordingly, the support further may include a second shaping catheter slidably disposed over the first shaping catheter such that movement of the second shaping catheter over the second preformed bend of the elongated rail adjusts an angle of the second adjustable bend. For example, the second bend shaping catheter may have a third flexibility less flexible than the second flexibility. Alternatively, the support further may include a pull wire catheter comprising a flexible middle portion disposed over the second adjustable bend, a rigid distal portion distal to the flexible middle portion, a rigid proximal portion proximal to the flexible middle portion, and a lumen sized and shaped to slidably receive the first shaping catheter therein, and a pull wire coupled to the rigid distal portion and extending along the flexible middle portion and the rigid proximal portion. The pull wire may be actuated to adjust an angle of the rigid distal portion relative to the rigid proximal portion to thereby adjust an angle of the second adjustable bend. For example, a proximal end of the pull wire may be operatively coupled to the handle, such that the one or more actuators may be actuated to actuate the pull wire, and to disconnect a distal implantation portion of the pull wire from a proximal removable portion of the pull wire.

Alternatively, the support may include a pull wire catheter comprising a flexible distal portion disposed at the first adjustable bend, a rigid distal portion distal to the flexible distal portion, a rigid middle portion proximal to the flexible distal portion, and a lumen sized and shaped to slidably receive the elongated rail therein, and a first pull wire coupled to the rigid distal portion and extending along the flexible distal portion and the rigid middle portion. Accordingly, the first pull wire may be configured to be actuated to adjust an angle of the rigid distal portion relative to the rigid middle portion to thereby adjust an angle of the first adjustable bend. Moreover, the support may include a second pull wire catheter comprising a flexible proximal portion disposed at the second adjustable bend, a second rigid middle portion distal to the flexible proximal portion, a rigid proximal portion proximal to the flexible proximal portion, and a lumen sized and shaped to receive the pull wire catheter therein, and a second pull wire coupled to the second rigid middle portion and extending along the flexible proximal portion and the rigid proximal portion. Accordingly, the second pull wire may be configured to be actuated to adjust an angle of the second rigid middle portion relative to the rigid proximal portion to thereby adjust an angle of the second adjustable bend.

In some embodiments, the pull wire catheter further may comprise a flexible proximal portion disposed at the second adjustable bend proximal to the rigid middle portion, and a rigid proximal portion proximal to the flexible proximal portion, and the system further may comprise a second pull wire coupled to the rigid middle portion and extending along the flexible proximal portion and the rigid proximal portion. Accordingly, the second pull wire may be configured to be actuated to adjust an angle of the rigid middle portion relative to the rigid proximal portion to thereby adjust an angle of the second adjustable bend. Additionally, the pull wire catheter further may comprise a flexible proximal portion disposed at the second adjustable bend proximal to the rigid middle portion, and a rigid proximal portion proximal to the flexible proximal portion, and the system further may comprise a second pull wire coupled to the rigid middle portion and extending along the flexible proximal portion and the rigid proximal portion. Accordingly, the second pull wire may be configured to be actuated to adjust an angle of the rigid middle portion relative to the rigid proximal portion to thereby adjust an angle of the second adjustable bend. The first guide tube may be interrupted or flexible at the flexible distal portion and the flexible proximal portion, and the second guide tube may be interrupted or flexible at the flexible proximal portion.

In addition, the pull wire catheter further may comprise a guide shaft fixedly disposed within the lumen of the pull wire catheter. The guide shaft may comprise a first channel sized and shaped to slidably receive the first pull wire therethrough, a proximal region of the first channel comprising a first plurality of notches, and a second channel sized and shaped to slidably receive the second pull wire therethrough. A proximal region of the second channel may comprise a second plurality of notches, a proximal region of the first pull wire may comprise one or more first bumps configured to engage with one or more notches of the first plurality of notches of the first channel, and a proximal region of the second pull wire may comprise one or more second bumps configured to engage with one or more notches of the second plurality of notches of the second channel. Moreover, a proximal region of the elongated rail may comprise a plurality of rail notches configured to receive at least a portion of the one or more first bumps of the first pull wire and at least a portion of the one or more second bumps of the second pull wire when the one or more first bumps are engaged with the one or more notches of the first plurality of notches and the one or more second bumps are engaged with the one or more notches of the second plurality of notches.

The system further may comprise a stent configured to anchor the support to a blood vessel coupled to the heart, the stent comprising a stent tube having a lumen sized and shaped to slidably receive the support therein. Accordingly, the system further may include a clamping hub coupled to a proximal portion of the pull wire catheter and slidably disposed within the lumen of the stent tube, the clamping hub having a lumen comprising a tapered distal portion and a tapered proximal portion. Additionally, the clamping hub may comprise one or more crush ribs extending radially outward from an outer surface of the clamping hub. The system further may include an anvil slidably disposed within the lumen of the stent tube proximal to the clamping hub, the anvil comprising a tapered distal portion configured to be at least partially received by the tapered proximal portion of the lumen of the clamping hub, and a collet slidably disposed within the lumen of the clamping hub, the collet having a lumen sized and shaped to slidably receive the elongated rail therethrough. In addition, the collet may comprise a tapered distal portion configured to be at least partially received by the tapered distal portion of the lumen of the clamping hub. The system further may include a collet pusher slidably disposed within a lumen of the anvil and at least a portion of the lumen of the clamping hub proximal to the collet, the collet pusher configured to be actuated to move the collet distally relative to the clamping hub, and an anvil pusher slidable disposed within the lumen of the stent tube proximal to the anvil, the anvil pusher configured to be actuated to move the anvil distally relative to the clamping hub. Accordingly, the first and second pull wires may extend between an outer surface of the tapered distal portion of the anvil and an inner surface of the tapered proximal portion of the lumen of the clamping hub, such that upon distal movement of the collet relative to the clamping hub, the tapered distal portion of the lumen of the clamping hub causes the tapered distal portion of the collet to clamp the elongated rail and fix an axial position of the elongated rail relative to the clamping hub. Moreover, upon distal movement of the anvil relative to the clamping hub, the tapered distal portion of the anvil clamps the first and second pull wires between the outer surface of the tapered distal portion of the anvil and the inner surface of the tapered proximal portion of the lumen of the clamping hub, and causes the one or more crush ribs of the clamping hub to expand radially outward and engage the stent tube to thereby fix an axial position of the clamping hub relative to the stent tube.

Alternatively, the system may include a first guide block disposed within the lumen of the pull wire catheter, the first guide block fixedly coupled to the pull wire catheter and comprising a first threaded lumen, a first collar slidably disposed within the lumen of the pull wire catheter distal to the first guide block and coupled to the first pull wire, and a first screw having a first threaded surface, a distal end coupled to the first collar, and a proximal end configured to be releasably coupled to a first torque cable configured to transmit rotary motion to the first screw. At least a portion of the first screw may be disposed within the first threaded lumen of the first guide block. In addition, the system may include a second guide block fixedly coupled to the pull wire catheter proximal to the first guide block, the second guide block comprising a second threaded lumen, a second collar slidably disposed within the lumen of the pull wire catheter distal to second guide block and coupled to the second pull wire, the second collar comprising a wedge extending radially outward from an outer surface of the second collar, and a second screw having a second threaded surface, a distal end coupled to the second collar, and a proximal end configured to be releasably coupled to a second torque cable configured to transmit rotary motion to the second screw. At least a portion of the second screw may be disposed within the second threaded lumen of the second guide block. The system further may include a stent lock tube slidably disposed between the stent tube and the pull wire catheter. The stent lock tube may be configured to be actuated to move distally relative to the pull wire catheter and cover the wedge of the second collar, such that the wedge causes the stent lock tube to expand radially and engage the stent tube to thereby fix an axial position of the pull wire catheter relative to the stent tube.

Moreover, the second collar may comprise a lumen at least partially defined by the wedge, the lumen sized and shaped to receive the elongated rail therethrough. Accordingly, when the stent tube lock covers the wedge, the wedge collapses radially inward against the elongated rail to thereby fix an axial position of the elongated rail relative to the pull wire catheter and the stent tube. Additionally, the second collar may comprise a lumen at least partially defined by the wedge, the lumen sized and shaped to receive the elongated rail therethrough. Accordingly, when the stent tube lock covers the wedge, the wedge collapses radially inward against the elongated rail to thereby fix an axial position of the elongated rail relative to the pull wire catheter and the stent tube. In addition, the proximal end of the second screw may comprise a second screw connection configured to releasably interlock with a second torque cable connection at a distal end of the second torque cable, the second screw connection and the second torque cable connection configured to remain coupled via a rod disposed through the second screw connection and the second torque cable connection when the second screw connection and the second torque cable connection are interlocked. The rod may be configured to be removed from at least the second screw connection to permit decoupling of the second screw connection and the second torque cable connection. Moreover, the elongated rail may be biased towards a straight, linear configuration, and the elongated rail may comprise a stiffness sufficient to cause the elongated rail to straighten upon release of tension by at least one of the first or second pull wires.

In some embodiments, the elongated rail may have variable flexibility along its length. For example, at least a portion of the elongated rail may have a first cross-sectional area, and at least a portion of the first preformed bend may have a second cross-sectional area smaller than the first cross-sectional area, thereby increasing flexibility at the first preformed bend. In addition, the elongated rail may have a second preformed bend disposed at the second adjustable bend, and at least a portion of the elongated rail comprises a first cross-sectional area, and at least a portion of the second preformed bend comprises a second cross-sectional area smaller than the first cross-sectional area, thereby increasing flexibility at the second preformed bend. Additionally or alternatively, at least a portion of the elongated rail may have a first cross-sectional area, and at least a portion of the elongated rail distal to the first preformed bend may have a second cross-sectional area smaller than the first cross-sectional area, thereby increasing flexibility at the portion of the elongated rail distal to the first preformed bend.

Moreover, at least a portion of the elongated rail may have a circular cross-sectional area having a first flexibility, and at least another portion of the elongated rail may have a non-circular cross-sectional area having a second flexibility more flexible than the first flexibility. For example, the first adjustable bend may have the at least another portion of the elongated rail comprising the non-circular cross-sectional area, such that rigidity of the elongated rail is maintained in a plane perpendicular to the first plane, while flexibility of the elongated rail is increased along the first plane. Additionally or alternatively, the second adjustable bend may have the at least another portion of the elongated rail comprising the non-circular cross-sectional area, such that rigidity of the elongated rail is maintained in a plane perpendicular to the second plane, while flexibility of the elongated rail is increased along the second plane.

The system further may include a stent configured to anchor the support to a blood vessel coupled to the heart. For example, the stent may include a stent tube having a lumen sized and shaped to slidably receive the support therein. In addition, the stent tube may have one or more flexible leaves configured to transition between a radially collapsed state and a radially expanded state, such that the one or more flexible leaves may be biased toward the radially collapsed state. The system further may include a disconnect catheter having a tab configured to be actuated to move from a first position where the tab engages with and maintains the one or more flexible leaves in the radially expanded state and a second position where the tab disengages with the one or more flexible leaves to permit the one or more flexible leaves to transition to the radially collapsed state to clamp the support. The stent tube further may include a retainer configured to maintain the tab in the first position. Moreover, a portion of the one or more flexible leaves that contact the support in the radially collapsed state may have teeth configured to increase friction between the one or more flexible leaves and the support. A base of the one or more flexible leaves may extend from the stent tube via a hinged portion. In addition, the disconnect catheter may have a lumen sized and shaped to slidably receive at least a portion of the stent tube therein. In addition, the support further may include a body support catheter having a distal end coupled to the prosthetic device, a proximal end operatively coupled to the handle, and a lumen configured to be slidably receive the elongated rail. The body support catheter may be actuated via the handle to move relative to the elongated rail to adjust an axial position of the prosthetic valve relative to the support.

### BRIEF DESCRIPTION OF THE DRAWINGS

FIG. 1 is a perspective view of an exemplary heart valve therapeutic device having a double bend for repairing a defective heart valve constructed in accordance with the principles of the present disclosure.
FIGS. 2A to 2C illustrate the implantable distal end of the heart valve therapeutic device of FIG. 1.
FIG. 2D illustrates an exemplary alternative elongated rail in accordance with the principles of the present disclosure.
FIG. 2E illustrates an exemplary alternative actuation mechanism of a double bend of a heart valve therapeutic device in accordance with the principles of the present disclosure.
FIG. 2F illustrates an exemplary alternative anchor constructed in accordance with the principles of the present disclosure.
FIGS. 3A and 3B illustrate the distal end of the heart valve therapeutic device of FIG. 1 implanted within the patient in accordance with the principles of the present disclosure.
FIGS. 4A to 4C illustrate an alternative exemplary stent configuration providing the second bend of the heart valve therapeutic device in accordance with the principles of the present disclosure.
FIGS. 5A and 5B illustrate the adjustment of the second bend of the heart valve therapeutic device using the stent configuration of FIGS. 4A to 4C.
FIGS. 6A to 6C illustrate the pivot point of the stent configuration of FIGS. 4A to 4C.
FIGS. 7A to 7C illustrate an exemplary collar of the stent configuration of FIGS. 6A to 6C constructed in accordance with the principles of the present disclosure.
FIGS. 8A and 8B illustrate an alternative exemplary collar of the stent configuration of FIGS. 6A to 6C constructed in accordance with the principles of the present disclosure.
FIGS. 9A to 9D illustrate an exemplary handle of the heart valve therapeutic device of FIG. 1 constructed in accordance with the principles of the present disclosure.
FIGS. 10A to 10D illustrate positional adjustment of the distal end of the heart valve therapeutic device using the handle of FIGS. 9A to 9D in accordance with the principles of the present disclosure.
FIGS. 11A and 11B illustrate locking of the distal end of the heart valve therapeutic device using the handle of FIGS. 9A to 9D in accordance with the principles of the present disclosure.
FIGS. 12A and 12B illustrate detachment of the distal end of the body support catheter of the heart valve therapeutic device using the handle of FIGS. 9A to 9D in accordance with the principles of the present disclosure.
FIGS. 13A to 13C illustrate detachment of the distal end of the elongated rail of the heart valve therapeutic device using the handle of FIGS. 9A to 9D in accordance with the principles of the present disclosure.
FIG. 14 illustrates an exemplary connection between a body support catheter and a valve spine constructed in accordance with the principles of the present disclosure.
FIGS. 15A and 15B illustrate an alternative exemplary connection between a body support catheter and a valve spine constructed in accordance with the principles of the present disclosure.
FIGS. 16A and 16B illustrate coupling of an anchor spine to a shaping catheter in accordance with the principles of the present disclosure.
FIG. 16C illustrates an alternative coupling of an anchor spine to a shaping catheter in accordance with the principles of the present disclosure.
FIGS. 17A and 17B illustrate an exemplary mechanism for adjusting the position of the one or more adjustable bends of the heart valve therapeutic device constructed in accordance with the principles of the present disclosure.
FIG. 17C illustrates an alternative exemplary for adjusting the position of the one or more adjustable bends of the heart valve therapeutic device constructed in accordance with the principles of the present disclosure.
FIGS. 18A to 18C illustrate an exemplary mechanism for limiting adjustment of the axial location of the one or more adjustable bends of the heart valve therapeutic device constructed in accordance with the principles of the present disclosure.
FIG. 19 illustrates an alternative exemplary mechanism for limiting adjustment of the axial location of the one or more adjustable bends of the heart valve therapeutic device constructed in accordance with the principles of the present disclosure.
FIG. 20 illustrates another alternative exemplary mechanism for limiting adjustment of the axial location of the one or more adjustable bends of the heart valve therapeutic device constructed in accordance with the principles of the present disclosure.
FIGS. 21A to 21C illustrate an exemplary actuator interlocking mechanism constructed in accordance with the principles of the present disclosure.
FIGS. 22A to 22F illustrate an exemplary pull wire actuation mechanism of a double bend of a heart valve therapeutic device in accordance with the principles of the present disclosure.
FIGS. 23A to 23F illustrate an alternative exemplary pull wire actuation mechanism of a double bend of a heart valve therapeutic device in accordance with the principles of the present disclosure.
FIG. 24 illustrates an exemplary depth and pull wire locking mechanism of a double bend of a heart valve therapeutic device in accordance with the principles of the present disclosure.
FIGS. 25A to 25F illustrate yet another alternative exemplary pull wire actuation mechanism of a double bend of a heart valve therapeutic device in accordance with the principles of the present disclosure.
FIGS. 26A to 26C illustrate an exemplary torque cable attachment mechanism in accordance with the principles of the present disclosure.

### DETAILED DESCRIPTION

Embodiments of the present invention are directed to exemplary systems for reducing cardiac valve regurgitation. Provided herein is a prosthetic device that may contain a prosthetic coaptation body to be positioned at a native cardiac valve. The prosthetic device may be suspended across the native heart valve by a support. For example, the support may be coupled to the prosthetic coaptation body and extend out of the heart into an adjacent blood vessel coupled to the heart (e.g., superior vena cava, inferior vena cava). The support may be coupled to the blood vessel with an anchor that preferably is expandable and has a stent structure. In some examples, the support is structured to suspend the prosthetic coaptation body in the native valve in a free-standing manner without anchoring to cardiac tissue, thereby minimizing damage to the heart. The prosthetic coaptation body may be formed from a frame (e.g., metal frame such as Nitinol) that is at least partially covered by a skirt made from biocompatible material, and also includes prosthetic leaflets. The frame, biocompatible material, and prosthetic leaflets may together form a conduit through which blood flows when the prosthetic leaflets open during the cardiac cycle.

The design of the prosthetic device improves coaptation with the native heart valve leaflets and allows for a more reliable delivery. The prosthetic device may be implanted percutaneously via a blood vessel, e.g., the jugular vein, femoral vein, femoral artery, for the treatment of a defective cardiac valve, e.g., tricuspid, mitral, pulmonary, or aortic valve. In one example, the prosthetic device may be used to treat symptomatic primary or functional (secondary) tricuspid regurgitation. For example, the prosthetic device may be positioned between the native tricuspid valve leaflets to restore the valve function without altering the native anatomy or obstructing flow during diastole and held in place by an anchor system deployed in an anchor site, e.g., within the heart and/or within a blood vessel coupled to the heart such as the superior vena cava (SVC) or the inferior vena cava (IVC). Accordingly, the support may include one or more adjustable bends that may be actuated, e.g., via a handle operatively coupled to the proximal end of the support, to facilitate navigation through the patient's anatomical structures to reach the target implantation site.

The frame may be designed with predefined kink points or collapsible / expandable features to allow the conduit to be compressed into a delivery sheath without being damaged, and to more reliably expand upon delivery. The frame may have a proximal ring and a distal ring, as well as an inner ring coupled to the proximal ring via a plurality of skirt anchors to which the prosthetic valve leaflets may be attached. One or more of the rings may exhibit a scallop, sinusoidal, zig-zag shape or otherwise oscillating pattern in the expanded state to further improve the compression and expansion of the frame. The skirt of the prosthetic coaptation body may join the proximal ring to the distal ring to improve coaptation of the native valve against the skirt. The prosthetic coaptation body may be coupled to the support by a plurality of tethers that may be formed of shape-memory material such as Nitinol. The tethers may be rigid or stiff and hold the prosthetic coaptation body in position more accurately than tensile wires.

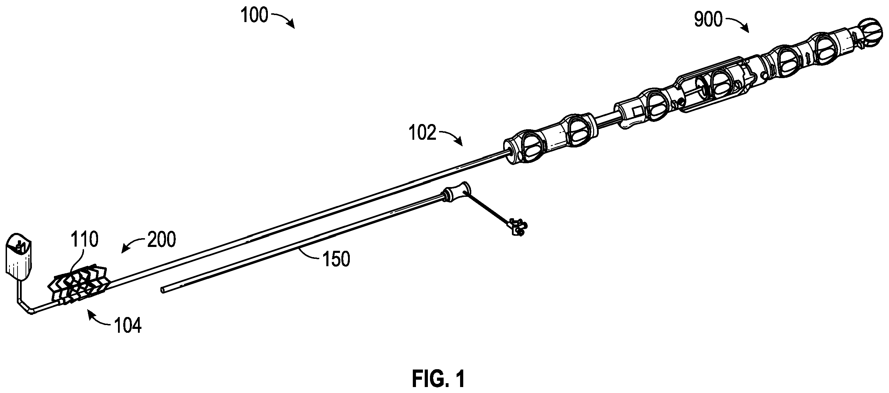

Referring to FIG. 1, an illustrative embodiment of exemplary heart valve therapeutic device 100 in accordance with the principles of the present disclosure is described. Illustratively, heart valve therapeutic device 100 is designed for repairing a defective tricuspid valve. As will be understood by a person having ordinary skill in the art, heart valve therapeutic device 100 may be readily adapted for other cardiac valves such as the mitral valve, aortic valve, or pulmonary valve.

As shown in FIG. 1, heart valve therapeutic device 100 may include prosthetic device 120 coupled to support 200 at distal region 104 of heart valve therapeutic device 100, as well as actuator 900 at proximal region 102 of heart valve therapeutic device 100. Preferably, prosthetic device 120, e.g., a prosthetic coaptation body, works together with the native leaflets to both provide a surface for the native leaflets to coapt and to provide a prosthetic valve in a conduit formed by prosthetic device 120. Unlike prior prosthetic valves that do not use the native leaflets (e.g., because they are cut away or pushed aside by the implant), prosthetic device 120 may use both the native leaflets and the prosthetic leaflets in the same native heart valve, thereby creating a "double-valve" configuration in the single heart valve. Actuator 900 may be a handle having a plurality of interfaces, e.g., knobs and switches, configured to be manipulated by a clinician to deliver the system for implantation.

Support 200 further may include anchor 110, e.g., a stent. Anchor 110 may be formed of a stent structure and is preferably collapsible in a contracted, delivery state and expandable to an expanded, deployed state to anchor prosthetic device 120 at the native cardiac valve. For example, anchor 110 may contact the inner wall of a blood vessel (e.g., the SVC or IVC) to anchor support 200 intraluminally, thereby anchoring prosthetic device 120 in a free-standing, suspended manner in the native heart valve. In addition, heart valve therapeutic device 100 may include introduction sheath 150 for facilitating delivery of prosthetic device 120, support 200, and anchor 110.

Anchor 110, prosthetic device 120, and support 200 may be constructed as described in U.S. Patent No. 11,219,525 to Vesely. For example, support 200 may include a proximal, delivery portion detachably coupled to a distal, implantable portion, such that anchor 110 and prosthetic device 120 are disposed on the distal, implantable portion of support 200. During delivery, the distal, implantable portion of support 200 is coupled to the proximal, delivery portion of support 200, which is operatively coupled to actuator 900. Actuator 900 may be held and manipulated by a clinician to deliver anchor 110 and prosthetic device 120 to the target implantation site across the native valve, and to detach the distal, implantable portion of support 200 from the proximal, delivery portion of support 200, such that the distal, implantable portion of support 200 including anchor 110 and prosthetic device 120 remains implanted within the patient.

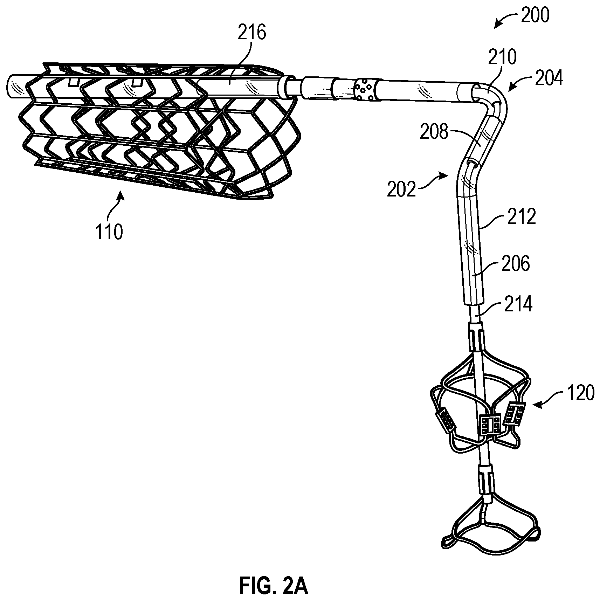

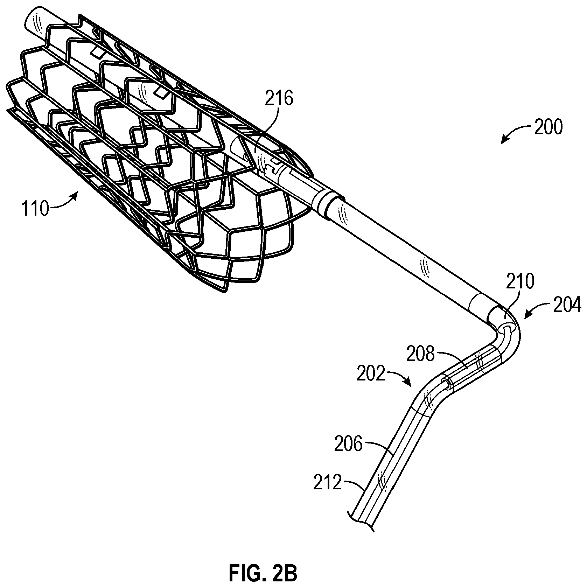

Referring now to FIGS. 2A to 2C, the distal, implantable portion of support 200 is described in further detail. As shown in FIGS. 2A and 2B, support 200 may include elongated rail 206 extending from actuator 900 to prosthetic device 120. For example, the distal end of elongated rail 206 may extend concentrically through prosthetic device 120, such that prosthetic device 120 may be slidably movable along elongated rail 206 until prosthetic device 120 is positioned at a desired location along elongated rail 206. Further, prosthetic device 120 may be locked relative to elongated railed 206 at the desired position such that prosthetic device 120 is coupled to elongated rail 206.

As shown in FIGS. 2A and 2B, elongated rail 206 may have one or more preformed bends, e.g., first preformed bend 202 and second preformed bend 204, to improve positioning of prosthetic device 120 across the native valve, as described in further detail below. For example, the bends may be predefined for a specific patient anatomy. Moreover, the preformed bends permits steering of support 200 from the predefined shape; this may have the effect of reducing stresses and strain on elongated rail 206 for long-term implant. In addition, the preformed bends may reduce the stress required to position prosthetic device 120 during delivery. For example, elongated rail 206 may be an elongated shaft made of metal (e.g., Nitinol) that is preformed in one or more areas along the length of elongated rail 206 to a predetermined angle (e.g., 50-150 degree bend, 100 degree bend). Moreover, heart valve therapeutic device 100 may include one or more radiopaque markers for *in-vivo* visualization during delivery of prosthetic device 120.

In addition, support 200 may include first shaping catheter 208 slidably disposed over and coaxial to elongated rail 206. The angle of first preformed bend 202 of elongated rail 206 may be based on the relative axial position between first shaping catheter 208 and elongated rail 206 responsive to actuation at handle 900. For example, as first shaping catheter 208 is advanced distally over first preformed bend 202 of elongated rail 206, the first preformed bend 202 of elongated rail 206 straightens, and as first shaping catheter 208 is retracted proximally relative to first preformed bend 202 of elongated rail 206, the elongated rail returns to its natural state with first preformed bend 202. Thus, first shaping catheter 208 may have a stiffness greater than first preformed bend 202, and accordingly, first preformed bend 202 has a flexibility that is more flexible than the flexibility of first shaping catheter 208, such that advancement of first shaping catheter 208 over first preformed bend 202 causes first preformed bend 202 of elongated rail 206 to straighten. As will be understood by a person having ordinary skill in the art, elongated rail 206 may be moved while first shaping catheter 208 remains stationary within the patient to bend and straighten elongated rail 206.

As second preformed bend 204 is proximal to first preformed bend 202, the section of first shaping catheter 208 that slides over second preformed bend 204 may have a stiffness less than second preformed bend 204, such that first shaping catheter 208 may slide over second preformed bend 204 without changing the position and angle of second preformed bend 204, whereas movement of first shaping catheter 208 over first preformed bend 202 adjusts the position and angle of first preformed bend 202 as described above.

First shaping catheter 208 may be formed of a braided, coiled, multi-durometer polymer. Alternatively, first shaping catheter 208 may be 3D printed or include a laser cut shaft. Preferably, the distal portion of first shaping catheter 208, e.g., adjacent to prosthetic device 120, may have greater flexibility along its length than the proximal portion of first shaping catheter 208, e.g., adjacent to anchor 110. Accordingly, movement of the distal portion of first shaping catheter 208 over first preformed bend 202 of elongated rail 206 may not adjust the angle of the preformed bend, whereas movement of the proximal portion of first shaping catheter 208 over first preformed bend 202 adjusts the angle of the preformed bend, thereby minimizing damage to support 200.

In addition, support 200 may include second shaping catheter 210 slidably disposed over and coaxial to first shaping catheter 208, and accordingly, to elongated rail 206. The angle of second preformed bend 204 of elongated rail 206 may be based on the relative axial position between second shaping catheter 210 and elongated rail 206 responsive to actuation at handle 900. For example, as second shaping catheter 210 is advanced distally over second preformed bend 204 of elongated rail 206, the elongated rail straightens, and as second shaping catheter 210 is retracted proximally relative to second preformed bend 204 of elongated rail 206, the elongated rail returns to its natural state with second preformed bend 204. Thus, second shaping catheter 210 may have a stiffness greater than second preformed bend 204, and accordingly, second preformed bend 204 has a flexibility that is more flexible than the flexibility of second shaping catheter 210, such that advancement of second shaping catheter 210 over second preformed bend 204 causes elongated rail 206 to straighten. As will be understood by a person having ordinary skill in the art, elongated rail 206 may be moved while second shaping catheter 210 remains stationary within the patient to bend and straighten elongated rail 206.

Like first shaping catheter 208, second shaping catheter 210 may be formed of a braided, coiled, multi-durometer polymer. Alternatively, second shaping catheter 210 may be 3D printed or include a laser cut shaft. Preferably, the distal portion of second shaping catheter 210 may have greater flexibility along its length than the proximal portion of second shaping catheter 210, e.g., adjacent to anchor 110. Accordingly, movement of the distal portion of second shaping catheter 210 over second preformed bend 204 of elongated rail 206 may not adjust the angle of the preformed bend, whereas movement of the proximal portion of second shaping catheter 210 over second preformed bend 204 adjusts the angle of the preformed bend, thereby minimizing damage to support 200. As will be understood by a person having ordinary skill in the art, elongated rail 206 may have more than two preformed bends along its length, and accordingly, support 200 may include additional shaping catheters slidably disposed over and coaxial to elongated rail 206 to adjust the position and angle of the respective preformed bends.

In addition, support 200 may include body support catheter 212 slidably disposed over and coaxial to second shaping catheter 210, and accordingly to first shaping catheter 208 and elongated rail 206. Body support catheter 212 may be formed of a braided, coiled, multi-durometer polymer. Alternatively, body support catheter 212 may be 3D printed or include a laser cut shaft. Body support catheter 212 may have high flexibility in at least the regions of first preformed bend 202 and second preformed bend 204 so as to not adjust, or only minimally adjust, the position and/or angle of first preformed bend 202 and second preformed bend 204. Preferably, body support catheter 212 does not adjust the position and/or angle of first preformed bend 202 and second preformed bend 204 as body support catheter 212 moves over first preformed bend 202 and second preformed bend 204; however, minimal adjustment thereof may occur and is acceptable. The distal end of body support catheter 212 may be coupled to prosthetic device 120, e.g., at connector 214, such that elongated rail 206 may extend concentrically through connector 214. Accordingly, body support catheter 212 may be used to deliver and adjust and finally stabilize the position, e.g., depth, of prosthetic device 120 across the native cardiac valve. In some embodiments, body support catheter 212 may be disposed directly over elongated rail within the lumen of first shaping catheter 208, or alternatively, between first shaping catheter 208 and second shaping catheter 210, to thereby adjust the depth of prosthetic device 120 across the native cardiac valve.

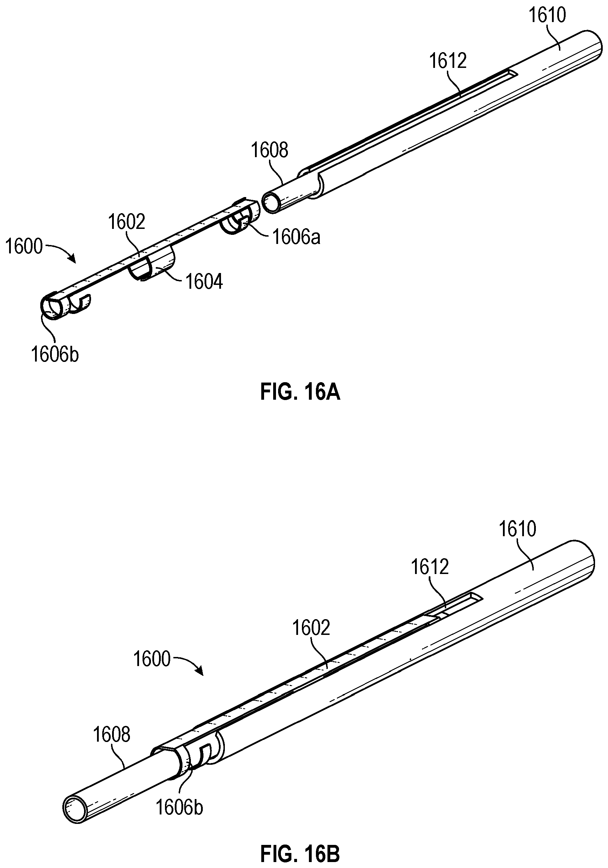

Moreover, support 200 may include anchor tube 216 coupled to anchor 110. For example, anchor tube 216 may include a first distal portion rigidly coupled to a first distal portion of anchor 110, and a second proximal portion rigidly coupled to anchor 110, such that anchor tube 216 does not move relative to anchor 110, e.g., through multiple cardiac cycles. In some embodiments, only one portion of anchor tube 216 is rigidly coupled to anchor 110 to thereby prevent relative movement between anchor tube 216 and anchor 110 through multiple cardiac cycles. Anchor tube 216 may be slidably disposed over and coaxial to body support catheter 212, and accordingly to second shaping catheter 210, first shaping catheter 208, and elongated rail 206. Anchor tube 216 permits telescoping of and anchoring of the catheter components of support 200, e.g., body support catheter 212, second shaping catheter 210, first shaping catheter 208, and elongated rail 206. Alternatively, in some embodiments, the anchor may be directly coupled to the body support catheter without the need for an anchor tube, as described in further detail below with regard to FIGS. 16A and 16B.

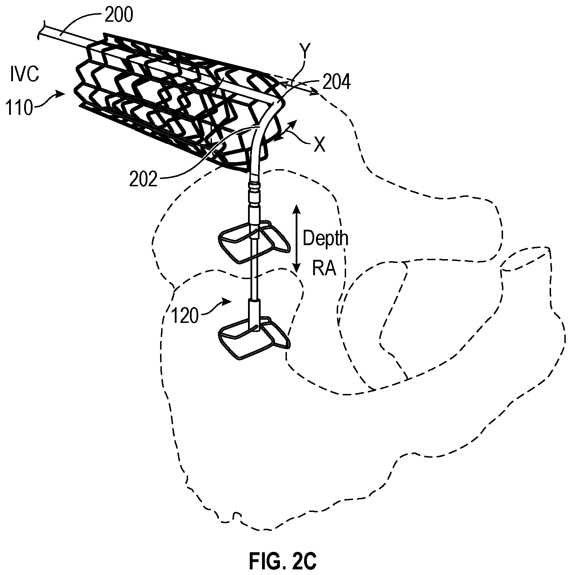

As shown in FIG. 2C, when anchor 110 is positioned within the IVC, support 200 may suspend prosthetic device 120 through the right atrium RA and across the native cardiac valve. Body support catheter 212 may be actuated via handle 900 to adjust the "depth" of prosthetic device 120 relative to the native cardiac valve. As described above, first shaping catheter 208 may be actuated via handle 900 to adjust the position of prosthetic device 120 by adjusting the angle of first preformed bend 202, such that the position of prosthetic device 120 is adjustable within a first plane along the "X" direction. Second shaping catheter 210 may be actuated via handle 900 to adjust the position of prosthetic device 120 by adjusting the angle of second preformed bend 204, such that the position of prosthetic device 120 is adjustable within a second plane along the "Y" direction. Accordingly, the orientation of the first plane formed along the "X" direction will be dependent on the angle of second preformed bend 204 within the second plane formed along the "Y" axis.

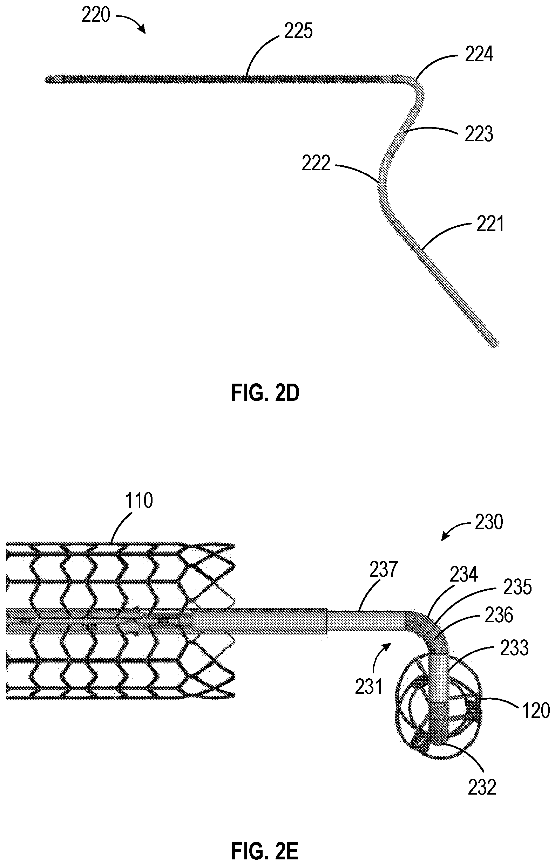

Referring now to FIG. 2D, an alternative exemplary elongated rail is provided. Like elongated rail 206, elongated rail 220 may have one or more preformed bends, e.g., first preformed bend 222 and second preformed bend 224, to improve positioning of prosthetic device 120 across the native valve. For example, elongated rail 220 may be an elongated shaft made of a super elastic metal (e.g., Nitinol) that is pre-formed in one or more areas along the length of elongated rail 220 to a predetermined angle (e.g., 50-150 degree bend, 100 degree bend). Elongated rail 220 is distinct from elongated rail 206 in that elongated rail 220 may have a variable thickness, and accordingly variable cross-sectional area, along its length between distal portion 221 and proximal portion 225 of elongated rail 220 to improve functionality and facilitate self-centering of prosthetic device 120. The variable thickness of elongated rail 220 along its length may be selected to increase the flexibility of predetermined regions along elongated rail 220 for improved angle placement control of the one or more preformed bends. For example, the portions of elongated rail 220 forming first preformed bend 222 and/or second preformed bend 224 may have a reduced cross-sectional area compared with the other portions of elongated rail 220, e.g., proximal portion 225 and middle portion 223 extending between first preformed bend 222 and second preformed bend 224, to thereby increase the bend flexibility of elongated rail 220 along first preformed bend 222 and/or second preformed bend 224. Accordingly, the increased flexibility at the one or more preformed bends may facilitate adjustments of the angle of the respective preformed bend when positioning prosthetic device 120.

Additionally or alternatively, at least a portion of distal portion 221 of elongated rail 220 also may have a reduced cross-section area compared with the other portions of elongated rail 220, e.g., proximal portion 225 and middle portion 223, to thereby increase flexibility at the distal end of elongated rail 220 and facilitate self-centering of prosthetic device 120. In addition, the increased flexibility of the portion of distal portion 221 distal to first preformed bend 222 may permit improved overall flexibility of elongated rail 220 over 360 degrees, e.g., during centering of prosthetic valve 120 across the native valve. In some embodiments, elongated rail 220 may have a circular shaped cross-sectional area along its entire length such that the portions having reduced/variable cross-sectional areas also have a circular shaped cross-sectional areas. Alternatively, the portions having reduced/variable cross-sectional areas may have a non-circular cross-sectional area, which may, e.g., permit rigidity of elongated rail 220 to be maintained in the plane perpendicular to the bend plane of the respective preformed bend, while providing increased flexibility of elongated rail 220 along the respective bend plane.

Referring now to FIG. 2E, an alternative actuation mechanism for the double bend of the heart valve therapeutic devices described herein is provided. As shown in FIG. 2E, like support 200, the elongated rail of support 230 may have one or more preformed bends, e.g., first preformed bend 232 and second preformed bend 234 proximal to first preformed bend 232, to facilitate positioning of prosthetic device 120 at the target location across the native valve. First preformed bend 232 may be actuated, e.g., via a handle operatively coupled to support 230, to adjust the bend angle of first preformed bend 232 along a first bend plane, and second preformed bend 234 may be actuated, e.g., via the handle, to adjust the bend angle of second preformed bend 234 along a second bend plane. For example, first preformed bend 232 may be actuated via the actuation mechanism described above, e.g., via relative movement between a shaping catheter and first preformed bend 232 of the elongated rail. For example, a shaping catheter having a higher stiffness than the elongated rail along first preformed bend 232 may be actuated, e.g., via the handle, to move relative to first preformed bend 232 of the elongated rail slidably disposed therein to adjust the angle of first preformed bend 232 along the first bend plane, such that the bend angle of first preformed bend corresponds to the amount of overlap between the shaping catheter and first preformed bend 232.

Unlike support 200, second preformed bend 234 of support 230 may be actuated, e.g., via the handle, via a pull wire mechanism. For example, as shown in FIG. 2E, support 230 may include pull wire catheter 231 having rigid distal region 233, flexible middle region 235, and rigid proximal region 237, and a lumen extending therethrough sized and shaped to slidably receive the shaping catheter therein. Flexible middle region 235 may be a lasercut section and may be disposed over second preformed bend 234 such that distal region 233 is disposed distal to second preformed bend 234 and proximal region 237 is disposed proximal to second preformed bend 234. Alternatively, flexible middle region 235 may be formed of a braided section between distal region 233 and proximal region 237. Moreover, support 230 may include pull wire 236 having a distal end coupled to distal region 233, e.g., via laser welding, and extending proximally along middle region 235 and proximal region 237, such that a proximal end of pull wire catheter 231 may be operatively coupled to the handle. For example, pull wire 236 may extend along an outer surface of middle portion 235 and an outer surface of proximal region 237 and within an outer catheter, e.g., a reflowed jacket disposed over proximal region 237. Pull wire 236 may be slidably coupled to middle region 235 in a manner to permit adjustment of distal region 233 relative to proximal region 237 upon application of tension to pull wire 236. In some embodiments, pull wire 236 may be slidably disposed within a flexible hypotube extending between pull wire catheter 231 and the jacket disposed over proximal region 237, to thereby facilitate containment of pull wire 236 under tension within the hypotube.

Accordingly, the handle may be actuated to move, e.g., retract or release, pull wire 236 to thereby apply a lever to distal region 233 and selectively adjust the angle of distal region 233 relative to proximal region 237, e.g., by bending flexible middle portion 235 along a bend plane of second preformed bend 234. For example, applying a tension to pull wire 236 may cause distal region 233 to straighten relative to proximal region 237. Moreover, the handle may be actuated to disconnect a distal, implantable portion of pull wire 236, which will remain implanted within the patient along with the implantable distal portion of support 200 as described above, with a proximal, removable portion of pull wire 236, which may be removed from the patient after the proper positioning and implantation of prosthetic device 120. Alternatively, as will be understood by a person having ordinary skill in the art, pull wire 236 may be actuated using other actuation mechanisms. For example, the proximal end of pull wire 236 may be coupled to a catheter slidably disposed over pull wire catheter 231, and operatively coupled to the handle, such that the handle may be actuated to move the catheter, and accordingly pull wire 236, to actuate second preformed bend 234. In some embodiments, the elongated rail may not include a second preformed bend, such that the tension on pull wire 236 alone is sufficient to selectively adjust the angle of distal region 233 relative to proximal region 237.

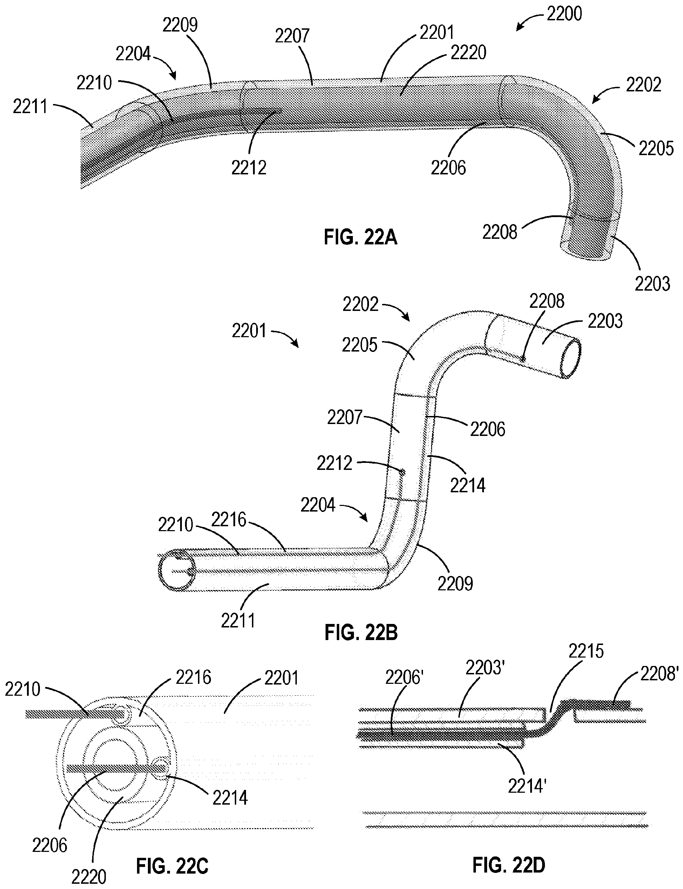

In some embodiments, first preformed bend 232 also may be actuated via a pull wire mechanism instead of relative movement between a shaping catheter and first preformed bend 232. Accordingly, support 230 may include another pull wire catheter having a rigid distal region disposed distal to first preformed bend 232, a flexible middle region disposed over first preformed bend 232, a rigid proximal region disposed proximal to first preformed bend 232, and a pull wire coupled to the distal region and extending along the middle and proximal regions, such that actuation of the pull wire adjusts the relative angle between the distal region and the proximal region by bending the flexible middle region to thereby adjust the bend angle of first preformed bend 232 along the bend plane of first preformed bend 232. Thus, the pull wire catheter for adjusting the bend angle of first preformed bend 232 may be disposed within the lumen of pull wire catheter 231 for adjusting the bend angle of second preformed bend 232. Alternatively, in some embodiments, first preformed bend 232 may be actuated via the pull wire mechanism while second preformed bend 234 may be actuated via relative movement of a shaping catheter and second preformed bend 234. Alternatively, in some embodiments, both first preformed bend 232 and second preformed bend 234 may be actuated via a pull wire mechanism, as described in further detail below with regard to FIGS. 22A to 25F.

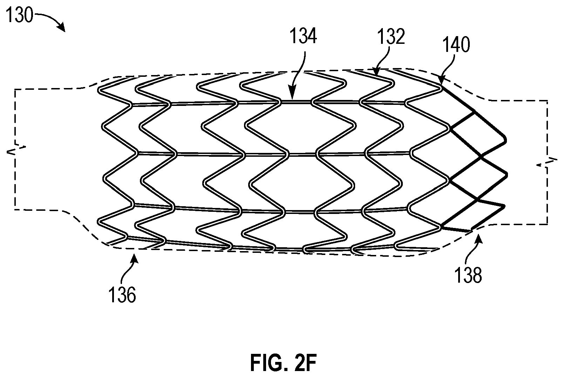

Referring now to FIG. 2F, an alternative exemplary anchor is provided. Like anchor 110, anchor 130 may be a stent formed of a stent structure, e.g., an expandable wire frame, and is preferably collapsible in a contracted, delivery state and expandable to an expanded, deployed state to anchor support 200 to a blood vessel coupled to the heart to thereby maintain prosthetic device 120 at the native cardiac valve. For example, anchor 130 may contact the inner wall of a blood vessel (e.g., the SVC or IVC) to anchor support 200 intraluminally, thereby maintaining prosthetic device 120 in a free-standing, suspended manner in the native heart valve.

As shown in FIG. 2F, the expandable wire frame of anchor 130 may include a plurality of strut rings 132, each extending around a circumference of anchor 130. For example, at least some of strut rings 132 may be coupled together via a plurality of longitudinal extending struts 134 to thereby define the stent structure of anchor 130. Anchor 130 may be configured to provide variability in radial stiffness along its length. For example, anchor 130 may include a first draping portion, e.g., distal draping portion 138, and a second draping portion, e.g., proximal draping portion 136, extending from apex 140, such that distal draping portion 138 has more flexibility that proximal draping portion 136. For example, as shown in FIG. 2F, the strut rings forming distal draping portion 138 may be formed of a thinner wire than the wire forming the strut rings forming at least proximal draping portion 136.

Moreover, as shown in FIG. 2F, distal draping portion 138 may be more flexible than proximal draping portion 136 as the strut rings forming distal draping portion 138 distal to apex 140 are directly coupled to each other without a plurality of longitudinal extending struts. Additionally or alternatively, anchor 130 may have radial stiffness variation with variations in diameter, strut length, and pitch. As will be understood by a person having ordinary skill in the art, in some embodiments, proximal draping portion 136 may have more flexibility than distal draping portion 138, such that the strut rings forming proximal draping portion 136 may be formed of a thinner wire than the wire forming the strut rings forming at least distal draping portion 138. Additionally or alternatively, both end regions of anchor 130 may include draping portions that have more flexibility than a middle portion of anchor 130, and/or anchor 130 may have any combination of the draping portion embodiments described herein.

Additionally or alternatively, anchor 130 may be configured to provide variability in stiffness along its circumference. For example, the wire frame forming anchor 130 may be stiffer closer to the anchor spine, e.g., where anchor 130 is coupled to support 200. For example, the length of the strut rings may vary along the circumference of anchor 130, e.g., the length may increase in a direction away from the anchor spine of anchor 130. Accordingly, anchor 130 may have variation in pitch and diameter axially, and strut length radially.

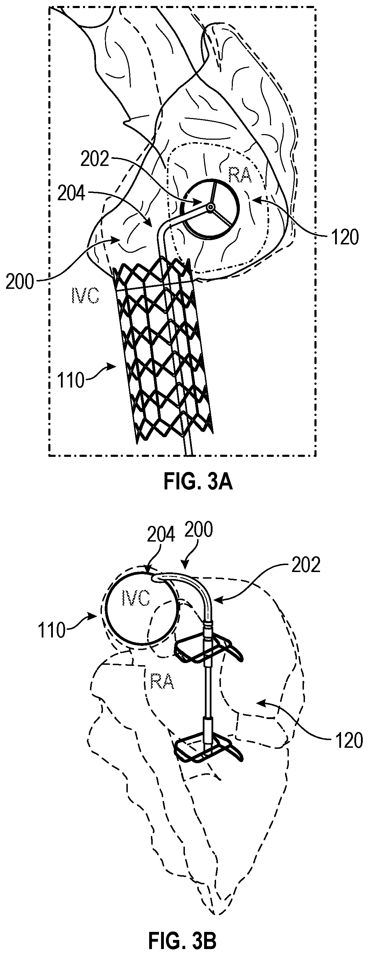

FIGS. 3A and 3B illustrate anchor 110 implanted within the patient's IVC such that support 200 suspends and maintains prosthetic device 120 across the patient's native cardiac valve. As shown in FIGS. 3A and 3B, second preformed bend 204 permits support 200 to navigate from the IVC into the RA, and first preformed bend 202 permits support 200 to navigate from the RA to be across and co-axial with the native cardiac valve.

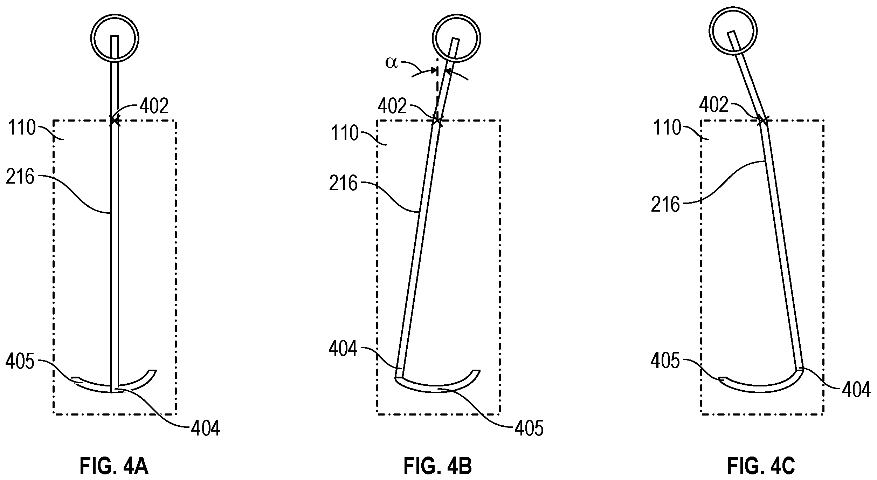

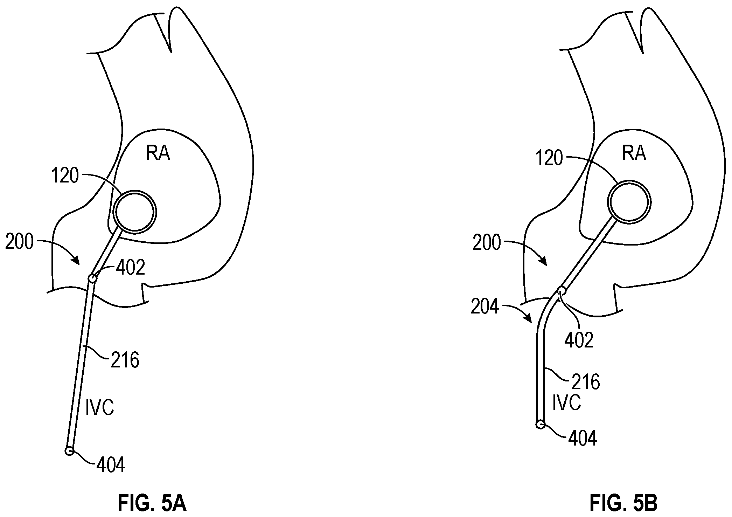

Referring now to FIGS. 4A to 4C, an alternative stent configuration for providing the adjustable second bend of heart valve therapeutic device 100 is provided. In this embodiment, elongated rail 206 may have first preformed bend 202, but does not have second preformed bend 204. Instead, the second adjustable bend, e.g., for navigation of support 200 from the IVC into the RA, may be provided by the stent configuration of FIGS. 4A to 4C, as described in further detail below. As shown in FIGS. 4A to 4C, a first end of anchor tube 216 may be pivotally coupled to anchor 110 at pivot point 402, while an opposing end 404 of anchor tube 216 may slidably coupled to stent 110, e.g., along rail 405. Accordingly, anchor tube 216 may be actuated, e.g., via handle 900, to move end 404 of anchor tube 216 along rail 405 such that anchor tube 216 pivots about pivot point 402. As shown in FIG. 4B, anchor tube 216 may be pivoted about pivot point 402 to provide support 200 a second bend angle of α. As shown in FIGS. 5A and 5B, by moving end 404 of anchor tube 216 relative to pivot point 402, the angle of second adjustable bend 204 may be selectively adjusted to facilitate navigation of support 200 through the IVC and right atrium RA..

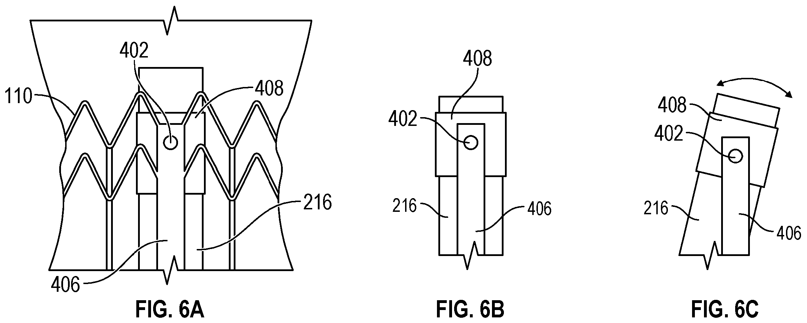

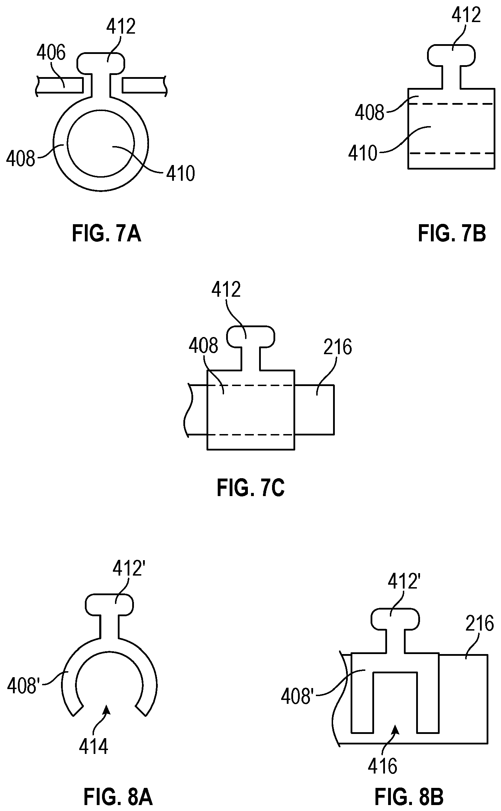

As shown in FIGS. 6A to 6C, anchor tube 216 may be pivotally coupled to spine 406 of anchor 110 at pivot point 402 via collar 408. Collar 408 is sized and shaped to receive and secure anchor tube 216 to pivot point 402. For example, as shown in FIGS. 7A to 7B, collar 408 may include lumen 410 sized and shaped to receive anchor tube 216 therethrough. Collar 408 may then be fixed to anchor tube 216 by, e.g., welding or an adhesive. In addition, collar 408 further may include tab 412 for rotatably engaging with spine 406 of anchor 110 at pivot point 402. Accordingly, tab 412 may extend from collar 408 via a neck portion sized and shaped to fit through an opening within spine 406, while providing adequate space such that collar 408 may rotate relative to spine 406. Tab 412 may have a larger diameter than the opening of spine 406, thereby preventing dislodgment of collar 408 from spine 406.

FIGS. 8A and 8B illustrate alternative collar 408' for pivotally coupling anchor tube 408 with spine 406 of anchor 110. Collar 408' may be constructed similar to collar 408, except that, as shown in FIG. 8A, collar 408' may include opening 414 extending longitudinally along the bottom portion of collar 408'. Accordingly, collar 408' may receive anchor tube 216 via opening 414, e.g., via a snap fit connection. For example, collar 408' may expand radially outwardly as anchor tube 216 is pushed through opening 414. As shown in FIG. 8B, collar 408' further may include opening 416, which may facilitate coupling of collar 408' and anchor tube 216. Collar 408' may then be fixed to anchor tube 216 by, e.g., welding or an adhesive.

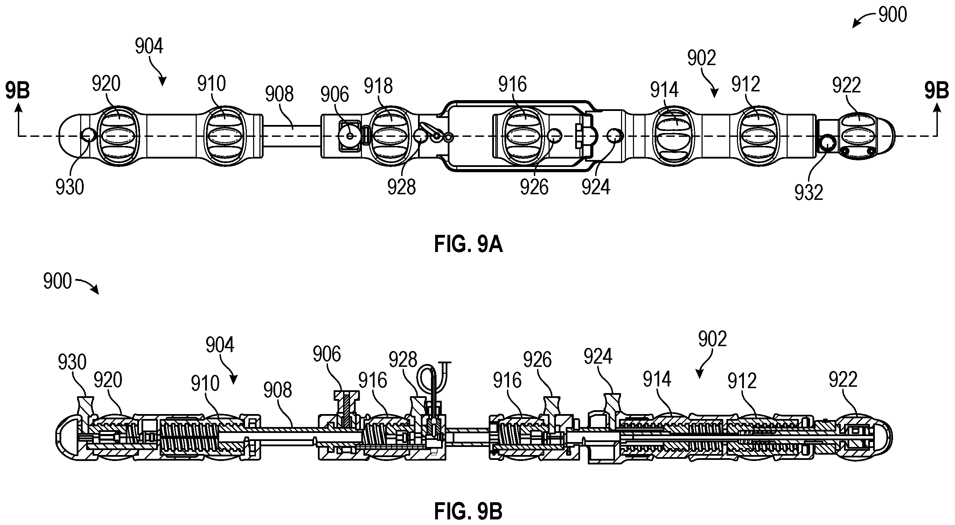

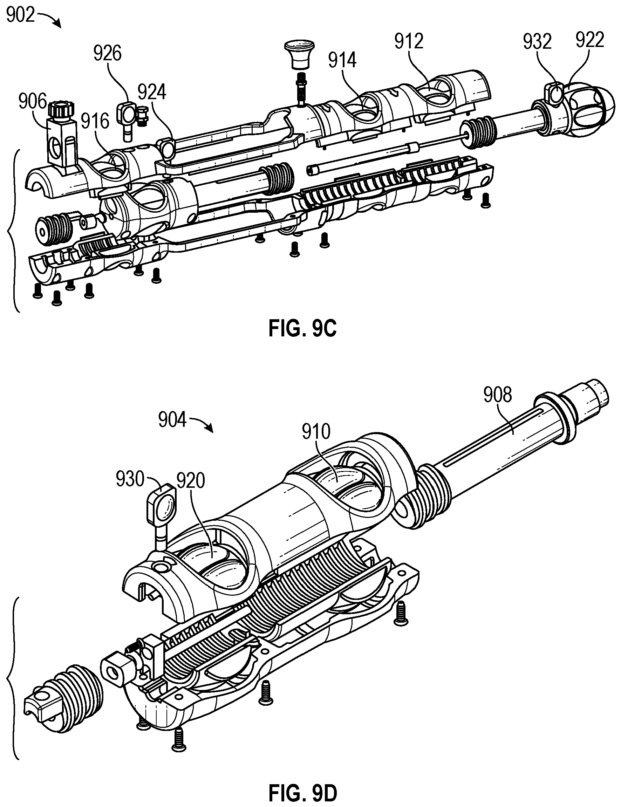

Referring now to FIGS. 9A to 9D, exemplary actuator 900 is provided. Actuator 900 may include proximal portion 902 rotatably coupled to distal portion 904, e.g., via rod 908. Actuator 900 may include tab 906, which may be moved between a locked position where proximal portion 902 is prevented from rotating relative to distal portion 904, and an unlocked positioned where proximal portion 902 may be rotated relative to distal portion 904, to thereby rotate prosthetic device 120, as described in further detail below.

Moreover, actuator 900 includes a plurality of interfaces, e.g., interfaces 910, 912, 914, 916, 918, 920, 922, for adjusting the position of prosthetic device 120 and support 200 relative to the patient's native cardiac valve, adjusting the bending angles of the first and second adjustable bends of elongated rail 206, locking the distal, implantable portion of support 200, and detaching the distal, implantable portion of support 200 from the proximal, delivery portion of support 200 to thereby implant the distal, implantable portion of support 200. For example, interfaces 910, 912 may be operatively coupled to support 200 and actuated to adjust the position of prosthetic device 120 and support 200 relative to the patient's native cardiac valve, and interface 914 may be operatively coupled to support 200 and actuated to adjust the bending angles of the first and second adjustable bends of elongated rail 206, as described in further detail below. Moreover, interfaces 916, 918 may be operatively coupled to support 200 and actuated to lock the distal, implantable portion of support 200 to each other, and interfaces 920, 922 may be operatively coupled to support 200 and actuated to detach the distal, implantable portion of support 200 from the proximal, delivery portion of support 200 to thereby implant the distal, implantable portion of support 200, as described in further detail below.

The interfaces of actuator 900 may be, e.g., rotatable knobs, buttons, sliders, or the like, which may be manipulated by a clinician. The interfaces of actuator 900 may be coupled to an internal lead screw, each lead screw connected to a collar rigidly attached to a select component of support 200, such that actuation of a given interface actuates a particular component of support 200. Actuator 900 further may include safety pins 924, 926, 928, 930, 932, which may initially be in an engaged positioned whereby the safety pins are coupled to actuator 900, thereby prevent actuation of a respective interface. For example, safety pins 924, 926, 928, 930, 932 may be associated with interfaces 914, 916, 918, 920, 922, respectively. The safety pins may be removed from actuator 900, e.g., by pulling them, by a clinician, and returned to the engaged position after actuation of the respective interface. In another embodiment the actuators may be replaced by alternative mechanism for controlling linear actuation, e.g. lever, thumbwheel, and/or linear slider.

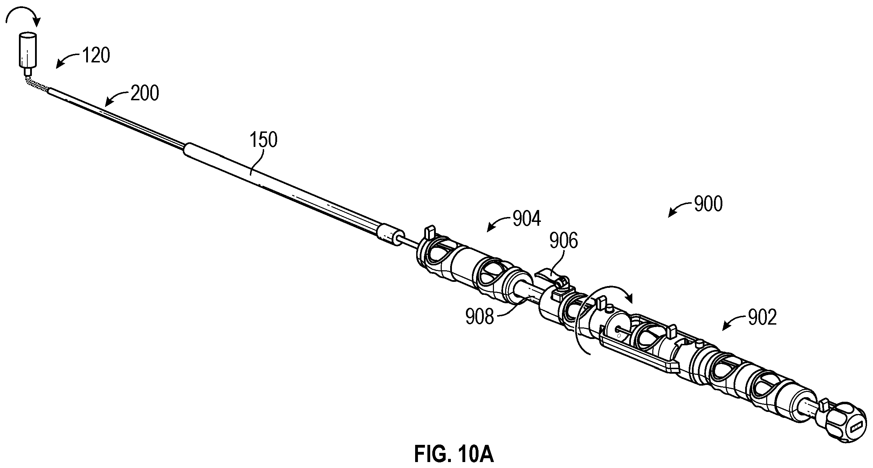

Referring now to FIGS. 10A to 10D, positional adjustment of the distal end of the heart valve therapeutic device 100, e.g., via support 200, using actuator 900 is described. As shown in FIG. 10A, tab 906 may be transitioned from the locked positioned to the unlocked positioned, e.g., by flipping tab 906. When tab 906 is in the unlocked position, proximal portion 902 of actuator 900 may be rotated relative to distal portion 904 of actuator 900. Proximal portion 902 may be coupled to elongated rail 206, such that rotation of proximal portion 902 causes elongated rail 206, and accordingly prosthetic device 120 coupled thereto, to rotate relative to the longitudinal axis of support 200. Accordingly, the clinician may hold distal portion 904 fixed relative to the patient, while rotating proximal portion 902 to adjust the rotational position of prosthetic device 120.

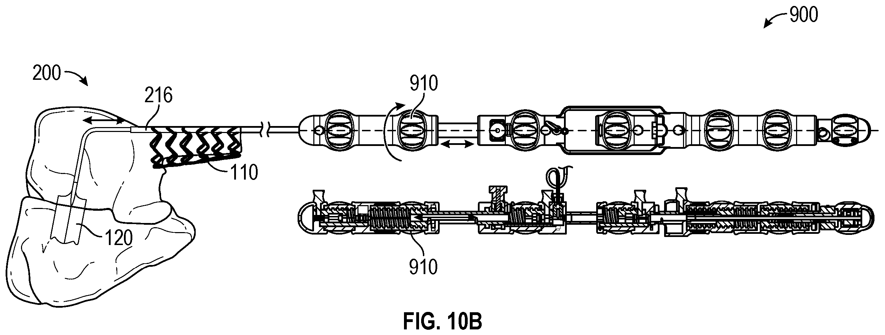

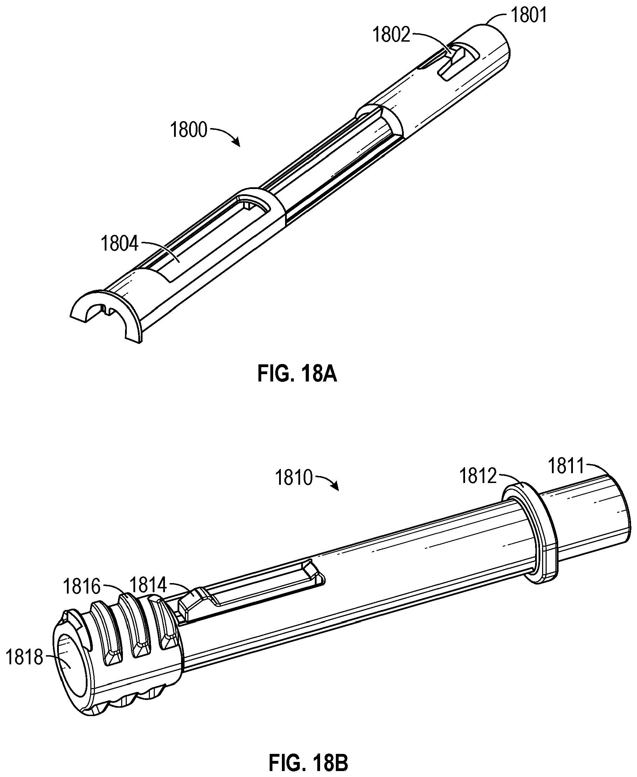

As shown in FIG. 10B, interface 910 of actuator 900 may be actuated to adjust the length of support 200, to thereby adjust the axial location of the one or more adjustable bends of elongated rail 206 relative to actuator 900. For example, actuation of interface 910 may cause the internal lead screw coupled thereto to rotate to thereby cause proximal portion 902 to move axially relative to distal portion 904. As proximal portion 902 is coupled to elongated rail 206 as described above, axially movement of proximal portion 902 relative to distal portion 904 while distal portion 904 is held fixed relative to the patient by the clinician, will cause elongated rail 206 to extend axially along the longitudinal axis of support 200. Thus, the axial distance of the adjustable bends of elongated rail 206 from actuator 900 may be shortened or lengthen via interface 910. The actuator may include a locking mechanism to limit the axial movement of distal portion 904 relative to proximal portion 902, as described in further detail below with regard to FIGS. 18A to 20.

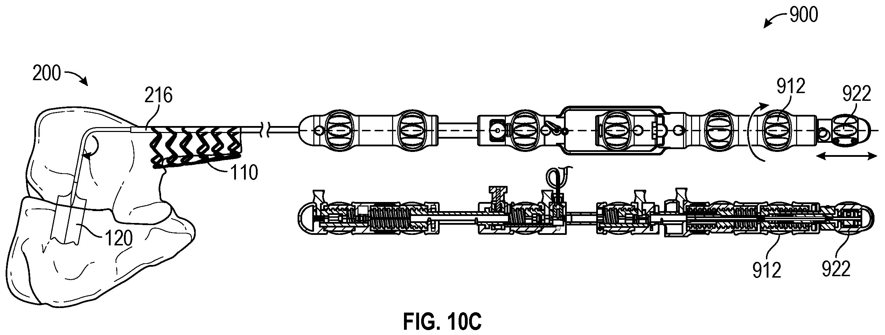

As shown in FIG. 10C, interface 912 of actuator 900 may be actuated to adjust the angle of an adjustable preformed bend, e.g., first preformed bend 202, of elongated rail 206. For example, actuation of interface 912 may cause the internal lead screw coupled thereto to rotate to thereby cause elongated rail 206 to move axially relative to first shaping catheter 208. As described above, because first shaping catheter 208 is more stiff than first preformed bend 202 of elongated rail 206, axial movement of first shaping catheter 208 relative to first preformed bend 202 will adjust the angle of first preformed bend 202. As will be understood by a person having ordinary skill in the art, first shaping catheter 208 may be actuated via an interface of actuator 900 while elongated rail 206 remains stationary relative to the patient, such that axial movement of first shaping catheter 208 relative to first preformed bend 202 adjusts the angle of first preformed bend 202. Similarly, second shaping catheter 210 may be actuated via an interface of actuator 900 while elongated rail 206 remains stationary relative to the patient, such that axial movement of second shaping catheter 210 relative to second preformed bend 204 adjusts the angle of second preformed bend 204. Actuator 900 may have any number of interfaces required to adjust any respective number of preformed bends of elongated rail 900, as described above.

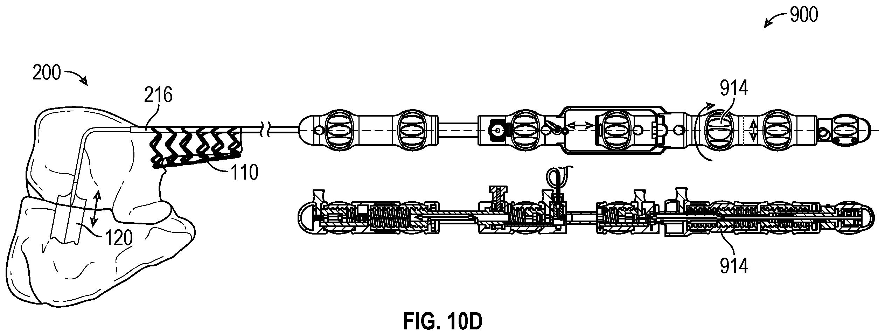

As shown in FIG. 10D, interface 914 of actuator 900 may be actuated to adjust the depth of prosthetic device 120 relative to the patient's native cardiac valve, e.g., toward the patient's right atrium or toward the patient's right ventricle. For example, actuation of interface 914 may cause the internal lead screw coupled thereto to rotate to thereby cause body support catheter 212 to move axially relative to actuator 900. As body support catheter 212 is coupled to prosthetic device 120, e.g., via connector 214, axial movement of body support catheter 212 will adjust the depth of prosthetic device 120 relative to the patient's native cardiac valve.

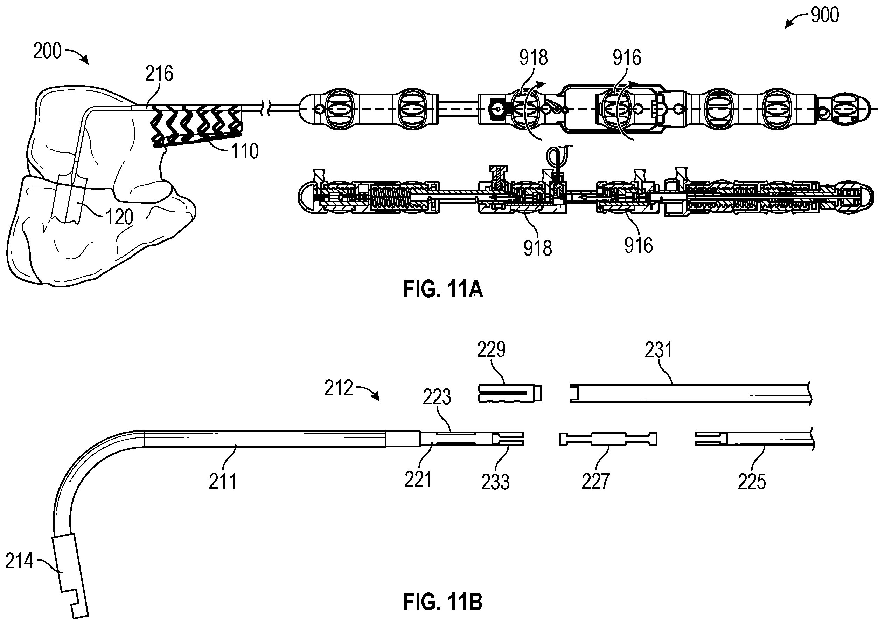

As shown in FIGS. 11A and 11B, interfaces 916, 918 may be actuated to lock the distal, implantable portion of support 200 to each other. Support 200 may include a locking mechanism as described in U.S. Patent No. 11,219,525 to Vesely. For example, as shown in FIG. 11B, body support catheter 212 may include distal, implantable portion 211 removably detachable to proximal, delivery portion 225 via body support catheter connection 227. Distal, implantable portion 211 may include distal body support locking portion 221 and distal body support connection portion 233. Distal body support locking portion 221 and distal body support connection portion 233 have a lumen sized and shaped to receive second shaping catheter 210, and accordingly first shaping catheter 208 and elongated rail 206, therethrough.

Distal body support locking portion 221 may include one or more slits, e.g., a U-shaped slit, forming interference locking portion 223. Interference locking portion 223 may be a wedged portion that has a thickness that is greater than the thickness of distal body support locking portion 221 and distal body support connection portion 233, such that the wedged portion may be pushed radially inward to lock distal, implantable portion 211 to second shaping catheter 210, as described in further detail below. Distal body support connection portion 233 may include an opening, e.g., a T-shaped opening, sized and shaped to interlink with body support catheter connection 227.

Body support catheter connection 227 may include a distal interlinking portion sized and shaped to releasably engage with distal body support connection portion 233 of distal, implantable portion 211, and a proximal interlinking portion sized and shaped to engage with proximal, delivery portion 225. For example, the distal interlinking portion of body support catheter connection 227 may have a shape that corresponds with the shape of the opening of distal body support connection portion 233, such that when the distal body support interlinking portion of body support catheter connection 227 is in its collapsed state, it interlinks with distal body support connection portion 233. The distal interlinking portion of body support catheter connection 227 may be biased radially outward, and may transition from the expanded state to a collapsed state upon application of a radially inward force. Accordingly, body support catheter lock 229 may be slidably disposed over body support catheter 212, such that when body support catheter lock 229 is disposed over the distal interlinking portion of body support catheter connection 227 and distal body support connection portion 233, body support catheter connection 227 remains engaged with distal body support connection portion 233.

Body support catheter lock 229 may include a proximal portion having a geometry sized and shaped to engage with the distal end of body support catheter pusher 231. Both body support catheter lock 229 and body support catheter pusher 231 have a lumen sized and shaped to slidably receive body support catheter 212 therethrough. Accordingly, body support catheter pusher 231 may be advanced distally responsive to actuation at actuator 900, e.g., by actuating interface 918 actuator 900 in a first direction as shown in FIG. 11A, such that body support catheter pusher 231 engages with body support catheter lock 229 to push body support catheter lock 229 distally from over body support catheter connection 227 to over distal body support locking portion 223. When body support catheter lock 229 is disposed over distal body support locking portion 223, the wedge shape of distal body support locking portion 223 causes distal body support locking portion 223 to move radially inward and engage with the outer surface of second shaping catheter 210 to thereby to lock distal, implantable portion 211 to second shaping catheter 210.

Body support catheter lock 229 may include one or more longitudinal slits which may permit the distal portion of body support catheter lock 229 to expand radially as body support catheter lock 229 is pushed over the wedge shape of distal body support locking portion 223 of distal locking portion 221. As body support catheter lock 229 expands radially, it engages with the inner surface of the lumen of anchor tube 216 to thereby lock distal, implantable portion 211 to anchor tube 216. Interface 918 may then be actuated in the opposite direction, such that body support catheter pusher 231 is retracted proximally relative to distal, implantable portion 211 to thereby expose the distal body support interlinking portion of body support catheter connection 227. Upon exposure from body support catheter pusher 231, the distal body support interlinking portion of body support catheter connection 227 transitions to the expanded state and disengages with distal body support connection portion 233. Proximal, delivery portion 225 of body support catheter 212 may then be removed from the patient while distal, implantable portion 211 of body support catheter 212 remains implanted. Similarly, interface 916 of actuator 900 may be actuated to move a corresponding pusher to lock and disconnect additional distal, implantable portions of support 200 to each other. As will be understood by a person having ordinary skill in the art, actuator 900 may include any number of interfaces to lock a corresponding number of distal, implantable portions of support 200.

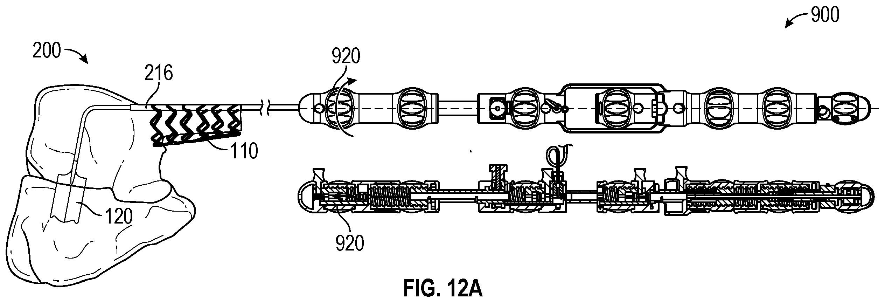

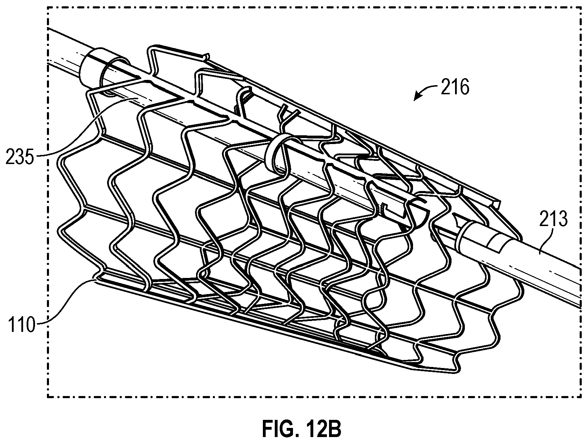

Referring now to FIGS. 12A and 12B, interface 920 of actuator 900 may be actuated to detach proximal, delivery portion 213 of anchor tube 216 from distal, implantable portion 235 of anchor tube 216. For example, proximal, delivery portion 213 may be retracted proximally responsive to actuation of actuator 900, e.g., by actuating interface 920 of actuator 900, to expose the distal anchor tube interlinking portion of the anchor tube connection. The distal anchor tube interlinking portion may self-expand from a collapsed delivery state within proximal, delivery portion 213 to an expanded state upon exposure from proximal, delivery portion 213, such that the distal anchor tube interlinking portion disengages with distal, implantable portion 235. As shown FIG. 12B, proximal, delivery portion 213 along with the other proximal, delivery components of support 200 may be removed from the patient while distal, implantable portion 235 remains implanted within the patient.

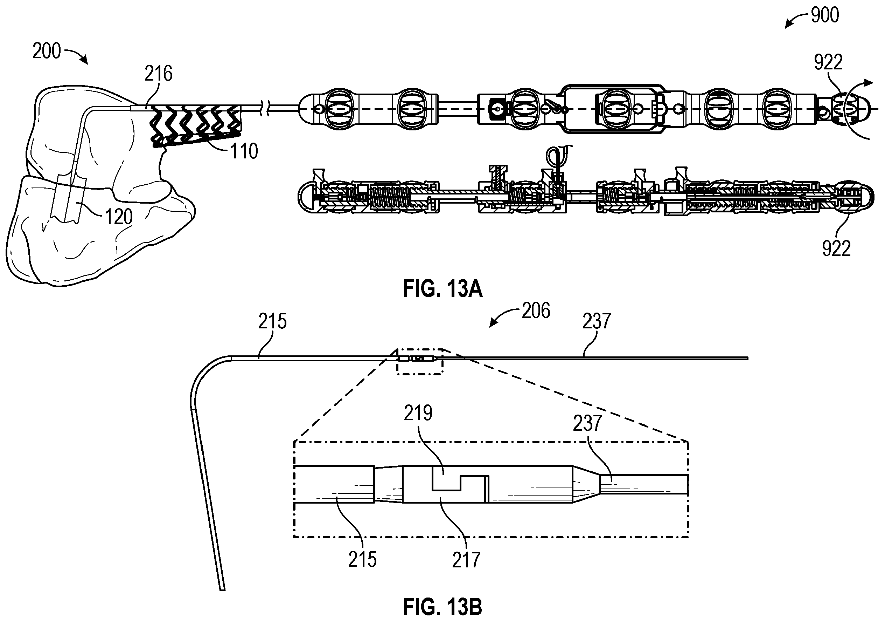

Referring now to FIGS. 13A to 13C, interface 922 of actuator 900 may be actuated to detach proximal, delivery portion 237 of elongated rail 206 from distal, implantable portion 215 of elongated rail 206. As shown in FIG. 13B, distal, implantable portion 215 and proximal, delivery portion 237 may be removably attached together. For example, distal, implantable portion 215 may have first geometry 217, and proximal, delivery portion 237 may have second geometry 219 corresponding to first geometry 217 such that distal, implantable portion 215 may engage with proximal, delivery portion 237 during delivery. For example, first and second geometries 217, 219 may be notches and teeth, wherein a tooth of first geometry 217 fits in the notch of second geometry 219 and a tooth of second geometry 219 fits in the notch of first geometry 217, thereby attaching elongated rail 206 during delivery. Distal, implantable portion 215 may be disengaged from proximal, delivery portion 237 for implantation of proximal, delivery portion 237 responsive to actuation of actuator 900, e.g., by actuating interface 922 of actuator 900.

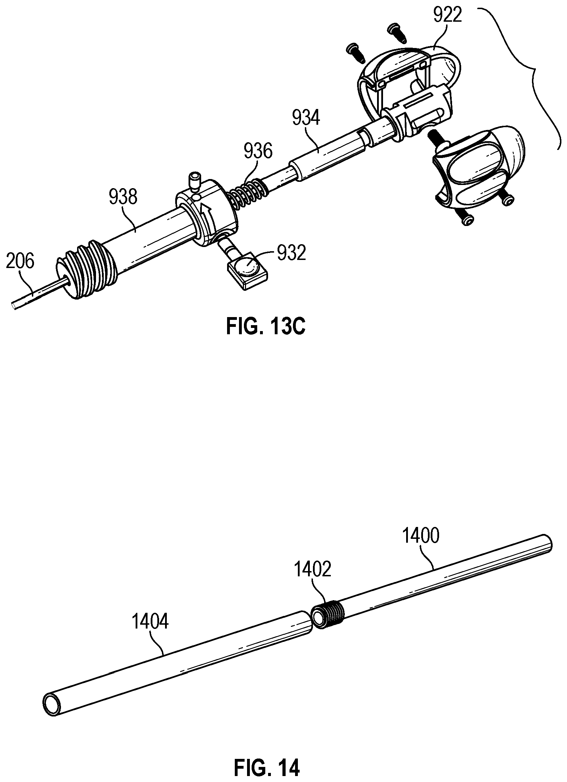

As shown in FIG. 13C, interface 922 may be coupled to internal housing 934, which is fixed elongated rail 206 extending through lead screw 938 having safety pin 932. Interface 922 may be rotated upon removal of pin 932 by the clinician. Accordingly, rotation of interface 922 causes internal housing 934 and elongated rail 206 to rotate relative to prosthetic device 120. As internal housing 934 and elongated rail 206 rotates to disengage proximal, delivery portion 237 of elongated rail 206 from distal, implantable portion 215 of elongated rail 206, spring 936 positioned between lead screw 938 and internal housing 934 simultaneously pushes internal housing 934 and interface 922 proximally, thereby releasing proximal, delivery portion 237 from distal, implantable portion 215.

Referring now to FIG. 14, an exemplary connector for coupling a body support catheter and a valve spine is provided. As described above, the distal end of the body support catheter may be coupled to prosthetic device 120, e.g., at a connector, such that elongated rail 206 may extend concentrically through the connector, and the body support catheter may be used to deliver and adjust and finally stabilize the position of prosthetic device 120 across the native cardiac valve. As described in U.S. Patent No. 11,219,525 to Vesely, the prosthetic device may include a valve spine slidably disposed over the elongated rail, such that the body support catheter is coupled to the prosthetic device via the valve spine.

As shown in FIG. 14, valve spine 1400 may include a plurality of ridges 1402, e.g., a plurality of barbs, disposed at a proximal region of valve spine 1400. For example ridges 1402 may extend circumferentially around an outer surface of proximal region of valve spine 1400. At least the distal region of body support catheter 1404 may be formed of a material that is configured to expand responsive to an external stimulus. For example, the distal region of body support catheter 1404 may be formed of a polymer that is configured to swell when immersed in a dedicated swelling fluid. Accordingly, upon interaction with the external stimulus, the diameter of the distal region of body support catheter 1404 may increase in size, such that the distal region of body support catheter 1404 may be advanced over ridges 1402 of valve spine 1400. Upon removal of the external stimulus, e.g., evaporation of the swelling fluid, the diameter of the distal region of body support catheter 1404 may return to its unexpanded size over ridges 1402, thereby locking body support catheter 1404 to valve spine 1400 via an interference fit between an inner surface of the distal region of body support catheter 1404 and ridges 1402.

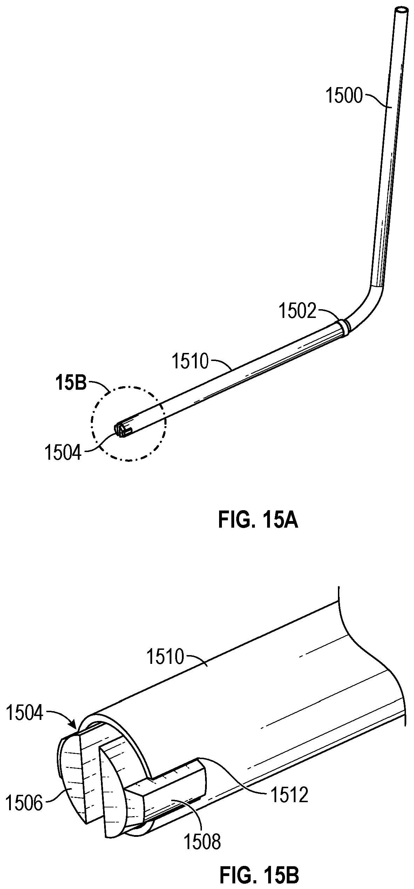

Referring now to FIGS. 15A and 15B, another exemplary connector for coupling a body support catheter and a valve spine is provided. As shown in FIG. 15A, the connector may include elongated rod 1504 having a proximal end coupled to a distal end of body support catheter 1500, e.g., adjacent to proximal abutment 1502 of support 1500. Proximal abutment 1502 may have an outer diameter that is larger than the outer diameter of elongated rod 1504. As shown in FIG. 15B, the distal end of elongated rod 1504 may include one or more collapsible prongs, e.g., pair of prongs 1506. Prongs 1506 are configured to be collapsed from an expanded state radially inward to a collapsed state upon application of a force thereon. Accordingly, upon removal of the force, prongs 1506 may return to their expanded state. As shown in FIG. 15B, each of prongs 1506 may include protrusion 1508 extending radially outward therefrom.

Valve spine 1510 of the prosthetic device may have a lumen sized and shaped to slidably receive elongated rod 1504 therein. As shown in FIG. 15B, the distal region of valve spine 1510 may include one or more grooves 1512 sized and shaped to receive protrusions 1508 therein. For example, grooves 1512 may have a geometry corresponding with the geometry of protrusions 1508, such that when protrusions 1508 are disposed within grooves 1512, valve spine 1510 cannot move distally or rotationally relative to elongated rod 1504. Accordingly, prongs 1506 may be collapsed to the collapsed state, such that the proximal end of valve spine 1510 may be advanced over prongs 1506 and along the length of elongated rod 1504 until the proximal end of valve spine 1510 abuts proximal abutment 1502. Valve spine 1510 and elongated rod 1504 may each have a length such that when the proximal end of valve spine 1510 abuts proximal abutment 1502, protrusions 1508 are aligned with grooves 1512 such that prongs 1506 may return to their expanded state and protrusions 1508 are disposed within grooves 1512. Accordingly, protrusions 1508 and proximal abutment 1502 may limit or prevent axial movement of valve spine 1510 relative to elongated rod 1504, while protrusions 1508 limits or prevents rotation of valve spine 1510 relative to elongated rod 1504.

Alternatively, instead of one or more prongs and protrusions at the distal end of the elongated rod, the distal end of the elongated rod may be configured to be coupled to a plug having a distal abutment. Like the proximal abutment, the distal abutment may have an outer diameter that is larger than the outer diameter of the elongated rod. Accordingly, the proximal end of the valve spine may be advanced over the distal end of the elongated rod and along the length of the elongated rod until the proximal end of the valve spine abuts the proximal abutment, and the distal end of the elongated rod is aligned with the distal end of the valve spine. The plug may then be coupled to the distal end of the elongated rod, e.g. via a threaded mating connection, a snap fit connection, or an interference fit connection, such that the distal abutment of the plug and the proximal abutment of the body support catheter sandwich the valve spine therebetween to thereby prevent axial and rotational movement of the valve spine relative to the elongated rod.

Referring now to FIGS. 16A and 16B, an exemplary coupling of an anchor spine to the support is provided. As described above, anchor 110 may be coupled to support 200 to anchor support 200 intraluminally, thereby anchoring prosthetic device 120 in a free-standing, suspended manner in the native heart valve. As described in U.S. Patent No. 11,219,525 to Vesely, the anchor may include an anchor spine that may be coupled to the support, e.g., via an anchor tube slidably disposed over the support. As described herein with regard to FIGS. 16A and 16B, anchor connector 1600 may include anchor spine 1602, which may be directly coupled to the support, e.g., second shaping catheter 1608, without the need for an anchor tube. Alternatively, anchor spine 1602 may be coupled to an anchor tube slidably disposed over second shaping catheter 1608.

As shown in FIG. 16A, anchor spine 1602, which is coupled to a stent (not shown), may include central guiding portion 1604 having a passageway sized and shaped to receive second shaping catheter 1608 therethrough. Central guiding portion 1604 may be aligned with anchor spine 1602 to centralize the support relative to the stent. In addition, anchor spine 1602 may include one or more flexible guiding portions, e.g., proximal flexible guiding portions 1606a disposed proximal to central guiding portion 1604 and distal flexible guiding portions 1606b disposed distal to central guiding portion 1604. The flexible guiding portions are configured to transition between a radially collapsed state and an radially expanded state, and are biased toward the radially collapsed state. Accordingly, the flexible guiding portions may be transitioned to the radially expanded state upon an application of force thereon. Moreover, in the radially expanded state, the axes of the passageways of the flexible guiding portions are aligned with the axis of the passageway of central guiding portion 1604, and are sized and shaped to receive second shaping catheter 1608 therethrough.

As shown in FIGS. 16A and 16B, a temporary catheter, e.g., stent support catheter 1610, may be used to facilitate coupling of anchor spine 1602 and second shaping catheter 1608. For example, stent support catheter 1610 may have a lumen sized and shaped to receive second shaping catheter 1608, central guiding portion 1604, and the flexible guiding portions in their radially expanded states therethrough. In addition, stent support catheter 1610 may have opening 1612 sized and shaped to receive anchor spine 1602 therein. For example, opening 1612 may have a geometry that corresponds with the geometry of stent support catheter 1610, such that when central guiding portion 1604 and the flexible guiding portions are disposed within the lumen of stent support catheter 1610, opening 1612 limits or prevents rotation of anchor spine 1602 relative to stent support catheter 1610, while permitting stent support catheter 1610 to slide axially over an entire length of anchor spine 1602, thereby providing rigidity to central guiding portion 1604 and the flexible guiding portions.

To couple anchor spine 1602 to second shaping catheter 1608, the flexible guiding portions may be expanded to their expanded states such that the flexible guiding portions and central guiding portion 1604 may be disposed within the lumen of stent support catheter 1610. The inner surface of stent support catheter 1610 applies a force to the flexible guiding portions disposed therein to maintain the flexible guiding portions in their expanded states. Accordingly, second shaping catheter 1608 may be advanced through proximal flexible guiding portions 1606a, central guiding portion 1604, and distal flexible guiding portions 1606b, until anchor spine 1602 is in a desired position along second shaping catheter 1608. Stent support catheter 1610 may then be removed, thereby removing the force applied to the flexible guiding portions, such that the flexible guiding portions transition toward their collapsed states. Accordingly, the flexible guiding portions will clamp onto second shaping catheter 1608 to thereby lock anchor spine 1602 relative to second shaping catheter 1608.

During the removal of stent support catheter 1610, the retention force between second shaping catheter 1608 and anchor spine 1602 may be increased by applying a biocompatible elastomer on the outer surface of second shaping catheter 1608. Moreover, although four proximal flexible guiding portions are illustrated in FIG. 16A, as will be understood by a person having ordinary skill in the art, less or more than four proximal flexible guiding portions may be coupled to anchor spine 1602 to decrease or increase the retention force between second shaping catheter 1608 and anchor spine 1602.

Alternatively, the outer surface of shaping catheter may include a number of grooves corresponding to the number of flexible guiding portions, such that the grooves are sized and shaped to receive the flexible guiding portions therein. For example, the grooves may have a geometry corresponding to the geometry of the flexible guiding portions, such that when the flexible guiding portions are disposed within the grooves, axial and rotational movement of the anchor spine relative to the shaping catheter is limited or prevented. In this embodiment, to couple the anchor spine to the shaping catheter, a stent support catheter may be disposed between the shaping catheter and the flexible guiding portions in their expanded state. For example, the flexible guiding portions may be expanded to their expanded state, and the stent support catheter may be inserted through the flexible guiding portions to thereby maintain the flexible guiding portions in their expanded state. The shaping catheter may then be inserted through the lumen of the stent support catheter until the flexible guiding portions are aligned with the grooves on the outer surface of the shaping catheter. Accordingly, upon removal of the stent support catheter, the flexible guiding portions will transition toward their collapsed states within the corresponding grooves to thereby limit or prevent axial and rotational movement of the anchor spine relative to the shaping catheter.

In some embodiments, the flexible guiding portions may be one or more compliant rings, such that a stent support catheter may be inserted through the compliant rings to expand the compliant rings to an expanded state, and the shaping catheter may be inserted through the lumen of the stent support catheter until the anchor spine is in a desired position relative to the shaping catheter. Upon removal of the stent support catheter, the compliant rings will transition toward their collapsed states and clamp the shaping catheter to thereby prevent axial and rotational movement of the anchor spine relative to the shaping catheter. As will be understood by a person having ordinary skill in the art, when only one shaping catheter is utilized, e.g., when the support only has one adjustable bend, the anchor spines described herein may be directly coupled to the corresponding shaping catheter or the outermost catheter of the support.

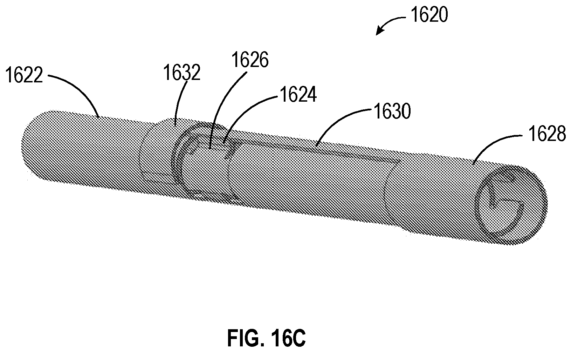

Referring now to FIG. 16C, another alternative mechanism for coupling the anchor spine to a shaping catheter slidably disposed therein, e.g., second shaping catheter 1608, is provided. As shown in FIG. 16C, anchor connector 1620 may include anchor spine 1622 coupled to the anchor stent (not shown), for coupling to the shaping catheter slidably disposed therein. Anchor spine 1622 may be formed of a superelastic material, e.g., nitinol, and may include one or more flexible leaves 1624. For example, leaves 1624 may be lasercut from anchor spine 1622, and may extend from anchor spine 1622 via hinge portion 1626 at the base of leaves 1624. Preferably, leaves 1624 are lasercut to have a maximum number of corners. Moreover, hinge portion 1626 of each of the one or more leaves 1624 may be disposed along anchor spine 1622 in a manner such that the axial rigidity of anchor spine 1622 is maintained. Leaves 1624 are transitionable between a collapsed state where leaves 1624 extend radially inward toward the longitudinal axis of anchor spine 1622, and an expanded state where leaves 1624 extend radially outward from the longitudinal axis of anchor spine 1622. Moreover, leaves 1624 are biased toward the collapsed state, such that a force is required to maintain leaves 1624 in the expanded state.

Accordingly, as shown in FIG. 16C, anchor connector 1620 further may include disconnect catheter 1628 slidably disposed over at least a portion of anchor spine 1622 for transitioning leaves 1624 from the expanded state to the collapsed state. For example, disconnect catheter 1628 may include tab 1630 extending longitudinally along an outer surface of anchor spine 1622 and configured to engage with leaves 1624 in the expanded state, to thereby maintain leaves 1624 in the expanded state. As shown in FIG. 16C, anchor spine 1622 may include retainer 1632, which may be welded onto an outer surface of anchor spine 1622 and configured to maintain tab 1630 in position relative to anchor spine 1622, and accordingly permit tab 1630 to maintain leaves 1624 in the expanded state. For example, retainer 1632 may be a flexible sleeve. Accordingly, retainer 1632 may apply a force on tab 1630 sufficient to maintain tab 1630 in position relative to leaves 1624 in the expanded state, and to anchor spine 1622, to thereby prevent inadvertent movement of tab 1630 and accordingly, premature release of leaves 1624.

Upon actuation of disconnect catheter 1628, e.g., via the handle, disconnect catheter 1628 may be moved relative to anchor spine 1622, such that tab 1630 disengages with retainer 1632 and leaves 1624, and thus permits leaves 1624 to transition from the expanded state to the collapsed state where leaves 1624 apply a radially inward clamping force to the shaping catheter slidably disposed within anchor spine 1622 to thereby couple anchor spine 1622 to the shaping catheter. Accordingly, a proximal end of disconnect catheter 1628 may be operatively coupled to the handle. In some embodiments, tab 1630 need not extend from a disconnect catheter, and instead may be actuated to be rotated about its longitudinal axis to disengage and release leaves 1624. For example, tab 1630 may have a rectangular cross-sectional area formed by two oppositely-facing wide planar sides and two oppositely-facing edges, such that the edge of tab 1630 engages with leaves 1630 in the expanded state, and upon rotation of tab 1630, the edge of tab 1630 disengages and releases leaves 1630 to permit leaves 1630 to transition to the collapsed state. In some embodiments, the portion of leaves 1624 that comes into contact with the shaping catheter may be lasercut to include teeth configured to facilitate engagement of leaves 1626 with the shaping catheter in the collapsed state. Additionally or alternatively, the outer surface of the shaping catheter may include a surface treatment configured to increase friction between the outer surface of the shaping catheter and leaves 1624 in the collapsed state, to thereby improve coupling between anchor spine 1622 and the shaping catheter. For example, the outer surface of the shaping catheter may include polymeric reflow, bead blasting, etc. As will be understood by a person having ordinary skill in the art, anchor connector 1620 may be used to coupled anchor spine 1622 to any catheter slidably disposed within the lumen of anchor spine 1622. In addition, the clamping mechanism utilized by anchor connector 1620 may be used to couple other adjacent catheters of support 200 described herein, e.g., first shaping catheter 208 to second shaping catheter 210, etc.

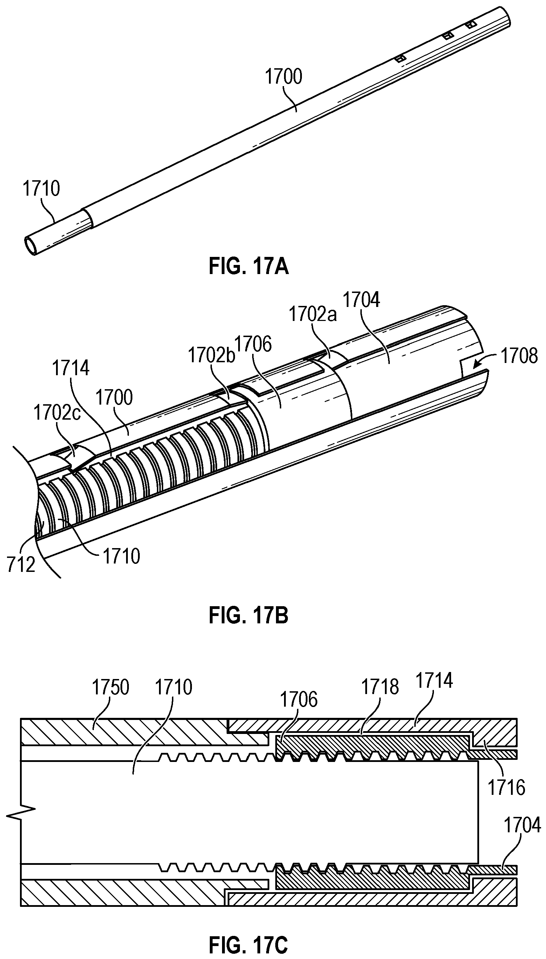

Referring now to FIGS. 17A to 17B, an exemplary mechanism for adjusting the position of the one or more adjustable bends of the heart valve therapeutic device is provided. As described in U.S. Patent No. 11,219,525 to Vesely, the anchor may be coupled to the support via an anchor tube slidably disposed over the support. For example, as shown in FIGS. 17A and 17B, anchor tube 1700 coupled to a stent (not shown) may be slidably disposed over at least the proximal region of the distal, implantable portion of the support, e.g., second shaping catheter 1710. As anchor tube 1700 is implanted within a vessel coupled to the patient's heart, second shaping catheter 1710 may be moved axially relative to anchor tube 1700 to thereby adjust the position (e.g., "height") of the one or more adjustable bends of the support relative to the patient's native heart valve.

As shown in FIG. 17B, at least a proximal portion of the outer surface of second shaping catheter 1710 may have a threaded surface. Moreover, driver 1704 may be rotatably disposed within the lumen of anchor tube 1700. For example, the proximal end of driver 1708 may include one or more grooves 1708 sized and shaped to engage with a rotation member, such that the rotation member may be engaged with groove 1708 and rotated to thereby cause rotation of driver 1704 relative to anchor tube 1700. Shoulder portion 1706 of driver 1704 may have an outer diameter larger than the proximal portion of driver 1704, and may have a lumen sized and shaped to receive the proximal portion of second shaping catheter 1710 therein. In addition, the inner surface of shoulder portion 1706 of driver 1704 may have a threaded surface configured to rotatably engage with the threaded surface on the outer surface of second shaping catheter 1710. Driver 1704 is axially controlled relative to anchor tube 1700 and second shaping catheter 1710 is rotationally fixed relative to anchor tube 1700, such that rotation of driver 1704 causes translational movement of second shaping catheter 1710 relative to driver 1704 and anchor tube 1700.

Anchor tube 1700 may include a control mechanism for controlling/limiting axial movement of driver 1704 relative to anchor tube 1700, e.g., one or more pins, tabs, flaps, etc. For example, as shown in FIG. 17B, anchor tube 1700 may have one or more pairs of inward facing tabs or flaps, e.g., flaps 1702a and 1702b, configured to control/limit axial movement of driver 1704 relative to anchor tube 1700. For example, flap 1702a may be positioned on a proximal side of shoulder portion 1706 and flap 1704b may be positioned on a distal side of shoulder portion 1706, such that driver 1704 may rotate within anchor tube 1700, but axial movement of driver 1704 is controlled or otherwise limited relative to anchor tube 1700. Moreover, second shaping catheter 1710 may include rail 1714 extending along at least a portion of the proximal portion of second shaping catheter 1710, such that rail 1714 is sized and shaped to slidably receive inward facing tab or flap of anchor tube 1700, e.g., tab 1702c. Rail 1714 permits tab 1702c to move axially along rail 1714, while preventing rotation of second shaping catheter 1710 relative to anchor tube 1700.

Referring now to FIG. 17C, an alternative exemplary mechanism for adjusting the position of the one or more adjustable bends of the heart valve therapeutic device is provided. As shown in FIG. 17C, the proximal end of anchor tube 1750 may include insert 1714 having lip portion 1716, such that the distal end of anchor tube 1750 and lip portion 1716 define groove 1718 extending circumferentially within the inner surface of anchor tube 1750 and insert 1714. Groove 1718 is sized and shaped to receive shoulder portion 1706 of driver 1704 therein, to thereby control or limit axial movement of driver 1704 relative to anchor tube 1750. Accordingly, as driver 1704 is rotated within groove 1718, the distal end of anchor tube 1700 and lip portion 1716 controls or limits axial movement of driver 1704 relative to anchor tube 1750, to thereby cause axial movement of second shaping catheter 1710 relative to anchor tube 1750.

Thus, in this embodiment, anchor tube 1750 also may include a control mechanism for controlling/limiting axial movement of driver 1704 relative to anchor tube 1750, e.g., one or more pins, tabs, flaps, etc. For example, second shaping catheter 1710 also may include a rail sized and shaped to slidably receive an inward facing tab or flap of anchor tube 1750, such that axial movement of second shaping catheter 1710 is permitted while rotational movement of second shaping catheter 1710 relative to the anchor tube is prevented. In some embodiments, insert 1714 and anchor tube 1750 may be formed as a single integrated component, such that groove 1718 is formed circumferentially along an inner surface of the anchor tube. As will be understood by a person having ordinary skill in the art, when only one shaping catheter is utilized, e.g., when the support only has one adjustable bend, the anchor tubes described herein may be slidably disposed over the corresponding shaping catheter or the outermost catheter of the support.

Referring now to FIGS. 18A to 18C, an exemplary mechanism for limiting adjustment of the axial location of the one or more adjustable bends of the heart valve therapeutic device is provided. As described above, proximal portion 902 of handle 900 may be rotatably coupled to distal portion 904, e.g., via a rod. Moreover, interface 910 of actuator 900 may be actuated to adjust the length of support 200, e.g., by adjusting the distance between proximal portion 902 and distal portion 904 along the rod, to thereby adjust the axial location of the one or more adjustable bends of elongated rail 206 relative to actuator 900. In addition, interface 918 may be operatively coupled to support 200 and actuated to lock the distal, implantable portion of support 200 to each other.

As described herein with regard to FIGS. 18A to 18C, handle 900 further may include axial control mechanism 1800, e.g., a slider component, configured to prevent a predefined additional amount of axial movement of proximal portion 902 relative to distal portion' 904 until interface 918 has been fully actuated to lock the distal, implantable portion of support 200. As shown in FIG. 18A, axial control mechanism 1800 may include proximal end 1801, retention clip 1802, and opening 1804 distal to retention clip 1802. Retention clip 1802 may have a protrusion extending therefrom and is configured to be collapsed radially inward upon application of a force thereon, e.g., an axial force. Accordingly, a distal side of the protrusion of retention clip 1802 may be tapered to facilitate collapsing of retention clip 1802 upon application of an axial force thereon. Opening 1804 is sized and shaped to receive the safety clip of rod 1810 as described in further detail below.

As shown in FIG. 18B, rod 1810 may be configured to rotatably couple proximal portion 902 and distal portion 904, as well as to permit relative axial movement between proximal portion 902 and distal portion 904. Rod 1810 may include proximal end 1811, ridge 1812, safety clip 1814, threaded portion 1816, and lumen 1818. Ridge 1812 may extend at least partially circumferentially around an outer surface of rod 1810 to thereby fix rod 1810 axially relative to proximal portion 902 as described in further detail below. Safety clip 1814 may have a protrusion extending therefrom and is configured to be collapsed radially inward upon application of a force thereon, e.g., an axial force. Accordingly, a proximal side of the protrusion of safety clip 1814 may be tapered to facilitate collapsing of safety clip 1814 upon application of an axial force thereon. Moreover, safety clip 1814 is sized and shaped to be received through opening 1804 of axial control mechanism 1800. Threaded surface 1816 is configured to rotatably engage with interface 910, such that actuation of interface 910 may cause axial movement of distal portion 904 relative to rod 1810. In addition, lumen 1818 is sized and shaped to slidably receive axial control mechanism 1800 therein.

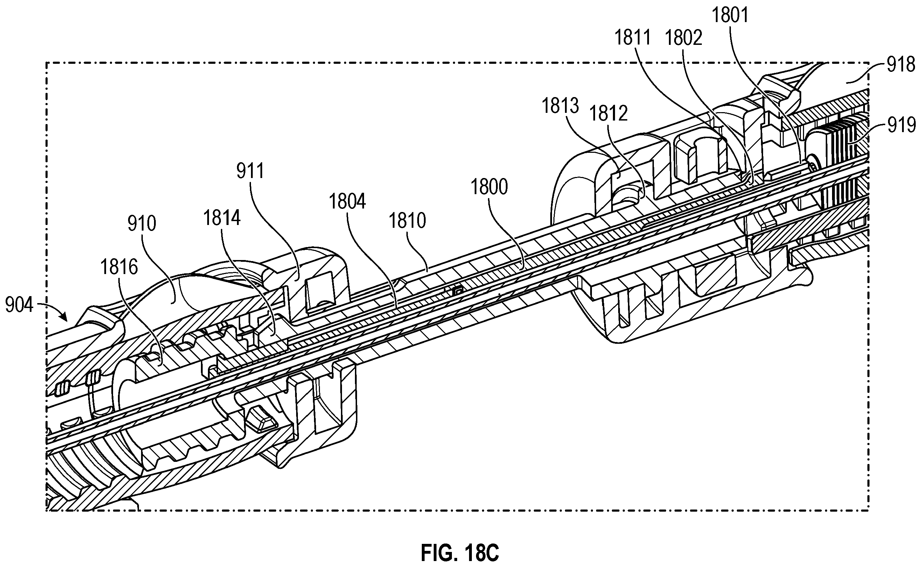

As shown in FIG. 18C, a proximal portion of rod 1810 may be disposed within proximal portion 902 of handle 900, such that ridge 1812 is disposed within groove 1813 of proximal portion 902, to thereby axially fix rod 1810 relative to proximal portion 902. In addition, a distal portion of rod 1810 may be disposed within distal portion 904 of handle 900, such that threaded surface 1816 is rotatably engaged with interface 910 and safety clip 1814 is adjacent to and/or abuts ridge 911 of the housing of distal portion 904. As shown in FIG. 18C, axial control mechanism 1800 may be disposed within the lumen of rod 1810, such that proximal end 1801 is disposed in proximity of driver 919 operatively coupled to interface 918. Axial control mechanism 1800 is slidably moveable within rod 1810, e.g., via an axial force applied by driver 919 upon actuation of interface 918, between a first position where retention clip 1802 is adjacent to and/or abuts proximal end 1811 of rod 1810 and opening 1804 is proximal to safety clip 1814 such that safety clip 1814 cannot collapse radially inward within opening 1804, and a second position where an axial force of proximal end 1811 of rod 1810 causes retention clip 1802 to collapse radially inward and opening 1804 is aligned with safety clip 1814 such that safety clip 1814 may collapse radially inward within opening 1804.

As described above, interface 910 may be actuated to cause axial movement of proximal portion 902 relative to distal portion 904 until ridge 911 abuts safety clip 1814. When axial control mechanism 1800 is in the first position, additional axial movement between proximal portion 902 and distal portion 904 is not permitted as safety clip 1814 cannot collapse radially inward due to an axial force applied by ridge 911, and thus safety clip 1814 prevents distal movement of distal portion 904 relative to rod 1810. Moreover, retention clip 1802 prevents axial movement of axial control mechanism 1800 relative to rod 1810. As described above, interface 918 may be actuated to lock the distal, implantable portion of support 200. As interface 918 is actuated, e.g., rotated, driver 919 operatively coupled thereto moves axially relative to interface 918. Accordingly, interface 918 may be required to be fully actuated, e.g., to fully lock the distal, implantable portion of support 200, before additional axial movement between proximal portion 902 and distal portion 904 is permitted.

For example, when interface 918 is fully actuated, driver 919 moves distally relative to interface 918, thereby abutting and applying an axial force on proximal end 1801 to cause distal movement of axial control mechanism 1800 relative to rod 1810. As axial control mechanism 1800 moves distally relative to rod 1810, proximal end 1811 of rod 1810 applies an axial force on retention clip 1802 to thereby radially collapse retention clip 1802 inward. Axial control mechanism 1800 may continue to move distally until interface 918 is fully actuated, and opening 1804 is aligned with safety clip 1814 in the second position. Accordingly, upon full actuation of interface 918, such that the distal, implantable portion of support 200 is fully locked, interface 910 may then be further actuated to provide a predefined additional amount of axial movement of proximal portion 902 relative to distal portion 904. Specifically, further actuation of interface 910 will cause ridge 911 to apply an axial force to safety clip 1814, e.g., as proximal portion 902 moves proximally relative to distal portion 904, such that safety clip 1814 collapses radially inward within opening 1804.

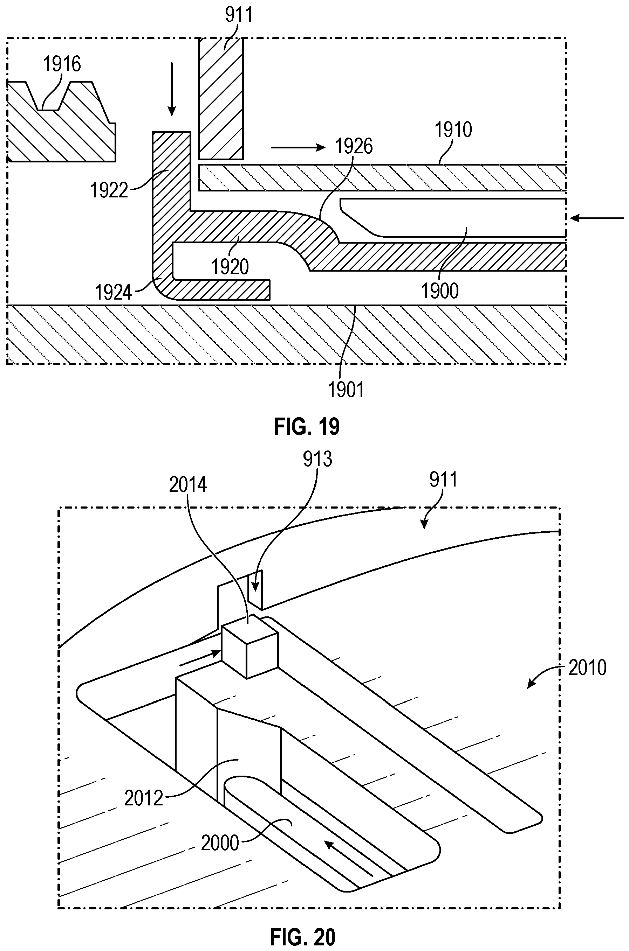

Referring now to FIG. 19, another exemplary mechanism for limiting adjustment of the axial location of the one or more adjustable bends of the heart valve therapeutic device is provided. As shown in FIG. 19, rod 1910 may include guide rail 1901 disposed therein having a lumen configured to slidably receive the proximal, delivery portion of support 200 therethrough. Moreover, the proximal and distal portions of the handle may be rotatably coupled to each other via rod 1910. Rod 1910 includes threaded surface 1916 rotatably coupled to interface 910, such that actuation of interface 910 causes relative axial movement between the proximal and distal portions of the handle along rod 1910. In addition, rod 1910 may be axially fixed relative to proximal portion 902.

As shown in FIG. 19, the axial control mechanism may include slider component 1900 and interlock component 1920. Interlock component 1920 may be at least partially disposed within slider component 1900, and slider component 1900 may be slidably disposed between interlock component 1920 and rod 1910. Interlock component 1920 may be axially fixed relative to rod 1910, and may be configured to collapse radially inward due to an application of force, e.g., an axial force. For example, interlock component 1920 may include upper portion 1922, hinge portion 1926, and optional spring portion 1924. Spring portion 1924 may contact guide rail 1901 to keep upper portion 1922 adjacent to ridge 911. Moreover, slider component 1900 may be slidably moveable between a first position where slider component does not interact with interlock component 1920, and upper portion 1922 is adjacent to or abuts ridge 911 of the housing of distal portion 904, and a second position where slider component 1900 applies an axial force to hinge portion 1926 thereby causing spring portion 1924 to contact guide rail 1901, such that guide rail 1910 causes spring portion 1924 to collapse/compress and interlock component 1920 collapses radially inward such that upper portion 1922 is no longer adjacent to ridge 911. In some embodiments, interlock component 1920 does not include spring portion 1924, such that hinge portion 1926 has sufficient force to maintain upper portion 1922 adjacent to ridge 911, and the axial force applied to hinge portion 1926 by slider component 1900 causes interlock component 1920 to collapse radially inward such that upper portion 1922 is no longer adjacent to ridge 911.

As described above, interface 918 may be required to be fully actuated, e.g., to fully lock the distal, implantable portion of support 200, before additional axial movement between the proximal and distal portions of the handle is permitted. For example, interface 910 may be actuated to cause axial movement of proximal portion 902 relative to distal portion 904 until ridge 911 abuts upper portion 1922. When slider component 1900 is in the first position, additional axial movement between proximal portion 902 and distal portion 904 is not permitted as upper portion 1922 prevents interlock component 1920 from collapsing radially inward due to an axial force applied by ridge 911, and thus upper portion 1922 prevents distal movement of distal portion 904 relative to rod 1910. As described above, as interface 918 is actuated, e.g., rotated, driver 919 operatively coupled thereto moves axially relative to interface 918. Accordingly, interface 918 may be required to be fully actuated, e.g., to fully lock the distal, implantable portion of support 200, before additional axial movement between proximal portion 902 and distal portion 904 is permitted.

For example, when interface 918 is fully actuated, driver 919 moves distally relative to interface 918, thereby abutting and applying an axial force on proximal end of slider component 1900 to cause distal movement of slider component 1900 relative to rod 1910. Slider component 1900 may continue to move distally relative to rod 1910 until interface 918 is fully actuated and the distal end of slider component 1900 abuts and applies an axial force to hinge portion 1926 to thereby collapse interlock component 1920 radially inward, such that upper portion 1922 disengages with ridge 911. Accordingly, upon full actuation of interface 918, such that the distal, implantable portion of support 200 is fully locked, interface 910 may then be further actuated to provide a predefined additional amount of axial movement of proximal portion 902 relative to distal portion 904. Specifically, further actuation of interface 910 will be permitted as upper portion 1922 will no longer prevent proximal movement of interlock component 1920 and rod 1910, and accordingly proximal portion 902, relative to ridge 911 and distal portion 904. Like axial control mechanism 1800, slider component 1900 also may include a retention clip mechanism configured to prevent distal movement of slider component 1900 relative to rod 1910 without application of an axial force thereon, e.g., via a driver operatively coupled to interface 918.

Referring now to FIG. 20, another exemplary mechanism for limiting adjustment of the axial location of the one or more adjustable bends of the heart valve therapeutic device is provided. As shown in FIG. 20, the proximal and distal portions of the handle may be rotatably coupled to each other via rod 2010. Rod 2010 may include safety clip 2012 configured to be collapsed sideways within rod 2010 upon application of a force thereon, e.g., an axial force. For example, a proximal surface of safety clip 2012 may be tapered to facilitate collapse of safety clip 2012 upon application of an axial force thereon. In addition, safety clip 2012 may include protrusion 2014 disposed thereon, such that protrusion 2014 prevents additional axial movement of proximal portion 902 relative to distal portion 904. For example, interface 910 may be actuated to cause axial movement of proximal portion 902 relative to distal portion 904 until ridge 911 abuts protrusion 2014.

As shown in FIG. 20, axial control mechanism 2000, e.g., a slider component, may be slidably disposed within rod 2010. Axial control mechanism 2000 may be slidably moveable between a first position when axial control mechanism 2000 does not interact with safety clip 2012, and a second position where axial control mechanism 2000 applies an axial force to safety clip 2012, thereby causing safety clip 2012 to collapse sideways, such that protrusion 2014 is aligned with opening 913 of ridge 911. As described above, interface 918 may be required to be fully actuated, e.g., to fully lock the distal, implantable portion of support 200, before additional axial movement between proximal portion 902 and distal portion 904 is permitted.

For example, when interface 918 is fully actuated, driver 919 moves distally relative to interface 918, thereby abutting and applying an axial force on proximal end of axial control mechanism 2000 to cause distal movement of axial control mechanism 2000 relative to rod 2010. Axial control mechanism 2000 may continue to move distally relative to rod 2010 until interface 918 is fully actuated and the distal end of axial control mechanism 2000 abuts and applies an axial force to safety clip 2012 to thereby collapse safety clip 2012 sideways, e.g., in a lateral direction relative to the direction of the axial force by axial control mechanism 2000, such that protrusion 2014 is aligned with opening 913 of ridge 911 of the housing of distal portion 904. Accordingly, upon full actuation of interface 918, such that the distal, implantable portion of support 200 is fully locked, interface 910 may then be further actuated to provide a predefined additional amount of axial movement of proximal portion 902 relative to distal portion 904. Specifically, further actuation of interface 910 will be permitted as protrusion 2014 is aligned with opening 913, and thus ridge 911 may move proximally relative to rod 2010 as protrusion 2014 passes through opening 913. Like axial control mechanism 1800, axial control mechanism 2000 also may include a retention clip mechanism configured to prevent distal movement of axial control mechanism 2000 relative to rod 2010 without application of an axial force thereon, e.g., via a driver operatively coupled to interface 918.

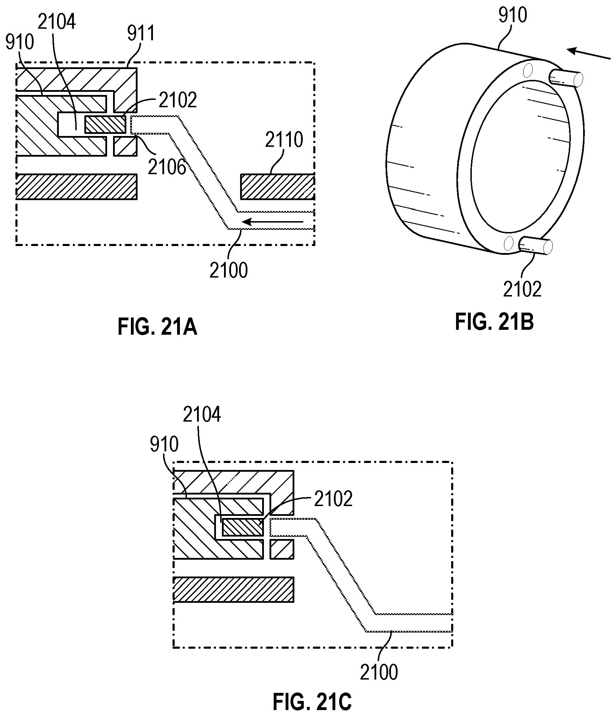

Referring now to FIGS. 21A to 21C, an exemplary actuator interlocking mechanism constructed in accordance with the principles of the present disclosure. Actuation of an actuator of handle 900, e.g., any of the interfaces described herein, may be locked until another corresponding interface is fully actuated. For example, interface 910 may be locked until interface 918 is fully actuated, such that interface 910 may not be actuated until interface 918 is fully actuated. As shown in FIG. 21A, interface 910 may include opening 2104 and ridge 911 of the housing of distal portion 904 may include opening 2106, such that openings 2104 and 2106 are sized and shaped to slidably receive pin 2102 therein. For example, openings 2104 and 2106 may have a geometry corresponding to the geometry of pin 2102 such that when pin 2102 is disposed within at least a portion of both openings 2104 and 2106, pin 2102 prevents rotational movement of interface 910 relative to ridge 911. Moreover, the friction forces between pin 2102 and openings 2104 and 2106 prevent pin 2102 from inadvertently disengaging with openings 2104 and 2106, such that pin 2102 is only removably from openings 2104 and 2106 via the axial force applied by axial control mechanism 2100. Accordingly, while pin is disposed within openings 2104 and 2106, interface 910 is locked relative to ridge 911 and thus cannot be actuated. As shown in FIG. 21B, interface 910 may include more than one opening for slidably receiving more than one pin.

Referring again to FIG. 21A, handle 900 may include axial control mechanism 2100 slidably disposed within rod 2110. Axial control mechanism 2100 may be slidably moveable between a first portion when axial control mechanism 2100 does not engage with pin 2102, and a second position where axial control mechanism 2100 applies an axial force to pin 2102, thereby causing pin 2102 to move further into opening 2104 of interface 910 and out of opening 2106 of ridge 911, as shown in FIG. 21C. Once pin 2102 is no longer disposed within opening 2106 of ridge 911, interface 910 may be freely actuated and rotate relative to ridge 911. Accordingly, interface 918 may be required to be fully actuated, e.g., to fully lock the distal, implantable portion of support 200, before actuation of interface 910 is permitted.

For example, when interface 918 is fully actuated, driver 919 moves distally relative to interface 918, thereby abutting and applying an axial force on proximal end of axial control mechanism 2100 to cause distal movement of axial control mechanism 2100 relative to rod 2110. Axial control mechanism 2100 may continue to move distally relative to rod 2110 until interface 918 is fully actuated and the distal end of axial control mechanism 2100 abuts and applies an axial force to pin 2102 to thereby push pin 2102 further into opening 2104 of interface 910 and out of opening 2106 of ridge 911. Accordingly, upon full actuation of interface 918, such that the distal, implantable portion of support 200 is fully locked, interface 910 may then be actuated to provide axial movement of proximal portion 902 relative to distal portion 904. As will be understood by a person having ordinary skill in the art, the actuator locking mechanism described with regard to FIGS. 21A to 21C may be implemented in other interfaces of handle 900.

Referring now to FIGS. 22A to 22F, an alternative actuation mechanism for the double bend of the heart valve therapeutic devices described herein is provided. As shown in FIG. 22A, support 2200 may include shaping catheter 2201 slidably disposed over body support catheter 2220, the distal end of which may be coupled to prosthetic device 120, such movement of body support catheter 2220 relative to shaping catheter 2201 may adjust the depth of prosthetic valve 120, e.g., relative to the native valve annulus. In some embodiments, support 2200 further may include an elongated rail disposed within a lumen of body support catheter 2220, as described above. Alternatively, support 2200 may not include a separate elongated rail, such that the elongated rail is integrated with the body support catheter or the body support catheter otherwise functions as an elongated rail. Like support 200, support 2200 may have one or more adjustable bends, e.g., first adjustable bend 2202 and second adjustable bend 2204 proximal to first adjustable bend 2202, to facilitate positioning of prosthetic device 120 at the target location across the native valve.

Unlike support 200, first adjustable bend 2202 may be actuated via a handle operatively coupled to support 2200 via a first pull wire mechanism, e.g., first pull wire 2206, to adjust the bend angle of first adjustable bend 2202 along a first bend plane, and second adjustable bend 2204 may be actuated via the handle operatively coupled to support 2200 via a second pull wire mechanism, e.g., second pull wire 2210, to adjust the bend angle of second adjustable bend 2204 along a second bend plane. Unlike support 200, the elongated rail of support 2200 need not comprise preformed bends that define first adjustable bend 2202 and second adjustable bend 2204. Rather, the elongated rail and body support catheter 2220 may be formed of a semi-rigid material configured to bend responsive to actuation of first and/or second pull wires 2206, 2210, as described in further detail below. At least one of the elongated rail, body support catheter 2220, or shaping catheter 2201 may be biased towards a straight, linear configuration, such that actuation of first and/or second pull wires 2206, 2210 applies tension to rigid distal region 2203 and/or rigid middle region 2207, respectively, to thereby adjust the bend angle of first adjustable bend 2202 and/or second adjustable bend 2204, respectively. Accordingly, the elongated rail, body support catheter 2220, and/or shaping catheter 2201 may have a stiffness sufficient to return to its straight, linear configuration upon release of tension of the respective pull wire.

For example, as shown in FIGS. 22A and 22B, shaping catheter 2201 may include rigid distal region 2203, flexible distal region 2205 proximal to rigid distal region 2203, rigid middle region 2207 proximal to flexible distal region 2205, flexible proximal region 2209 proximal to rigid middle region 2207, rigid proximal region 2211 proximal to flexible proximal region 2209, and a lumen extending therethrough sized and shaped to slidably receive body support catheter 2220 therein. A proximal end of support 2200 may be operatively coupled to the handle. Flexible distal and proximal regions 2205, 2209 may be lasercut sections and may be disposed at first and second adjustable bends 2202, 2204, respectively, such that rigid distal region 2203 is disposed distal to first adjustable bend 2202, rigid middle region 2207 is disposed proximal to first adjustable bend 2202 and distal to second adjustable bend 2204, and rigid proximal region 2211 is disposed proximal to second adjustable bend 2204. Alternatively, flexible distal and proximal regions 2205, 2209 may be formed of a braided section between rigid distal region 2203 and rigid middle region 2207 and between rigid middle region 2207 and rigid proximal region 2211, respectively.

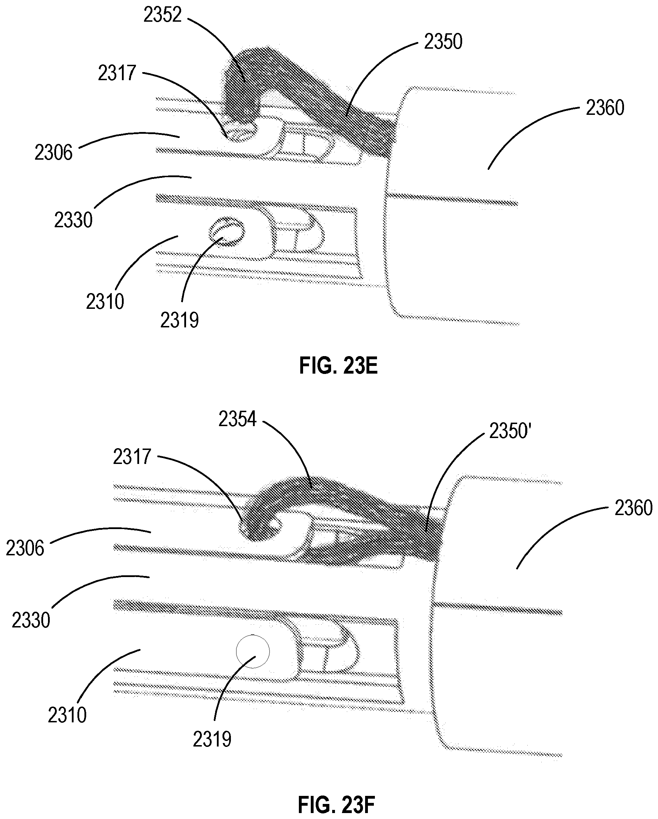

Moreover, support 2200 may include first pull wire 2206 having distal end 2208 coupled to rigid distal region 2203, e.g., via laser welding, and extending proximally along flexible distal region 2205, rigid middle region 2207, flexible proximal region 2209, and rigid proximal region 2211, such that a proximal end of first pull wire 2206 may be operatively coupled to the handle. In addition, support 2200 may include second pull wire 2210 having distal end 2212 coupled to rigid middle region 2207, e.g., via laser welding, and extending proximally along flexible proximal region 2209 and rigid proximal region 2211, such that a proximal end of second pull wire 2210 may be operatively coupled to the handle. First and second pull wires 2206, 2210 may each have an implantable, distal portion having distal ends 2208, 2212, respectively, configured to remain implanted within the patient, and a removable, proximal portion operatively coupled to the handle and configured to be removably coupled to the implantable, distal portion during delivery, and decoupled from the implantable, distal portion upon actuation at the handle, as described in further detail below with regard to FIGS. 23E and 23F. First and/or second pull wires 2206, 2210 may a circular cross section, or alternatively, a flat cross section.

As shown in FIGS. 22B and 22C, support 2200 further may include first guide tube 2214 and second guide tube 2216 disposed between shaping catheter 2201 and body support catheter 2220. First guide tube 2214 may be interrupted (e.g., not present) or flexible at flexible distal region 2207 and flexible proximal region 2209, and similarly second guide tube 2216 may be interrupted or flexible at flexible proximal region 2209. As shown in FIG. 22C, second guide tube 2216 preferably may be offset from first guide tube 2214 along the circumference of shaping catheter 2201 by 90 degrees. As will be understood by a person having ordinary skill in the art, second guide tube 2216 may be offset from first guide tube 2214 along the circumference of shaping catheter 2201 by an angle other than 90 degrees, e.g., 180 degrees, etc. In some embodiments, support 220 may include four guide tubes (e.g., each 90 degrees apart), each configured to receive a respective pull wire, with each pair of opposite facing pull wires configured to be actuated to adjust one of the two adjustable bends, e.g., along perpendicular planes. This may facilitate adjustment of the bend angles of the first and second adjustable bends when the elongated rail is not stiff enough to straighten upon release of tension via the respective pull wire. In some embodiments, first and second guide tubes 2214, 2216 may be integrated with the outer surface of body support catheter 2220, or alternatively, disposed along the outer surface of shaping catheter 2201 and within a jacket.

As shown in FIG. 22B, first guide tube 2214 may extend along rigid proximal region 2211, flexible proximal region 2209, rigid middle region 2207, and flexible distal region 2205, and along at least a portion of rigid distal region 2203, and may have a lumen sized and shaped to receive first pull wire 2206 therethrough to thereby facilitate containment of first pull wire 2206 under tension within first guide tube 2214. Distal end 2208 of first pull wire 2206 may extend out of the distal end of first guide tube 2214, and may be coupled to an inner surface of rigid distal region 2203, e.g., via laser welding. Alternatively, as shown in FIG. 22D, rigid distal region 2203' may have opening 2215 adjacent the distal end of first guide tube 2214', such that distal end 2208' of first pull wire 2206' may extend out of the distal end of first guide tube 2214' and through opening 2215, and may be coupled to the outer surface of rigid distal region 2203', e.g., via laser welding.

Referring again to FIG. 22B, first pull wire 2206 may be slidably coupled to flexible distal region 2205, rigid middle region 2207, flexible proximal region 2209, and rigid proximal region 2211 via first guide tube 2214 in a manner such that, upon actuation of first pull wire 2206, e.g., via the handle, tension is applied to rigid distal region 2203, which causes movement of rigid distal region 2003 relative to at least rigid middle region 2207 along the bend plane of first adjustable bend 2202, thereby adjusting the bend angle of first adjustable bend 2202. Accordingly, the handle may be actuated to move, e.g., retract or release, first pull wire 2206 to thereby apply a lever to rigid distal region 2203 and selectively adjust the angle of rigid distal region 2203 relative to rigid middle region 2207, e.g., by bending flexible distal portion 2205 along the bend plane of first adjustable bend 2202. For example, releasing a tension to first pull wire 2206 may cause rigid distal region 2203 to straighten relative to rigid proximal region 2207. Moreover, the handle may be actuated to disconnect a distal, implantable portion of first pull wire 2206, which will remain implanted within the patient along with the implantable distal portion of support 2200 as described above, from a proximal, removable portion of first pull wire 2206, which may be removed from the patient after the proper positioning and implantation of prosthetic device 120.

Moreover, second guide tube 2216 may extend along rigid proximal region 2211 and flexible proximal region 2209, and at least a portion of rigid middle region 2207, and may have a lumen sized and shaped to receive second pull wire 2210 therethrough to thereby facilitate containment of second pull wire 2210 under tension within second guide tube 2216. Distal end 2212 of second pull wire 2210 may extend out of the distal end of second guide tube 2216, and may be coupled to an inner surface of rigid middle region 2007, e.g., via laser welding. Alternatively, like the embodiment of FIG. 22D, the rigid middle region may have an opening adjacent the distal end of the second guide tube, such that the distal end of the second pull wire may extend out of the distal end of the first guide tube and through the opening, and may be coupled to the outer surface of the rigid middle region, e.g., via laser welding.

In addition, second pull wire 2210 may be slidably coupled to flexible proximal region 2209 and rigid proximal region 2211 via second guide tube 2216 in a manner such that, upon actuation of second pull wire 2210, e.g., via the handle, tension is applied to rigid middle region 2207, which causes movement of rigid middle region 2207 (and accordingly, the components distal of rigid middle region 2207, e.g., rigid distal region 2003) relative to rigid proximal region 2211 along the bend plane of second adjustable bend 2204, thereby adjusting the bend angle of second adjustable bend 2204. Accordingly, the handle may be actuated to move, e.g., retract or release, second pull wire 2210 to thereby apply a lever to rigid middle region 2207 and selectively adjust the angle of rigid middle region 2207 relative to rigid proximal region 2211, e.g., by bending flexible proximal portion 2209 along the bend plane of second adjustable bend 2204. For example, releasing a tension to second pull wire 2210 may cause rigid middle region 2207 to straighten relative to rigid proximal region 2211. Moreover, the handle may be actuated to disconnect a distal, implantable portion of second pull wire 2210, which will remain implanted within the patient along with the implantable distal portion of support 2200 as described above, from a proximal, removable portion of second pull wire 2210, which may be removed from the patient after the proper positioning and implantation of prosthetic device 120.

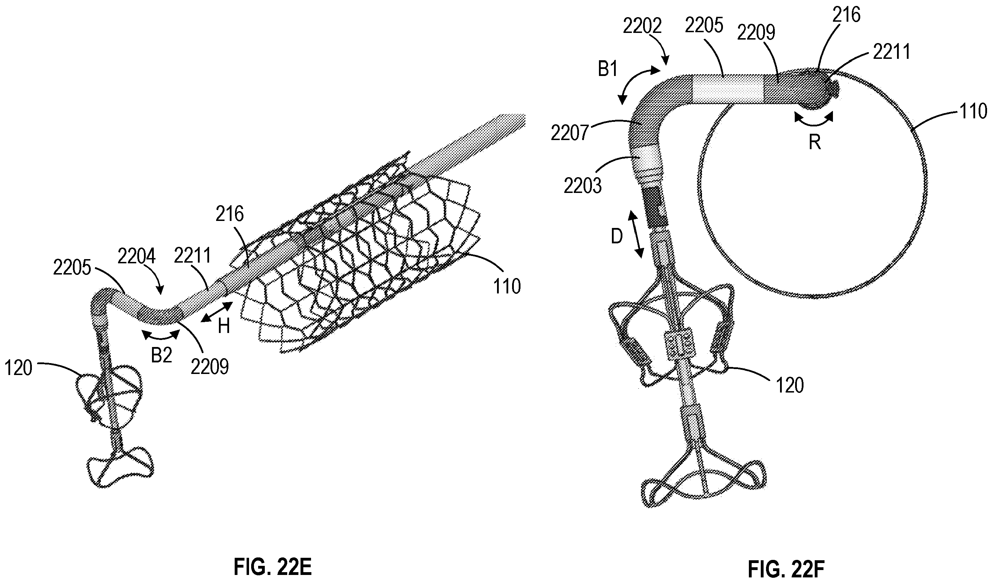

Accordingly, as shown in FIGS. 22E and 22F, in addition to adjusting the rotational angle R of the shaping catheter (and accordingly, all of the components distal to and within the shaping catheter) relative to anchor tube 216 (and accordingly, anchor 110), the depth D of prosthetic device 120 relative to the shaping catheter, e.g., via a body support catheter, and the height H of the shaping catheter (and accordingly, all of the components distal to and within the shaping catheter) relative to anchor tube 216 (and accordingly, anchor 110), as described above, actuation of the first and/or second pull wires may adjust bend angle B1 of first adjustable bend 2204 and/or bend angle B2 of second adjustable bend 2204, respectively, to thereby provide the angle required to position prosthetic device 120 at the target location within the patient, e.g., perpendicular to the tricuspid annulus.

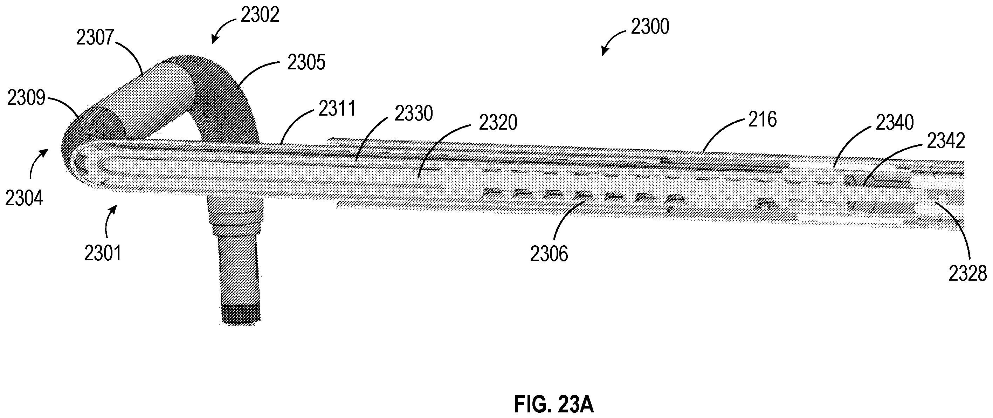

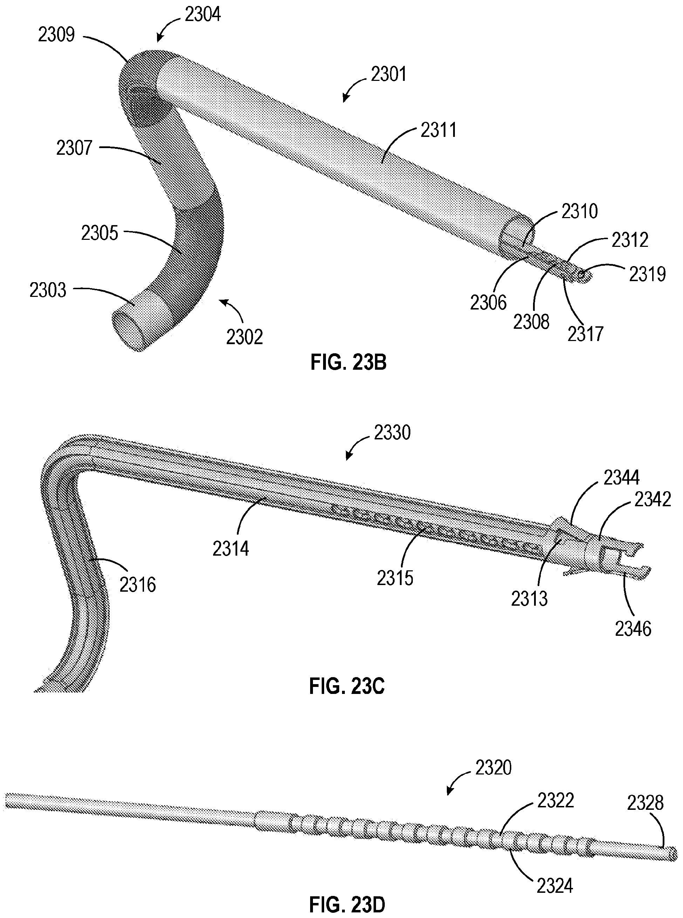

Referring now to FIGS. 23A to 23F, an alternative exemplary pull wire actuation mechanism for the double bend of the heart valve therapeutic devices described herein is provided. As shown in FIG. 23A, support 2300 includes anchor tube 216, shaping catheter 2301 slidably disposed within a lumen of anchor tube 216 (e.g., for adjusting the height of prosthetic device 120, guide shaft 2330 disposed within and coupled to shaping catheter 2301, body support catheter 2320 slidably disposed within guide shaft 2330 and coupled to prosthetic device 120 (e.g., for adjusting the depth of prosthetic device 120). As shown in FIG. 23B, shaping catheter 2301 having first and second adjustable bends 2302, 2304 may be constructed similar to shaping catheter 2201 having first and second adjustable bends 2202, 2204, such that rigid distal region 2303, flexible distal region 2305, rigid middle region 2307, flexible proximal region 2309, and rigid proximal region 2311 correspond with rigid distal region 2203, flexible distal region 2205, rigid middle region 2207, flexible proximal region 2209, and rigid proximal region 2211. Support 2300 includes first and second pull wires 2306, 2310, each having a distal, implantable portion removably coupled to a proximal, removable portion.

Like support 2200, the distal end of the distal, implantable portion of first pull wire 2306 may be coupled to rigid distal region 2303 to thereby adjust the bend angle of first adjustable bend 2302 upon actuation of first pull wire 2306, and the distal end of the distal, implantable portion of second pull wire 2310 may be coupled to rigid middle region 2307 to thereby adjust the bend angle of second adjustable bend 2304 upon actuation of second pull wire 2310. As shown in FIG. 23B, the proximal regions of the distal, implantable portions of first and second pull wires 2306, 2310 may include one or more bumps 2308, 2312, respectively, extending radially inward. In addition, the proximal regions of the distal, implantable portions of first and second pull wires 2306, 2310, e.g., proximal to bumps 2308, 2312, further may include connection points 2317, 2319, respectively, configured to removably couple the distal, implantable portions of first and second pull wires 2306, 2310 to the proximal, removable portions of first and second pull wires 2306, 2310, as described in further detail below with regard to FIGS. 23E and 23F.

As shown in FIG. 23C, guide shaft 2330 may include first guide channel 2314 extending along the outer surface of guide shaft 2330 and sized and shaped to slidably receive first pull wire 2306, and second guide channel 2316 extending along the outer surface of guide shaft 2330 and sized and shaped to slidably receive second pull wire 2310. The proximal regions of first and second guide channels 2314, 2316 may each include a plurality of notches, e.g., notches 2315 of first guide channel 2314, as shown in FIG. 23C, each having a geometry corresponding to bumps 2308 of first pull wire 2306 and bumps 2312 of second pull wire 2310, respectively. Additionally, each of the plurality of notches may have an opening extending therethrough to thereby permit at least a portion of bumps 2308, 2312 to extend therethrough and within the lumen of guide shaft 2330, e.g., when the first and second pull wires 2306, 2310 are in the desired position relative to first and second guide channels 2314, 2316 of guide shaft 2330.

In addition, as shown in FIG. 23C, support 2300 further may include connector 2342 configured to removably couple the implantable distal portion of support 2300 that remains implanted within the patient with the removable proximal portion of support 2300 that may be removed from the patient upon implantation of prosthetic device 120. Like body support catheter connection 227, connector 2342 may include distal interlinking portion 2344 sized and shaped to releasably engage with connection portion 2313, e.g., a T-shaped opening, disposed on the proximal end of guide shaft 2330, and proximal interlinking portion 2346 sized and shaped to engage with a corresponding connection portion disposed on the distal end of the removable proximal portion of support 2300. In some embodiments, proximal interlinking portion 2346 may be coupled to or integrated with the distal end of the removable proximal portion of support 2300. Moreover, distal interlinking portion 2344 may be biased towards an expanded configuration where distal interlinking portion 2344 extends radially outward.

Guide shaft 2330 may comprise a series of rigid and flexible regions that are aligned with rigid distal region 2303, flexible distal region 2305, rigid middle region 2307, flexible proximal region 2309, and rigid proximal region 2311 of shaping catheter 2301 when guide shaft 2330 is disposed within shaping catheter 2301, such that upon actuation of first and/or second pull wires 2306, 2310, the flexible regions of guide shaft 2330 may bend along with flexible distal region 2305 and/or flexible proximal region 2309 at first and second adjustable bends 2302, 2304. Alternatively, guide shaft 2330 may be flexible along its entire length.

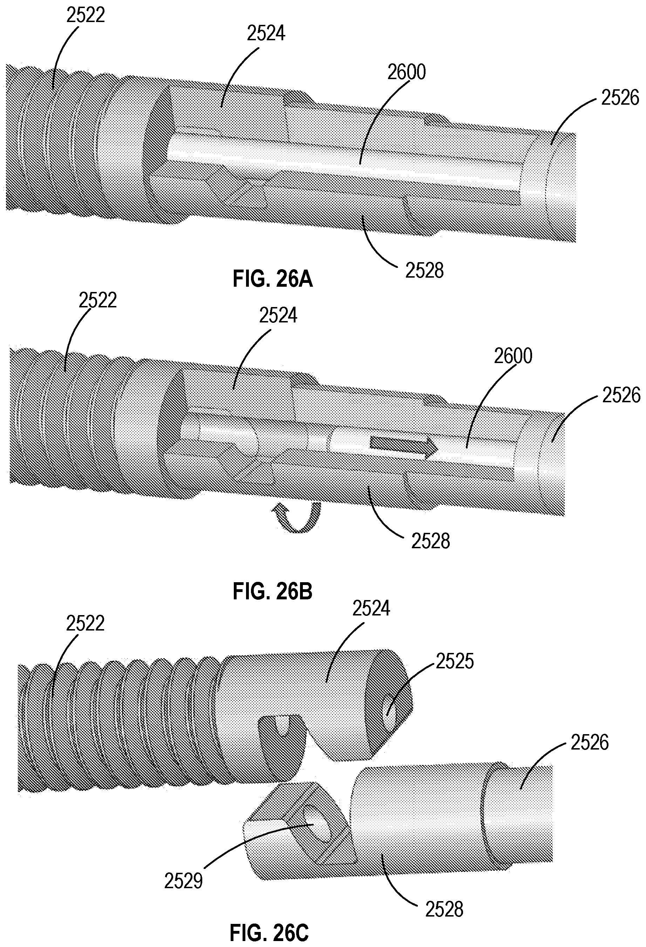

As shown in FIG. 23D, body support catheter 2320 may include a plurality of notches 2322 separated by a plurality of protrusions 2322, e.g., in an alternating manner. Each of notches 2322 may be sized and shaped to receive at least a portion of bumps 2308, 2312, e.g., when bumps 2308, 2312 extend through the plurality of notches of first and second guide channels 2314, 2316 of guide shaft 2330. Body support catheter 2320 may have a polymeric coating. Moreover, the proximal end of the implantable distal portion of body support catheter 2328 may include body support catheter connection 2328 configured to be removable coupled to the distal end of the removable proximal portion of the body support catheter, e.g., via a connection mechanism as described above with reference to FIG. 13B, or alternatively, as described in further detail below with regard to FIGS. 26A to 26C. In some embodiments, body support catheter 2320 further may include a lumen extending therethrough, and sized and shaped to receive an elongated rail, as described above. Alternatively, support 2300 may not include a separate elongated rail, such that the elongated rail is integrated with the body support catheter or the body support catheter otherwise functions as an elongated rail.

Referring again to FIG. 23A, support 2300 further may include pull wire lock 2340 slidably disposed over distal interlinking portion 2344 of connector 2342, thereby maintaining distal interlinking portion 2344 in its collapsed configuration within connection portion 2313 of guide shaft 2330 during delivery of prosthetic device 120. During positioning of prosthetic device 120 within the patient, e.g., relative to the native valve annulus, first and/or second pull wires 2306, 2310 may be actuated as described above to thereby adjust the bend angles of first and/or second adjustable bends 2302, 2304 by moving first and/or second pull wires 2306 axially relative to guide shaft 2330. When first and/or second pull wires 2306 are in their desired position relative to guide shaft 2330, such that the desired tension of first and/or second pull wires 2306 and accordingly the desired bend angles of first and/or second adjustable bends 2302, 2304 are achieved, bumps 2308 of first pull wire 2306 may be disposed through corresponding notches 2315 of first guide channel 2314 of guide shaft 2330 and at least partially disposed within notches 2322 of body support catheter 2320, and bumps 2312 of second pull wire 2310 may be disposed through the corresponding notches of second guide channel 2316 of guide shaft 2330 and at least partially disposed within notches 2322 of body support catheter 2320. Pull wire lock 2340 may then be moved axially in a distal direction, e.g., via actuation at the handle, until pull wire lock 2340 is disposed over the proximal region of first and second pull wires 2306, 2310, e.g., adjacent to bumps 2308, 2312, thereby exposing distal interlinking portion 2344 from within pull wire lock 2340. The stacking between body support catheter 2320, bumps 2308, 2312 of first and second pull wires 2306, 2310, pull wire lock 2340, and anchor tube 216 creates an interference fit that maintains the relative axial position between first and second pull wires 2306, 2310, body support catheter 2320, shaping catheter 2301 via guide shaft 2330, and anchor tube 216, thereby locking the depth of prosthetic valve 120 as well as the bend angles of first and second adjustable bends 2302, 2304. Additionally, exposed distal interlinking portion 2344 may transition to its expanded configuration to thereby permit decoupling of the removable proximal portion of support 2300 from the implantable distal portion of support 2300.

Referring now to FIGS. 23E an 23F, coupling mechanisms for removably coupling the implantable distal portions of the pull wires described herein with respective removable proximal portions of the pull wires are provided. As shown in FIG. 23E, removable proximal portion 2350 may comprise hook portion 2352 configured to releasably engage with connection point 2317 of first pull wire 2306. For example, hook portion 2352 may be pre-formed such that it is biased towards a deflected configuration, e.g., radially outward, and maintained in a collapsed configuration when disposed within, e.g., slidable sleeve 2340. Accordingly, upon retraction of sleeve 2340 to thereby expose hook portion 2352, hook portion 2352 transitions to its deflected configuration and disengages from connection point 2317, such that first pull wire 2306 may remain implanted within the patient. As will be understood by a person having ordinary skill in the art, second pull wire 2210 may utilize a similar hook type coupling mechanism.

As shown in FIG. 23F, removable proximal portion 2350' may comprise loop 2354, such that removable proximal portion 2350' extends from the handle towards and through connection point 2317 of first pull wire 2306, and back to the handle. When first pull wire 2306 is ready to be decoupled from the removable proximal portion, actuation at the handle may release an end of removable proximal portion 2350', such that the released end of removable proximal portion 2350' may be pulled through connection point 2317, such that first pull wire 2306 may remain implanted within the patient. As will be understood by a person having ordinary skill in the art, second pull wire 2210 may utilize a similar loop type coupling mechanism.

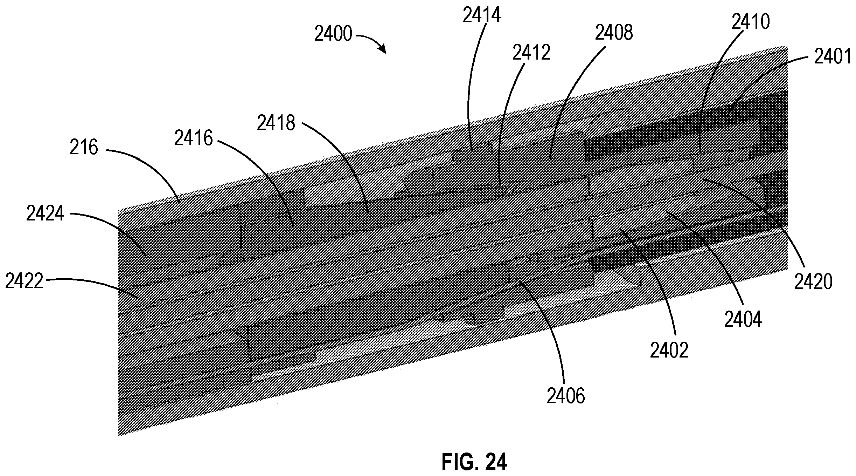

Referring now to FIG. 24, an exemplary depth and pull wire locking mechanism for the double bend of the heart valve therapeutic devices described herein is provided. As shown in FIG. 24, support 2400 may include anchor tube 216 having a lumen sized and shaped to slidably receive at least a portion of shaping catheter 2401 (which may be constructed similar to the double bend shaping catheters described herein, e.g., shaping catheters 2201, 2301, 2501), clamping hub 2408 coupled to shaping catheter 2401, anvil 2416, and anvil pusher 2424. Additionally, clamping hub 2408, anvil 2416, and anvil pusher 2424 each may have a lumen extending therethrough sized and shaped to slidably receive collet 2402 and collet pusher 2422, and collet 2402 and collet pusher 2422 each may have a lumen extending therethrough sized and shaped to slidably receive body support catheter 2420 coupled to prosthetic device 120 (e.g., for adjusting the depth of prosthetic device 120). In some embodiments, body support catheter 2420 further may include a lumen extending therethrough, and sized and shaped to receive an elongated rail, as described above. Alternatively, support 2400 may not include a separate elongated rail, such that the elongated rail is integrated with the body support catheter or the body support catheter otherwise functions as an elongated rail. Moreover, each of shaping catheter 2401, clamping hub 2408, anvil 2416, and anvil pusher 2424 may include one or more channels sized and shaped to receive first and second pull wires therethrough, e.g., pull wire 2406. As shown in FIG. 24, at least a portion of pull wire 2406 may extend between an inner surface of clamping hub 2408 and an outer surface of anvil 2416.

As shown in FIG. 24, clamping hub 2408 may have one or more crush ribs 2414 extending radially outward from an outer surface at a proximal region of clamping hub 2408, and configured to engage with an inner surface of anchor tube 216 via an interference fit when clamping hub 2408 is in its expanded state, as described in further detail below. Accordingly, clamping hub 2408 may include one or more split side openings extending longitudinally along at least the proximal region of clamping hub 2408, and configured to facilitate transitioning of clamping hub 2408 to its expanded state within anchor tube 216. The proximal region of clamping hub 2408 further may have a lumen having tapered portion 2412. In addition, the distal region of clamping hub 2408 may have an outer diameter configured to fit and be fixed within a lumen of shaping catheter 2401, and a lumen having tapered portion 2410.

As shown in FIG. 24, the outer surface of at least the distal region of collet 2402 may have distal tapered portion 2404, which may have a taper angle corresponding with the taper angle of tapered portion 2410 of clamping hub 2408. Additionally, collet 2402 may include one or more split side openings extending longitudinally along at least the proximal region of collet 2402, and configured to facilitate transitioning of the proximal region of collet 2402 to an expanded state within the lumen of clamping hub 2408 as tapered portion 2404 transitions to a collapsed state within tapered portion 2410 of clamping hub 2408, as described in further detail below. Alternatively, collet 2402 may not include one or more split side openings. As shown in FIG. 24, the outer surface of at least the distal region of anvil 2416 may have distal tapered portion 2418, which may have a taper angle corresponding with the taper angle of tapered portion 2412 of clamping hub 2408.

Accordingly, when the desired depth of prosthetic device 120 is achieved, the position of body support catheter 2420 relative to shaping catheter 2401 may be fixed by actuating collet pusher 2422 at the handle to cause collet pusher 2422 to push collet 2402 distally relative to clamping hub 2408. As collet 2402 moves distally within the lumen of clamping hub 2408, tapered portion 2410 of clamping hub 2408 causes at least distal tapered portion 2404 of collet 2402 to collapse radially inward towards body support catheter 2420, thereby fixing the axial position of body support catheter 2420 relative to collet 2402 and of collet 2402 relative to clamping hub 2410 and accordingly, shaping catheter 2401, via interference fit. As described above, collet 2402 may have one or more split side openings which may facilitate transitioning of at least the proximal region of collet 2402 to an expanded state such that it presses against the inner surface of clamping hub 2408 as distal tapered portion 2404 of collet 2402 collapses radially inward over body support catheter 2420, to thereby create a further interference fit between collet 2402 and clamping hub 2408. Collet pusher 2422 may then be removed from the patient, or alternatively, an implantable portion thereof may remain implanted within support 2400 within the patient.

As described above, the pull wires, e.g., pull wire 2406, may be actuated at the handle to adjust the band angles of the first and/or second adjustable bends of support 2400. When the desired bend angles of the first and/or second adjustable bends of support 2400 has been achieved, the position of the pull wires relative to anchor tube 216 may be fixed by actuating anvil pusher 2424 at the handle to cause anvil pusher 2424 to push anvil 2416 distally relative to clamping hub 2408. As anvil 2416, a rigid component, moves distally within the lumen of clamping hub 2408, tapered portion 2412 of clamping hub 2408 causes at least distal tapered portion 2418 of anvil 2416 to press against the pull wires, such that, e.g., pull wire 2406 is fixedly pinned between the outer surface of distal tapered portion 2418 of anvil 2416 and the inner surface of tapered portion 2412 of clamping hub 2408, as shown in FIG. 24. Simultaneously, the distal movement of anvil 2416 relative to tapered portion 2412 of clamping hub 2408 causes the proximal region of clamping hub 2408 to expand radially outward via the split side openings of clamping hub 2408 such that crush ribs 2414 of clamping hub 2408 fixedly engages with the inner surface of anchor tube 216, thereby fixing the axial position of anvil 2416 relative to clamping hub 2408 and of anchor tube 216 relative to clamping hub 2408, and accordingly shaping catheter 2401 and body support catheter 2420 via collet 2402. Anvil pusher 2424 may then be removed from the patient, or alternatively, an implantable portion thereof may remain implanted within support 2400 within the patient.

In some embodiments, instead of having collet 2402 and collet pusher 2422, the outer surface of the distal region of clamping hub 2408 may comprise a collet, e.g., a tapered surface, having a taper angle corresponding with a taper angle of the lumen of shaping catheter 2401 configured to receive the distal region of clamping hub 2408. Accordingly, upon actuation of anvil pusher 2424 at the handle, anvil pusher 2424 may cause anvil 2416 to press against clamping hub 2408 and move clamping hub 2408 distally relative to shaping catheter 2401 such that the tapered surface of the lumen of shaping catheter 2401 engages the tapered outer surface of the distal region of clamping hub 2408 and causes the distal region of clamping hub 2408 to collapse radially inward and clamp against body support catheter 2420, while distal movement of anvil 2416 relative to tapered portion 2412 of clamping hub 2408 causes the proximal region of clamping hub 2406 to expand radially outward via the split side openings of clamping hub 2408 such that crush ribs 2414 fixedly engages with the inner surface of anchor tube 216. Accordingly, locking of the depth of prosthetic device 120 and the bend angles of the first and second adjustable bends of support 2400 may be accomplished in a single action requiring only a single actuation at the handle.

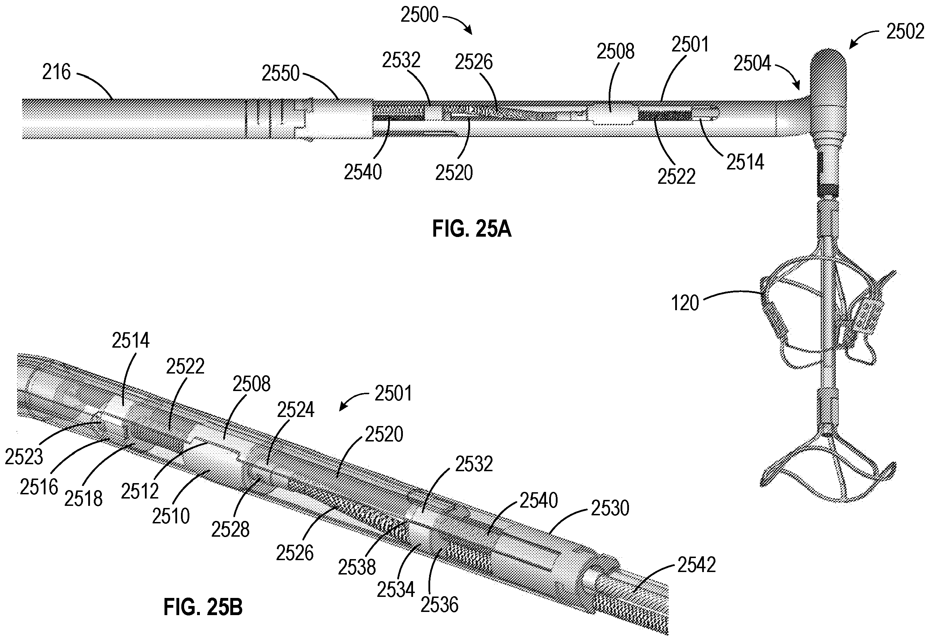

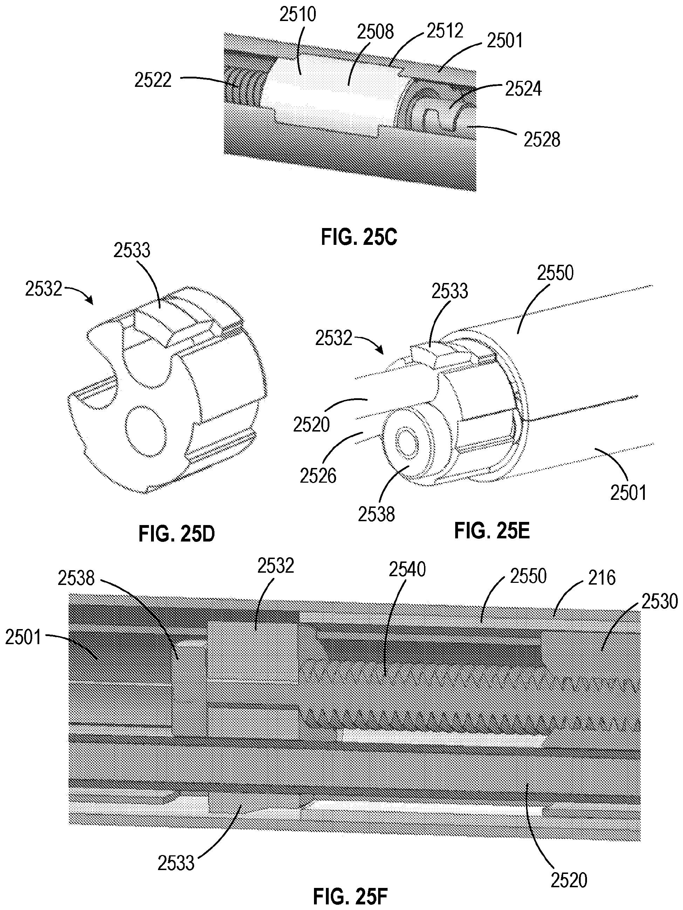

Referring now to FIGS. 25A to 25F, another alternative exemplary pull wire actuation mechanism for the double bend of the heart valve therapeutic devices described herein is provided. As shown in FIG. 25A, support 2500 may include anchor tube 216 having a lumen sized and shaped to slidably receive at least a portion of shaping catheter 2501 having first adjustable bend 2502 and second adjustable bend 2504, anchor tube lock 2550 slidably disposed over shaping catheter 2501, and body support catheter 2520 coupled to prosthetic device 120 (e.g., for adjusting the depth of prosthetic device 120). In some embodiments, body support catheter 2520 further may include a lumen extending therethrough, and sized and shaped to receive an elongated rail, as described above. Alternatively, support 2500 may not include a separate elongated rail, such that the elongated rail is integrated with the body support catheter or the body support catheter otherwise functions as an elongated rail. Support 2500 further may include first guide block 2508 fixedly coupled to shaping catheter 2501, e.g., via protrusion 2510 of first guide block 2508 extending at least partially through opening 2512 having a geometry corresponding to the geometry of protrusion 2510, as shown in FIG. 25C. Additionally or alternatively, the edges of first guide block 2508 may be laser welded to shaping catheter 2501.

Referring again to FIG. 25B, support 2500 further may include second guide block 2530 fixedly coupled to shaping catheter 2501, e.g., at a location proximal to first guide block 2508. In addition, support 2500 may include first collar 2514 slidably disposed within the lumen of shaping catheter 2501 at a location distal to first guide block 2508, and fixedly coupled to a first pull wire (not shown), such that movement of first collar 2514 relative to first guide block 2508 causes movement of the first pull wire relative to shaping catheter 2501, as described in further detail below. For example, first collar 2514 may include protrusion 2516 sized and shaped to move axially within channel 2518 of shaping catheter 2501 in a manner such that the engagement between channel 2518 and protrusion 2516 prevents rotation of first collar 2514 relative to shaping catheter 2501. Support 2500 further may include second collar 2532 slidably disposed within the lumen of shaping catheter 2501 at a location distal to second guide block 2530 and proximal to first guide block 2508, and fixedly coupled to a second pull wire (not shown), such that movement of second collar 2532 relative to second guide block 2530 causes movement of the second pull wire relative to shaping catheter 2501, as described in further detail below. For example, second collar 2532 may include protrusion 2534 sized and shaped to move axially within channel 2536 of shaping catheter 2501 in a manner such that the engagement between channel 2536 and protrusion 2534 prevents rotation of second collar 2532 relative to shaping catheter 2501.

As shown in FIG. 25B, both first guide block 2508 and first collar 2514 may have a first lumen sized and shaped to slidably receive body support catheter 2520 therethrough. In addition, first guide block 2508 may include a second lumen having a threaded inner surface sized and shaped to receive first screw 2522 having a threaded outer surface therethrough, and first collar 2514 may include a second lumen sized and shaped to receive a non-threaded distal portion of first screw 2522. The distal end of the non-threaded distal portion of first screw 2522 may be coupled to first cap 2523 on a distal side of first collar 2514 such that the axial position of first collar 2514 relative to first screw 2522 is fixed. For example, first cap 2523 may be fixedly coupled to first collar 2514 such that first screw 2522 may rotate within and relative to first cap 2523, or alternatively, first cap 2523 may be fixedly coupled to first screw 2522 such that first cap 2523 may rotate relative to first collar 2514 along with first screw 2522. Additionally, the tension provided to first collar 2514 by the first pull wire maintains contact between first collar 2514 and first cap 2523.

Moreover, the proximal end of first screw 2522 may comprise first screw connection 2524 configured to be releasably coupled to first torque cable connection 2528 of first torque cable 2526 on a proximal side of first guide block 2508, e.g., via a connection mechanism such as that described in further detail below with regard to FIGS. 26A to 26C. A proximal end of first torque cable 2526 may be operatively coupled to the handle, such that upon actuation at the handle to rotate first torque cable 2526, first torque cable 2526 may transmit a rotary motion to first screw 2522 via first screw connection 2524 and first torque cable connection 2528. As first guide block 2508 is rotationally and axially fixed relative to shaping catheter 2501, the engagement between the threaded inner surface of first guide block 2508 and the threaded outer surface of first screw 2522 as first screw 2522 is rotated via first torque cable 2526 will cause axial movement of first screw 2522 (and accordingly first collar 2514 via first cap 2523) relative to first guide block 2508 (and accordingly shaping catheter 2501), to thereby move the first pull wire fixedly coupled to first collar 2514. The axial position of the first pull wire (and accordingly the bend angle of the first adjustable bend of support 2500) relative to shaping catheter 2501 is maintained via the screw/thread interface between first screw 2522 and first guide block 2508.

Similarly, as shown in FIG. 25B, both second guide block 2530 and second collar 2532 may have a first lumen sized and shaped to slidably receive body support catheter 2520 therethrough. In addition, both second guide block 2530 and second collar 2532 may have a second lumen sized and shaped to slidably receive first torque cable 2526 therethrough. Moreover, second guide block 2530 may include a third lumen having a threaded inner surface sized and shaped to receive second screw 2540 having a threaded outer surface therethrough, and second collar 2532 may include a third lumen sized and shaped to receive a non-threaded distal portion of second screw 2540. The distal end of the non-threaded distal portion of second screw 2540 may be coupled to second cap 2538 on a distal side of second collar 2532 such that the axial position of second collar 2532 relative to second screw 2540 is fixed. For example, second cap 2538 may be fixedly coupled to second collar 2532 such that second screw 2540 may rotate within and relative to second cap 2538, or alternatively, second cap 2538 may be fixedly coupled to second screw 2540 such that second cap 2538 may rotate relative to second collar 2532 along with second screw 2540. Additionally, the tension provided to second collar 2532 by the second pull wire maintains contact between second collar 2532 and second cap 2538.

Moreover, the proximal end of second screw 2540 may comprise a second screw connection configured to be releasably coupled to a second torque cable connection of second torque cable 2542 on a proximal side of second guide block 2530, e.g., via a connection mechanism such as that described in further detail below with regard to FIGS. 26A to 26C. A proximal end of second torque cable 2542 may be operatively coupled to the handle, such that upon actuation at the handle to rotate second torque cable 2542, second torque cable 2542 may transmit a rotary motion to second screw 2540 via the second screw and torque cable connections. As second guide block 2530 is rotationally and axially fixed relative to shaping catheter 2501, the engagement between the threaded inner surface of second guide block 2530 and the threaded outer surface of second screw 2540 as second screw 2540 is rotated via second torque cable 2542 will cause axial movement of second screw 2540 (and accordingly second collar 2532 via second cap 2538) relative to second guide block 2530 (and accordingly shaping catheter 2501), to thereby move the second pull wire fixedly coupled to second collar 2532. The axial position of the second pull wire (and accordingly the bend angle of the second adjustable bend of support 2500) relative to shaping catheter 2501 is maintained via the screw/thread interface between second screw 2540 and second guide block 2530.

As shown in FIG. 25D, second collar 2532 further may include wedge 2533 extending radially outward from the outer surface of second collar 2532. The inner surface of wedge 2533 may define at least a portion of the lumen of that receives body support catheter 2520. For example, wedge 2533 may be sized and shaped to extend radially outward through a channel of shaping catheter 2501 adjacent to the lumen of second collar 2532 that receives body support catheter 2520. As shown in FIGS. 25E and 25F, anchor tube lock 2550 slidably disposed between shaping catheter 2501 and anchor tube 216, may initially be positioned proximal to wedge 2533. When the desired depth of prosthetic device 120 via the axial position of body support catheter 2520 relative to shaping catheter 2501, as well as the desired bend angles of first and second adjustable bends 2502, 2504 via the axial positions of first and second collars 2514, 2532 relative to first and second guide blocks 2508, 2530 have been achieved, the handle may be actuated to cause distal movement of anchor tube lock 2550 relative second collar 2532 until anchor tube lock 2550 is disposed over wedge 2533, thereby causing wedge 2533 to at least partially collapse radially inward towards body support catheter 2520 while body support catheter 2520 causes wedge 2533 to apply a radially outward force against the inner surface of anchor tube lock 2550, which causes anchor tube lock 2550 to expand and engage with the inner surface of the lumen of anchor tube 216. Accordingly, the axial position of body support catheter 2520 relative to shaping catheter 2501 will be fixed via second screw 2540 and second guide block 2530 and interference fit between wedge 2533 and body support catheter 2520, and the axial position of anchor tube 216 relative to shaping catheter 2501 will be fixed via interference fit between anchor tube lock 2550 and anchor tube 216 and between anchor tube lock 2550 and wedge 2533, which as described above is fixed axially relative to body support catheter 2520.

Referring now to FIGS. 26A to 26C, an exemplary torque cable attachment mechanism is provided. As shown in FIGS. 26A and 26B, first screw connection 2524 of first screw 2522 may be sized and shaped to be releasably coupled to first torque cable connection 2528 of first torque cable 2526. For example, first screw connection 2524 may have a geometry corresponding with the geometry of first torque cable connection 2528 such that, when coupled, first screw 2522 and first torque cable 2526 are coaxial. Moreover, first torque cable 2526 and at least a portion of first screw 2522 may have a lumen extending therethrough sized and shaped to receive rod 2600, and first screw connection 2524 may have opening 2525 and first torque cable connection 2528 may have opening 2529, both sized and shaped to receive rod 2600 therethrough. As shown in FIG. 26A, rod 2600 may be positioned through the lumens and openings of first screw 2522 and first torque cable 2526 when first screw connection 2524 and first torque cable connection 2528 are coupled to thereby prevent disengagement of first screw connection 2524 from first torque cable connection 2528. As shown in FIG. 26B, rod 2600 may be retracted, e.g., via actuation at the handle, to permit disengagement of first screw connection 2524 from first torque cable connection 2528, as shown in FIG. 26C. As will be understood by a person having ordinary skill in the art, the torque cable attachment mechanism may be used to releasably couple other implantable distal components and removable proximal components described herein.

While various illustrative embodiments of the invention are described above, it will be apparent to one skilled in the art that various changes and modifications may be made therein without departing from the invention, within the scope of the claims. The appended claims are intended to cover all such changes and modifications that fall within the true scope of the invention.

## Claims

1. A system for implanting a therapeutic heart valve device (100) at a native heart valve of a patient's heart, the system comprising:
a prosthetic device (120) configured to be implanted at the native heart valve;
a support (200) configured to maintain the prosthetic device (120) at the native heart valve, the support (200) comprising:
a first adjustable bend (202) configured to be actuated to adjust a position of the prosthetic device (120) along a first plane;
a second adjustable bend (204) proximal to the first adjustable bend (202), the second adjustable bend (204) configured to be actuated to adjust the position of the prosthetic device (120) along a second plane; and
an elongated rail (206) configured to be coupled to the prosthetic device (120); and
a handle (900) operatively coupled to the support (200) and comprising one or more actuators (910, 912, 914, 916, 918, 920, 922), the one or more actuators (912) configured to be actuated to independently actuate the first adjustable bend (202) and the second adjustable bend (204).

2. The system of claim 1, wherein the elongated rail (206) comprises a first preformed bend disposed at the first adjustable bend (202).

3. The system of claim 2, wherein the elongated rail (206) comprises a first flexibility, and wherein the support (200) further comprises a first shaping catheter (208) slidably disposed over the elongated rail (206) such that movement of the first shaping catheter (208) over the first preformed bend of the elongated rail (206) adjusts an angle of the first adjustable bend (202), the first shaping catheter (208) having a second flexibility less flexible than the first flexibility.

4. The system of claim 3, wherein the first shaping catheter (208) comprises a distal portion having the second flexibility, and a proximal portion having a third flexibility such that movement of the proximal portion of the first shaping catheter (208) over the second adjustable bend (204) does not adjust an angle of the second adjustable bend (204),
or optionally wherein the elongated rail (206) comprises a second preformed bend disposed at the second adjustable bend (204),
and optionally wherein the support (200) further comprises a second shaping catheter (210) slidably disposed over the first shaping catheter (208) such that movement of the second shaping catheter (210) over the second preformed bend of the elongated rail (206) adjusts an angle of the second adjustable bend (204), the second shaping catheter (210) having a third flexibility less flexible than the second flexibility.

5. The system of claim 3, wherein the support (230) further comprises:
a pull wire catheter (231) comprising a flexible middle portion (235) disposed over the second adjustable bend (204), a rigid distal portion (233) distal to the flexible middle portion (235), a rigid proximal portion (237) proximal to the flexible middle portion (235), and a lumen sized and shaped to slidably receive the first shaping catheter (208) therein; and
a pull wire (236) coupled to the rigid distal portion (233) and extending along the flexible middle portion (235) and the rigid proximal portion (237), the pull wire (236) configured to be actuated to adjust an angle of the rigid distal portion (233) relative to the rigid proximal portion (237) to thereby adjust an angle of the second adjustable bend (204),
and optionally wherein a proximal end of the pull wire (236) is operatively coupled to the handle (900), such that the one or more actuators are configured to be actuated to actuate the pull wire (236), and to disconnect a distal implantation portion of the pull wire (236) from a proximal removable portion of the pull wire (236).

6. The system of claim 1, wherein the support (230) further comprises:
a pull wire catheter (231) comprising a flexible middle portion (235) disposed at the second adjustable bend (234), a rigid distal portion (233) distal to the flexible middle portion (235), a rigid proximal portion (237) proximal to the flexible middle portion (235), and a lumen sized and shaped to slidably receive the elongated rail therein; and
a first pull wire (236) coupled to the rigid distal portion (233) and extending along the flexible middle portion (235) and the rigid proximal portion (237), the first pull wire (236) configured to be actuated to adjust an angle of the rigid distal portion (233) relative to the rigid proximal portion (237) to thereby adjust an angle of the second adjustable bend (234), and optionally wherein the support (230) further comprises:
a second pull wire catheter comprising a second flexible middle portion disposed at the first adjustable bend (232), a second rigid distal portion distal to the second flexible middle portion, a second rigid proximal portion proximal to the second flexible middle portion, and a lumen sized and shaped to receive the pull wire catheter (231) therein; and
a second pull wire coupled to the second rigid distal portion and extending along the second flexible middle portion and the second rigid proximal portion, the second pull wire configured to be actuated to adjust an angle of the second rigid distal portion relative to the second rigid proximal portion to thereby adjust an angle of the first adjustable bend (232).

7. The system of claim 1, wherein the support (2200) further comprises:
a pull wire catheter (2201) comprising a flexible distal portion (2205) disposed at the first adjustable bend (2202), a rigid distal portion (2203) distal to the flexible distal portion (2205), a rigid middle portion (2207) proximal to the flexible distal portion (2205), a flexible proximal portion (2209) disposed at the second adjustable bend (2204) proximal to the rigid middle portion (2207), a rigid proximal portion (2211) proximal to the flexible proximal portion (2209), and a lumen sized and shaped to slidably receive the elongated rail therein;
a first pull wire (2206) coupled to the rigid distal portion (2203) and extending along the flexible distal portion (2205) and the rigid middle portion (2207), the first pull wire (2206) configured to be actuated to adjust an angle of the rigid distal portion (2203) relative to the rigid middle portion (2207) to thereby adjust an angle of the first adjustable bend (2202); and
a second pull wire (2210) coupled to the rigid middle portion (2207) and extending along the flexible proximal portion (2209) and the rigid proximal portion (2211), the second pull wire (2210) configured to be actuated to adjust an angle of the rigid middle portion (2207) relative to the rigid proximal portion (2211) to thereby adjust an angle of the second adjustable bend (2204),
and optionally wherein the elongated rail is biased towards a straight, linear configuration, the elongated rail comprising a stiffness sufficient to cause the elongated rail to straighten upon release of tension by at least one of the first or second pull wires (2206, 2210).

8. The system of claim 7, wherein the pull wire catheter (2201) further comprises:
a first guide tube (2214) extending along the pull wire catheter (2201) and having a distal end adjacent to the rigid distal portion (2203) of the pull wire catheter (2201), the first guide tube (2214) having a lumen configured to receive the first pull wire (2206) therethrough, such that a distal end of the first pull wire (2206) extends beyond the distal end of the first guide tube (2214) and is coupled to the rigid distal portion (2203); and
a second guide tube (2216) extending along the pull wire catheter (2201) and having a distal end adjacent to the rigid middle portion (2207) of the pull wire catheter (2201), the second guide tube (2216) having a lumen configured to receive the second pull wire (2210) therethrough, such that a distal end of the second pull wire (2210) extends beyond the distal end of the second guide tube (2216) and is coupled to the rigid middle portion (2207),
and optionally wherein the first guide tube (2214) is interrupted or flexible at the flexible distal portion (2205) and the flexible proximal portion (2209), and wherein the second guide tube (2216) is interrupted or flexible at the flexible proximal portion (2209).

9. The system of claim 7, wherein the pull wire catheter (2301) further comprises a guide shaft (2330) fixedly disposed within the lumen of the pull wire catheter (2301), the guide shaft (2330) comprising a first channel (2314) sized and shaped to slidably receive the first pull wire (2306) therethrough, a proximal region of the first channel (2314) comprising a first plurality of notches (2315), and a second channel (2316) sized and shaped to slidably receive the second pull wire (2310) therethrough, a proximal region of the second channel (2316) comprising a second plurality of notches,
wherein a proximal region of the first pull wire (2306) comprises one or more first bumps (2308) configured to engage with one or more notches of the first plurality of notches (2315) of the first channel (2314), and
wherein a proximal region of the second pull wire (2310) comprises one or more second bumps (2312) configured to engage with one or more notches of the second plurality of notches of the second channel (2316),
and optionally wherein a proximal region of the elongated rail (2320) comprises a plurality of rail notches (2322) configured to receive at least a portion of the one or more first bumps (2308) of the first pull wire (2306) and at least a portion of the one or more second bumps (2312) of the second pull wire (2310) when the one or more first bumps (2308) are engaged with the one or more notches of the first plurality of notches (2315) and the one or more second bumps (2312) are engaged with the one or more notches of the second plurality of notches, and
optionally wherein the system further comprises a pull wire lock (2340) configured to be actuated to be moved axially until pull wire lock (2340) is disposed over the proximal regions of the first and second pull wires (2306, 2310) to thereby maintain relative axial position between the first and second pull wires (2306, 2310), the elongated rail (2320), the pull wire catheter (2301), and the guide shaft (2330) via interference fit.

10. The system of claim 7, further comprising a stent (110) configured to anchor the support (2400) to a blood vessel coupled to the heart, the stent (110) comprising a stent tube (216) having a lumen sized and shaped to slidably receive the support (200) therein,
and optionally wherein the system further comprises:
a clamping hub (2408) coupled to a proximal portion of the pull wire catheter (2401) and slidably disposed within the lumen of the stent tube (216), the clamping hub (2408) having a lumen comprising a tapered distal portion (2410) and a tapered proximal portion (2412), the clamping hub (2408) comprising one or more crush ribs (2414) extending radially outward from an outer surface of the clamping hub (2408);
an anvil (2416) slidably disposed within the lumen of the stent tube (216) proximal to the clamping hub (2408), the anvil (2416) comprising a tapered distal portion (2418) configured to be at least partially received by the tapered proximal portion (2412) of the lumen of the clamping hub (2408);
a collet (2402) slidably disposed within the lumen of the clamping hub (2408), the collet (2402) having a lumen sized and shaped to slidably receive the elongated rail therethrough, the collet (2402) comprising a tapered distal portion (2404) configured to be at least partially received by the tapered distal portion (2410) of the lumen of the clamping hub (2408);
a collet pusher (2422) slidably disposed within a lumen of the anvil (2416) and at least a portion of the lumen of the clamping hub (2408) proximal to the collet (2402), the collet pusher (2422) configured to be actuated to move the collet (2402) distally relative to the clamping hub (2408); and
an anvil pusher (2424) slidable disposed within the lumen of the stent tube (216) proximal to the anvil (2416), the anvil pusher (2424) configured to be actuated to move the anvil (2416) distally relative to the clamping hub (2408),
wherein the first and second pull wires (2406) extend between an outer surface of the tapered distal portion (2418) of the anvil (2416) and an inner surface of the tapered proximal portion (2412) of the lumen of the clamping hub (2408),
wherein, upon distal movement of the collet (2402) relative to the clamping hub (2408), the tapered distal portion (2410) of the lumen of the clamping hub (2408) causes the tapered distal portion (2404) of the collet (2402) to clamp the elongated rail (206) and fix an axial position of the elongated rail relative to the clamping hub (2408), and
wherein, upon distal movement of the anvil (2416) relative to the clamping hub (2408), the tapered distal portion (2418) of the anvil (2416) clamps the first and second pull wires (2406) between the outer surface of the tapered distal portion (2418) of the anvil (2416) and the inner surface of the tapered proximal portion (2412) of the lumen of the clamping hub (2408), and causes the one or more crush ribs (2414) of the clamping hub (2408) to expand radially outward and engage the stent tube (216) to thereby fix an axial position of the clamping hub (2408) relative to the stent tube (216).

11. The system of claim 7, further comprising:
a stent (110) configured to anchor the support (2500) to a blood vessel coupled to the heart, the stent (110) comprising a stent tube (216) having a lumen sized and shaped to slidably receive the support (2500) therein, and optionally, wherein the system further comprises:
a first guide block (2508) disposed within the lumen of the pull wire catheter (2501), the first guide block (2508) fixedly coupled to the pull wire catheter (2501) and comprising a first threaded lumen;
a first collar (2514) slidably disposed within the lumen of the pull wire catheter (2501) distal to the first guide block (2508) and coupled to the first pull wire;
a first screw (2522) having a first threaded surface, a distal end coupled to the first collar (2514), and a proximal end configured to be releasably coupled to a first torque cable (2526) configured to transmit rotary motion to the first screw (2522), at least a portion of the first screw (2522) disposed within the first threaded lumen of the first guide block (2508);
a second guide block (2530) fixedly coupled to the pull wire catheter (2501) proximal to the first guide block (2508), the second guide block (2530) comprising a second threaded lumen;
a second collar (2532) slidably disposed within the lumen of the pull wire catheter (2501) distal to second guide block (2530) and coupled to the second pull wire, the second collar (2532) comprising a wedge (2533) extending radially outward from an outer surface of the second collar (2532);
a second screw (2540) having a second threaded surface, a distal end coupled to the second collar (2532), and a proximal end configured to be releasably coupled to a second torque cable (2542) configured to transmit rotary motion to the second screw (2540), at least a portion of the second screw (2540) disposed within the second threaded lumen of the second guide block (2530); and
a stent tube lock (2550) slidably disposed between the stent tube (216) and the pull wire catheter (2501), the stent tube lock (2550) configured to be actuated to move distally relative to the pull wire catheter (2501) and cover the wedge (2533) of the second collar (2532), such that the wedge (2533) causes the stent tube lock (2550) to expand radially and engage the stent tube (216) to thereby fix an axial position of the pull wire catheter (2501) relative to the stent tube (216).

12. The system of claim 11, wherein the second collar (2532) comprises a lumen at least partially defined by the wedge (2533), the lumen sized and shaped to receive the elongated rail therethrough, and wherein, when the stent tube lock (2550) covers the wedge (2533), the wedge (2533) collapses radially inward against the elongated rail to thereby fix an axial position of the elongated rail relative to the pull wire catheter (2501) and the stent tube (216),
or optionally wherein the proximal end of the first screw (2522) comprises a first screw connection (2524) configured to releasably interlock with a first torque cable (2526) connection at a distal end of the first torque cable (2526), the first screw connection (2524) and the first torque cable connection (2528) configured to remain coupled via a rod (2600) disposed through the first screw connection (2524) and the first torque cable connection (2528) when the first screw connection (2524) and the first torque cable connection (2528) are interlocked, and wherein the rod (2600) is configured to be removed from at least the first screw connection (2524) to permit decoupling of the first screw connection (2524) and the first torque cable connection (2528),
or optionally wherein the proximal end of the second screw (2540) comprises a second screw connection configured to releasably interlock with a second torque cable connection at a distal end of the second torque cable (2542), the second screw connection and the second torque cable connection configured to remain coupled via a rod (2600) disposed through the second screw connection and the second torque cable connection when the second screw connection and the second torque cable connection are interlocked, and wherein the rod (2600) is configured to be removed from at least the second screw connection to permit decoupling of the second screw connection and the second torque cable connection.

13. The system of claim 2, wherein the elongated rail (220) comprises variable flexibility along its length.

14. The system of claim 13, wherein at least a portion of the elongated rail (220) comprises a first cross-sectional area, and wherein at least a portion of the first preformed bend (222) comprises a second cross-sectional area smaller than the first cross-sectional area, thereby increasing flexibility at the first preformed bend (222),
or optionally wherein the elongated rail (220) comprises a second preformed bend (224) disposed at the second adjustable bend (204), wherein at least a portion of the elongated rail (220) comprises a first cross-sectional area, and wherein at least a portion of the second preformed bend (224) comprises a second cross-sectional area smaller than the first cross-sectional area, thereby increasing flexibility at the second preformed bend (224),
or optionally wherein at least a portion of the elongated rail (220) comprises a first cross-sectional area, and wherein at least a portion of the elongated rail (220) distal to the first preformed bend (222) comprises a second cross-sectional area smaller than the first cross-sectional area, thereby increasing flexibility at the portion of the elongated rail (220) distal to the first preformed bend (222).

15. The system of claim 13, wherein at least a portion of the elongated rail (220) comprises a circular cross-sectional area having a first flexibility, and wherein at least another portion of the elongated rail (220) comprises a non-circular cross-sectional area having a second flexibility more flexible than the first flexibility,
and optionally wherein the first adjustable bend (202) comprises the at least another portion of the elongated rail (220) comprising the non-circular cross-sectional area, such that rigidity of the elongated rail (220) is maintained in a plane perpendicular to the first plane, while flexibility of the elongated rail (220) is increased along the first plane,
or optionally wherein the second adjustable bend (204) comprises the at least another portion of the elongated rail (220) comprising the non-circular cross-sectional area, such that rigidity of the elongated rail (220) is maintained in a plane perpendicular to the second plane, while flexibility of the elongated rail (220) is increased along the second plane.

16. The system of claim 1, wherein an orientation of the first plane is dependent on an angle of the second adjustable bend (204) within the second plane.

17. The system of claim 1, further comprising a stent (110) configured to anchor the support (200) to a blood vessel coupled to the heart.

18. The system of claim 17, wherein the stent (110) comprises a stent tube (1622) having a lumen sized and shaped to slidably receive the support (200) therein, the stent tube (1622) having one or more flexible leaves (1624) configured to transition between a radially collapsed state and a radially expanded state, the one or more flexible leaves (1624) biased toward the radially collapsed state, the system further comprising:
a disconnect catheter (1628) having a tab (1630) configured to be actuated to move from a first position where the tab (1630) engages with and maintains the one or more flexible leaves (1624) in the radially expanded state and a second position where the tab (1630) disengages with the one or more flexible leaves (1624) to permit the one or more flexible leaves (1624) to transition to the radially collapsed state to clamp the support (200).

19. The system of claim 18, wherein the stent tube (1622) further comprises a retainer (1632) configured to maintain the tab (1630) in the first position,
or optionally wherein a portion of the one or more flexible leaves (1624) that contact the support (200) in the radially collapsed state comprises teeth configured to increase friction between the one or more flexible leaves (1624) and the support (200),
or optionally wherein a base of the one or more flexible leaves (1624) extends from the stent tube (1622) via a hinged portion (1626),
or optionally wherein the disconnect catheter (1628) comprises a lumen sized and shaped to slidably receive at least a portion of the stent tube (200) therein.

20. The system of claim 1, wherein the support (200) further comprises a body support catheter (212) having a distal end coupled to the prosthetic device (120), a proximal end operatively coupled to the handle (900), and a lumen configured to slidably receive the elongated rail (206), the body support catheter (212) configured to be actuated via the handle (900) to move relative to the elongated rail (206) to adjust an axial position of the prosthetic device (120) relative to the support (200).

## Patentansprüche

1. System zur Implantation einer therapeutischen Herzklappenvorrichtung (100) an einer nativen Herzklappe des Herzens eines Patienten, wobei das System Folgendes umfasst:
eine Prothesevorrichtung (120), die zur Implantation an der nativen Herzklappe konfiguriert ist;
einen Träger (200), der zum Halten der Prothesevorrichtung (120) an der nativen Herzklappe konfiguriert ist, wobei der Träger (200) Folgendes umfasst:
eine erste einstellbare Biegung (202), die zur Betätigung konfiguriert ist, um eine Position der Prothesevorrichtung (120) entlang einer ersten Ebene einzustellen;
eine zweite einstellbare Biegung (204) proximal zu der ersten einstellbaren Biegung (202), wobei die zweite einstellbare Biegung (204) zur Betätigung konfiguriert ist, um die Position der Prothesevorrichtung (120) entlang einer zweiten Ebene einzustellen; und
eine längliche Stange (206), die zur Verbindung mit der Prothesevorrichtung (120) konfiguriert ist; und
einen Griff (900), der funktionsfähig mit dem Träger (200) verbunden ist und ein oder mehrere Betätigungselemente (910, 912, 914, 916, 918, 920, 922) umfasst, wobei das eine oder die mehreren Betätigungselemente (912) zur Betätigung konfiguriert sind, um die erste einstellbare Biegung (202) und die zweite einstellbare Biegung (204) unabhängig zu betätigen.

2. System nach Anspruch 1, wobei die längliche Stange (206) eine erste vorgeformte Biegung umfasst, die an der ersten einstellbaren Biegung (202) angeordnet ist.

3. System nach Anspruch 2, wobei die längliche Stange (206) eine erste Flexibilität umfasst und wobei der Träger (200) weiter einen ersten formgebenden Katheter (208) umfasst, der über der länglichen Stange (206) verschiebbar angeordnet ist, sodass eine Bewegung des ersten formgebenden Katheters (208) über die erste vorgeformte Biegung der länglichen Stange (206) einen Winkel der ersten einstellbaren Biegung (202) einstellt, wobei der erste formgebende Katheter (208) eine zweite Flexibilität aufweist, die weniger flexibel ist als die erste Flexibilität.

4. System nach Anspruch 3, wobei der erste formgebende Katheter (208) Folgendes umfasst: einen distalen Teil, der die zweite Flexibilität aufweist, und einen proximalen Teil, der eine dritte Flexibilität aufweist, sodass eine Bewegung des proximalen Teils des ersten formgebenden Katheters (208) über die zweite einstellbare Biegung (204) nicht einen Winkel der zweiten einstellbaren Biegung (204) einstellt,
oder optional wobei die längliche Stange (206) eine zweite vorgeformte Biegung umfasst, die an der zweiten einstellbaren Biegung (204) angeordnet ist,
und optional wobei der Träger (200) weiter einen zweiten formgebenden Katheter (210) umfasst, der über dem ersten formgebenden Katheter (208) verschiebbar angeordnet ist, sodass eine Bewegung des zweiten formgebenden Katheters (210) über die zweite vorgeformte Biegung der länglichen Stange (206) einen Winkel der zweiten einstellbaren Biegung (204) einstellt, wobei der zweite formgebende Katheter (210) eine dritte Flexibilität aufweist, die weniger flexibel ist als die zweite Flexibilität.

5. System nach Anspruch 3, wobei der Träger (230) weiter Folgendes umfasst:
einen Zugdraht-Katheter (231), der Folgendes umfasst: einen flexiblen Mittelteil (235), der über der zweiten einstellbaren Biegung (204) angeordnet ist, einen starren distalen Teil (233) distal zu dem flexiblen Mittelteil (235), einen starren proximalen Teil (237) proximal zu dem flexiblen Mittelteil (235) und ein Lumen, das zur verschiebbaren Aufnahme des ersten formgebenden Katheters (208) darin dimensioniert und geformt ist; und
einen Zugdraht (236), der mit dem starren distalen Teil (233) verbunden ist und sich entlang des flexiblen Mittelteils (235) und des starren proximalen Teils (237) erstreckt, wobei der Zugdraht (236) zur Betätigung konfiguriert ist, um einen Winkel des starren distalen Teils (233) in Bezug auf den starren proximalen Teil (237) einzustellen, um dadurch einen Winkel der zweiten einstellbaren Biegung (204) einzustellen,
und optional wobei ein proximales Ende des Zugdrahts (236) funktionsfähig mit dem Griff (900) verbunden ist, sodass das eine oder die mehreren Betätigungselemente zur Betätigung konfiguriert sind, um den Zugdraht (236) zu betätigen und ein distales Implantationsteil des Zugdrahts (236) von einem proximalen entfernbaren Teil des Zugdrahts (236) zu trennen.

6. System nach Anspruch 1, wobei der Träger (230) weiter Folgendes umfasst:
einen Zugdraht-Katheter (231), der Folgendes umfasst: einen flexiblen Mittelteil (235), der an der zweiten einstellbaren Biegung (234) angeordnet ist, einen starren distalen Teil (233) distal zu dem flexiblen Mittelteil (235), einen starren proximalen Teil (237) proximal zu dem flexiblen Mittelteil (235) und ein Lumen, das zur verschiebbaren Aufnahme der länglichen Stange darin dimensioniert und geformt ist; und
einen ersten Zugdraht (236), der mit dem starren distalen Teil (233) verbunden ist und sich entlang des flexiblen Mittelteils (235) und des starren proximalen Teils (237) erstreckt, wobei der erste Zugdraht (236) zur Betätigung konfiguriert ist, um einen Winkel des starren distalen Teils (233) in Bezug auf den starren proximalen Teil (237) einzustellen, um dadurch einen Winkel der zweiten einstellbaren Biegung (234) einzustellen, und optional wobei der Träger (230) weiter Folgendes umfasst:
einen zweiten Zugdraht-Katheter, der Folgendes umfasst: einen zweiten flexiblen Mittelteil, der an der ersten einstellbaren Biegung (232) angeordnet ist, einen zweiten starren distalen Teil distal zu dem zweiten flexiblen Mittelteil, einen zweiten starren proximalen Teil proximal zu dem zweiten flexiblen Mittelteil und ein Lumen, das zur Aufnahme des Zugdraht-Katheters (231) darin dimensioniert und geformt ist; und
einen zweiten Zugdraht, der mit dem zweiten starren distalen Teil verbunden ist und sich entlang des zweiten flexiblen Mittelteils und des zweiten starren proximalen Teils erstreckt, wobei der zweite Zugdraht zur Betätigung konfiguriert ist, um einen Winkel des zweiten starren distalen Teils in Bezug auf den zweiten starren proximalen Teil einzustellen, um dadurch einen Winkel der ersten einstellbaren Biegung (232) einzustellen.

7. System nach Anspruch 1, wobei der Träger (2200) weiter Folgendes umfasst:
einen Zugdraht-Katheter (2201), der Folgendes umfasst: einen flexiblen distalen Teil (2205), der an der ersten einstellbaren Biegung (2202) angeordnet ist, einen starren distalen Teil (2203) distal zu dem flexiblen distalen Teil (2205), einen starren Mittelteil (2207) proximal zu dem flexiblen distalen Teil (2205), einen flexiblen proximalen Teil (2209), der an der zweiten einstellbaren Biegung (2204) proximal zu dem starren Mittelteil (2207) angeordnet ist, einen starren proximalen Teil (2211) proximal zu dem flexiblen proximalen Teil (2209) und ein Lumen, das zur verschiebbaren Aufnahme der länglichen Stange darin dimensioniert und geformt ist;
einen ersten Zugdraht (2206), der mit dem starren distalen Teil (2203) verbunden ist und sich entlang des flexiblen distalen Teils (2205) und des starren Mittelteils (2207) erstreckt, wobei der erste Zugdraht (2206) zur Betätigung konfiguriert ist, um einen Winkel des starren distalen Teils (2203) in Bezug auf den starren Mittelteil (2207) einzustellen, um dadurch einen Winkel der ersten einstellbaren Biegung (2202) einzustellen; und
einen zweiten Zugdraht (2210), der mit dem starren Mittelteil (2207) verbunden ist und sich entlang des flexiblen proximalen Teils (2209) und des starren proximalen Teils (2211) erstreckt, wobei der zweite Zugdraht (2210) zur Betätigung konfiguriert ist, um einen Winkel des starren Mittelteils (2207) in Bezug auf den starren proximalen Teil (2211) einzustellen, um dadurch einen Winkel der zweiten einstellbaren Biegung (2204) einzustellen,
und optional wobei die längliche Stange zu einer geraden linearen Konfiguration vorgespannt ist, die längliche Stange eine Steifigkeit umfasst, die ausreicht, um zu bewirken, dass sich die längliche Stange bei Freisetzung von Spannung durch mindestens einen des ersten oder zweiten Zugdrahts (2206, 2210) gerade ausrichtet.

8. System nach Anspruch 7, wobei der Zugdraht-Katheter (2201) weiter Folgendes umfasst:
ein erstes Führungsrohr (2214), das sich entlang des Zugdraht-Katheters (2201) erstreckt und ein distales Ende angrenzend an den starren distalen Teil (2203) des Zugdraht-Katheters (2201) aufweist, wobei das erste Führungsrohr (2214) ein Lumen aufweist, das zur Aufnahme des ersten Zugdrahts (2206) dahindurch konfiguriert ist, sodass sich ein distales Ende des ersten Zugdrahts (2206) über das distale Ende des ersten Führungsrohrs (2214) hinaus erstreckt und mit dem starren distalen Teil (2203) verbunden ist; und
ein zweites Führungsrohr (2216), das sich entlang des Zugdraht-Katheters (2201) erstreckt und ein distales Ende angrenzend an den starren Mittelteil (2207) des Zugdraht-Katheters (2201) aufweist, wobei das zweite Führungsrohr (2216) ein Lumen aufweist, das zur Aufnahme des zweiten Zugdrahts (2210) dahindurch konfiguriert ist, sodass sich ein distales Ende des zweiten Zugdrahts (2210) über das distale Ende des zweiten Führungsrohrs (2216) hinaus erstreckt und mit dem starren Mittelteil (2207) verbunden ist;
und optional wobei das erste Führungsrohr (2214) unterbrochen oder flexibel an dem flexiblen distalen Teil (2205) und dem flexiblen proximalen Teil (2209) ist und wobei das zweite Führungsrohr (2216) unterbrochen oder flexibel an dem flexiblen proximalen Teil (2209) ist.

9. System nach Anspruch 7, wobei der Zugdraht-Katheter (2301) weiter einen Führungsschaft (2330) umfasst, der innerhalb des Lumens des Zugdraht-Katheters (2301) fest angeordnet ist, wobei der Führungsschaft (2330) Folgendes umfasst: einen ersten Kanal (2314), der zur verschiebbaren Aufnahme des ersten Zugdrahts (2306) dahindurch dimensioniert und geformt ist, wobei ein proximaler Bereich des ersten Kanals (2314) eine erste Vielzahl von Einkerbungen (2315) umfasst, und einen zweiten Kanal (2316), der zur verschiebbaren Aufnahme des zweiten Zugdrahts (2310) dahindurch dimensioniert und geformt ist, wobei ein proximaler Bereich des zweiten Kanals (2316) eine zweite Vielzahl von Einkerbungen umfasst,
wobei ein proximaler Bereich des ersten Zugdrahts (2306) eine oder mehrere erste Erhebungen (2308) umfasst, die zum Eingreifen in eine oder mehrere Einkerbungen der ersten Vielzahl von Einkerbungen (2315) des ersten Kanals (2314) konfiguriert sind, und
wobei ein proximaler Bereich des zweiten Zugdrahts (2310) eine oder mehrere zweite Erhebungen (2312) umfasst, die zum Eingreifen in eine oder mehrere Einkerbungen der zweiten Vielzahl von Einkerbungen des zweiten Kanals (2316) konfiguriert sind,
und optional wobei ein proximaler Bereich der länglichen Stange (2320) eine Vielzahl von Stangeneinkerbungen (2322) umfasst, die zur Aufnahme von zumindest einem Teil der einen oder mehreren ersten Erhebungen (2308) des ersten Zugdrahts (2306) und zumindest einem Teil der einen oder mehreren zweiten Erhebungen (2312) des zweiten Zugdrahts (2310) konfiguriert sind, wenn die eine oder mehreren ersten Erhebungen (2308) in die eine oder mehreren Einkerbungen der ersten Vielzahl von Einkerbungen (2315) eingreifen und die eine oder mehreren zweiten Erhebungen (2312) in die eine oder mehreren Einkerbungen der zweiten Vielzahl von Einkerbungen eingreifen, und
optional wobei das System weiter ein Zugdraht-Verriegelungselement (2340) umfasst, das zur Betätigung konfiguriert ist, um axial bewegt zu werden, bis das Zugdraht-Verriegelungselement (2340) über den proximalen Bereichen des ersten und zweiten Zugdrahts (2306, 2310) angeordnet ist, um dadurch eine relative axiale Position zwischen dem ersten und zweiten Zugdraht (2306, 2310), der länglichen Stange (2320), dem Zugdraht-Katheter (2301) und dem Führungsschaft (2330) über eine Presspassung zu halten.

10. System nach Anspruch 7, das weiter einen Stent (110) umfasst, der zur Verankerung des Trägers (2400) an einem Blutgefäß, das mit dem Herzen verbunden ist, konfiguriert ist, wobei der Stent (110) ein Stent-Röhrchen (216) umfasst, das ein Lumen aufweist, das zur verschiebbaren Aufnahme des Trägers (200) darin dimensioniert und geformt ist,
und optional wobei das System weiter Folgendes umfasst:
eine Klemmnabe (2408), die mit einem proximalen Teil des Zugdraht-Katheters (2401) verbunden ist und innerhalb des Lumens des Stents-Röhrchens (216) verschiebbar angeordnet ist, wobei die Klemmnabe (2408) ein Lumen aufweist, das einen verjüngten distalen Teil (2410) und einen verjüngten proximalen Teil (2412) umfasst, wobei die Klemmnabe (2408) eine oder mehrere Quetschrippen (2414) umfasst, die sich radial nach außen von einer äußeren Oberfläche der Klemmnabe (2408) erstrecken;
einen Amboss (2416), der innerhalb des Lumens des Stent-Röhrchens (216) proximal zu der Klemmnabe (2408) verschiebbar angeordnet ist, wobei der Amboss (2416) einen verjüngten distalen Teil (2418) umfasst, der zur zumindest teilweisen Aufnahme durch den verjüngten proximalen Teil (2412) des Lumens der Klemmnabe (2408) konfiguriert ist;
eine Klemmhülse (2402), die innerhalb des Lumens der Klemmnabe (2408) verschiebbar angeordnet ist, wobei die Klemmhülse (2402) ein Lumen aufweist, das zur verschiebbaren Aufnahme der länglichen Stange dahindurch dimensioniert und geformt ist, wobei die Klemmhülse (2402) einen verjüngten distalen Teil (2404) umfasst, der zur zumindest teilweisen Aufnahme durch den verjüngten distalen Teil (2410) des Lumens der Klemmnabe (2408) konfiguriert ist;
ein Klemmhülsenschieber (2422), der innerhalb eines Lumens des Ambosses (2416) und zumindest eines Teils des Lumens der Klemmnabe (2408) proximal zu der Klemmhülse (2402) verschiebbar angeordnet ist, wobei der Klemmhülsenschieber (2422) zur Betätigung konfiguriert ist, um die Klemmhülse (2402) distal relativ zu der Klemmnabe (2408) zu bewegen; und
einen Ambossschieber (2424), der innerhalb des Lumens des Stent-Röhrchens (216) proximal zu dem Amboss (2416) verschiebbar angeordnet ist, wobei der Ambossschieber (2424) zur Betätigung konfiguriert ist, um den Amboss (2416) distal relativ zu der Klemmnabe (2408) zu bewegen,
wobei sich der erste und zweite Zugdraht (2406) zwischen einer äußeren Oberfläche des verjüngten distalen Teils (2418) des Ambosses und einer inneren Oberfläche des verjüngten proximalen Teils (2412) des Lumens der Klemmnabe (2408) erstrecken,
wobei bei distaler Bewegung der Klemmhülse (2402) relativ zu der Klemmnabe (2408) der verjüngte distale Teil (2410) des Lumens der Klemmnabe (2408) bewirkt, dass der verjüngte distale Teil (2404) der Klemmhülse (2402) die längliche Stange (206) einklemmt und eine axiale Position der länglichen Stange in Bezug auf die Klemmnabe (2408) fixiert, und
wobei bei distaler Bewegung des Ambosses (2416) relativ zu der Klemmnabe (2408) der verjüngte distale Teil (2418) des Ambosses (2416) den ersten und zweiten Zugdraht (2406) zwischen der äußeren Oberfläche des verjüngten distalen Teils (2418) des Ambosses (2416) und der inneren Oberfläche des verjüngten proximalen Teils (2412) des Lumens der Klemmnabe (2408) einklemmt und bewirkt, dass sich die eine oder mehreren Quetschrippen (2414) der Klemmnabe (2408) radial nach außen ausweitet und in das Stent-Röhrchen (216) eingreifen, um dadurch eine axiale Position der Klemmnabe (2408) in Bezug auf das Stent-Röhrchen (216) zu fixieren.

11. System nach Anspruch 7, das weiter Folgendes umfasst:
einen Stent (110) der zur Verankerung des Trägers (2500) an einem Blutgefäß, das mit dem Herzen verbunden ist, konfiguriert ist, wobei der Stent (110) ein Stent-Röhrchen (216) umfasst, das ein Lumen aufweist, das zur verschiebbaren Aufnahme des Trägers (2500) darin dimensioniert und geformt ist, und optional wobei das System weiter Folgendes umfasst:
einen ersten Führungsblock (2508), der innerhalb des Lumens des Zugdraht-Katheters (2501) angeordnet ist, wobei der erste Führungsblock (2508) mit dem Zugdraht-Katheter (2501) fest verbunden ist und ein erstes Lumen mit Gewinde umfasst;
eine erste Manschette (2514), die innerhalb des Lumens des Zugdraht-Katheters (2501) distal zu dem ersten Führungsblock (2508) verschiebbar angeordnet ist und mit dem ersten Zugdraht verbunden ist;
eine erste Schraube (2522), die Folgendes aufweist: eine erste Gewindeoberfläche, ein distales Ende, das mit der ersten Manschette (2514) verbunden ist, und ein proximales Ende, das zur lösbaren Verbindung mit einem ersten Drehmomentkabel (2526) konfiguriert ist, das zur Übertragung von Drehbewegung auf die erste Schraube (2522) konfiguriert ist, wobei zumindest ein Teil der ersten Schraube (2522) innerhalb des ersten Lumens mit Gewinde des ersten Führungsblocks (2508) angeordnet ist;
einen zweiten Führungsblock (2530), der mit dem Zugdraht-Katheter (2501) proximal zu dem ersten Führungsblock (2508) fest verbunden ist, wobei der zweite Führungsblock (2530) ein zweites Lumen mit Gewinde umfasst;
eine zweite Manschette (2532), die innerhalb des Lumens des Zugdraht-Katheters (2501) distal zu dem zweiten Führungsblock (2530) verschiebbar angeordnet ist und mit dem zweiten Zugdraht verbunden ist, wobei die zweite Manschette (2532) einen Keil (2533) umfasst, der sich radial nach außen von einer äußeren Oberfläche der zweiten Manschette (2532) erstreckt;
eine zweite Schraube (2540), die Folgendes aufweist: eine zweite Gewindeoberfläche, ein distales Ende, das mit der zweiten Manschette (2532) verbunden ist, und ein proximales Ende, das zur lösbaren Verbindung mit einem zweiten Drehmomentkabel (2542) konfiguriert ist, das zur Übertragung von Drehbewegung auf die zweite Schraube (2540) konfiguriert ist, wobei zumindest ein Teil der zweiten Schraube (2540) innerhalb des zweiten Lumens mit Gewinde des zweiten Führungsblocks (2530) angeordnet ist; und
ein Stent-Röhrchenverriegelungselement (2550), das zwischen dem Stent-Röhrchen (216) und dem Zugdraht-Katheter (2501) verschiebbar angeordnet ist, wobei das Stent-Röhrchenverriegelungselement (2550) zur Betätigung konfiguriert ist, um distal in Bezug auf den Zugdraht-Katheter (2501) bewegt zu werden und den Keil (2533) der zweiten Manschette (2532) abzudecken, sodass der Keil (2533) bewirkt, dass sich das Stent-Röhrchenverriegelungselement (2550) radial ausweitet und in das Stent-Röhrchen (216) eingreift, um dadurch eine axiale Position des Zugdraht-Katheters (2501) in Bezug auf das Stent-Röhrchen (216) zu fixieren.

12. System nach Anspruch 11, wobei die zweite Manschette (2532) ein Lumen umfasst, das zumindest teilweise durch den Keil (2533) definiert ist, wobei das Lumen zur Aufnahme der länglichen Stange dahindurch dimensioniert und geformt ist, und wobei, wenn das Stent-Röhrchenverriegelungselement (2550) den Keil (2533) abdeckt, der Keil (2533) radial nach innen gegen die längliche Stange einfällt, um dadurch eine axiale Position der länglichen Stange in Bezug auf den Zugdraht-Katheter (2501) und das Stent-Röhrchen (216) zu fixieren,
oder optional wobei das proximale Ende der ersten Schraube (2522) eine Verbindung (2524) der ersten Schraube umfasst, die zur lösbaren Verriegelung mit einer Verbindung des ersten Drehmomentkabels (2526) an einem distalen Ende des ersten Drehmomentkabels (2526) konfiguriert ist, wobei die Verbindung (2524) der ersten Schraube und die Verbindung (2528) des ersten Drehmomentkabels zur bleibenden Verbindung über einen Stab (2600) konfiguriert sind, der durch die Verbindung (2524) der ersten Schraube und die Verbindung (2528) des ersten Drehmomentkabels angeordnet ist, wenn die Verbindung (2524) der ersten Schraube und die Verbindung (2528) des ersten Drehmomentkabels verriegelt sind, und wobei der Stab (2600) zur Entfernung aus zumindest der Verbindung (2524) der ersten Schraube konfiguriert ist, um ein Lösen der Verbindung (2524) der ersten Schraube und der Verbindung (2528) des ersten Drehmomentkabels zu erlauben,
oder optional wobei das proximale Ende der zweiten Schraube (2540) eine Verbindung der zweiten Schraube umfasst, die zur lösbaren Verriegelung mit einer Verbindung des zweiten Drehmomentkabels an einem distalen Ende des zweiten Drehmomentkabels (2542) konfiguriert ist, wobei die Verbindung der zweiten Schraube und die Verbindung des zweiten Drehmomentkabels zur bleibenden Verbindung über einen Stab (2600) konfiguriert sind, der durch die Verbindung der zweiten Schraube und die Verbindung des zweiten Drehmomentkabels angeordnet ist, wenn die Verbindung der zweiten Schraube und die Verbindung des zweiten Drehmomentkabels verriegelt sind, und wobei der Stab (2600) zur Entfernung aus zumindest der Verbindung der zweiten Schraube konfiguriert ist, um ein Lösen der Verbindung der zweiten Schraube und der Verbindung des zweiten Drehmomentkabels zu erlauben.

13. System nach Anspruch 2, wobei die längliche Stange (220) eine variierende Flexibilität entlang ihrer Länge umfasst.

14. System nach Anspruch 13, wobei zumindest ein Teil der länglichen Stange (220) eine erste Querschnittsfläche umfasst und wobei zumindest ein Teil der ersten vorgeformten Biegung (222) eine zweite Querschnittsfläche umfasst, die kleiner ist als die erste Querschnittsfläche, wodurch die Flexibilität an der ersten vorgeformten Biegung (222) erhöht ist,
oder optional wobei die längliche Stange (220) eine zweite vorgeformte Biegung (224) umfasst, die an der zweiten einstellbaren Biegung (204) angeordnet ist, wobei zumindest ein Teil der länglichen Stange (220) eine erste Querschnittsfläche umfasst und wobei zumindest ein Teil der zweiten vorgeformten Biegung (224) eine zweite Querschnittsfläche umfasst, die kleiner ist als die erste Querschnittsfläche, wodurch die Flexibilität an der zweiten vorgeformten Biegung (224) erhöht ist,
oder optional wobei zumindest ein Teil der länglichen Stange (220) eine erste Querschnittsfläche umfasst und wobei zumindest ein Teil der länglichen Stange (220) distal zu der ersten vorgeformten Biegung (222) eine zweite Querschnittsfläche umfasst, die kleiner ist als die erste Querschnittsfläche, wodurch die Flexibilität an dem Teil der länglichen Stange (220) distal zu der ersten vorgeformten Biegung (222) erhöht ist.

15. System nach Anspruch 13, wobei zumindest ein Teil der länglichen Stange (220) eine kreisförmige Querschnittsfläche umfasst, die eine erste Flexibilität aufweist, und wobei zumindest ein anderer Teil der länglichen Stange (220) eine nicht-kreisförmige Querschnittsfläche umfasst, die eine zweite Flexibilität aufweist, die flexibler ist als die erste Flexibilität,
und optional wobei die erste einstellbare Biegung (202) den zumindest einen anderen Teil der länglichen Stange (220) umfasst, der die nicht-kreisförmige Querschnittsfläche umfasst, sodass die Starrheit der länglichen Stange (220) in einer Ebene senkrecht zu der ersten Ebene erhalten bleibt, während die Flexibilität der länglichen Stange (220) entlang der ersten Ebene erhöht ist,
und optional wobei die zweite einstellbare Biegung (204) den zumindest einen anderen Teil der länglichen Stange (220) umfasst, der die nicht-kreisförmige Querschnittsfläche umfasst, sodass die Starrheit der länglichen Stange (220) in einer Ebene senkrecht zu der zweiten Ebene erhalten bleibt, während die Flexibilität der länglichen Stange (220) entlang der zweiten Ebene erhöht ist.

16. System nach Anspruch 1, wobei eine Ausrichtung der ersten Ebene von einem Winkel der zweiten einstellbaren Biegung (204) innerhalb der zweiten Ebene abhängt.

17. System nach Anspruch 1, das weiter einen Stent (110) umfasst, der zur Verankerung des Trägers (200) an einem Blutgefäß, das mit dem Herzen verbunden ist, konfiguriert ist.

18. System nach Anspruch 17, wobei der Stent (110) ein Stent-Röhrchen (1622) umfasst, das ein Lumen aufweist, das zur verschiebbaren Aufnahme des Trägers (200) darin dimensioniert und geformt ist, wobei das Stent-Röhrchen (1622) einen oder mehrere flexible Flügel (1624) aufweist, die zum Übergang zwischen einem radial zusammengeklappten Zustand und einem radial expandierten Zustand konfiguriert sind, wobei der eine oder die mehreren flexiblen Flügel (1624) zu dem radial zusammengeklappten Zustand vorgespannt sind, wobei das System weiter Folgendes umfasst:
einen Trennkatheter (1628), der einen Vorsprung (1630) aufweist, der zur Betätigung zur folgenden Bewegung konfiguriert ist: von einer ersten Position, wo der Vorsprung (1630) in den einen oder die mehreren flexiblen Flügel (1624) in dem radial expandierten Zustand eingreift und diesen erhält, und einer zweiten Position, wo der Vorsprung (1630) den Eingriff in den einen oder die mehreren flexiblen Flügel (1624) löst, um einen Übergang des einen oder der mehreren flexiblen Flügel (1624) zu dem radial zusammengeklappten Zustand zu erlauben, um den Träger (200) einzuklemmen.

19. System nach Anspruch 18, wobei das Stent-Röhrchen (1622) weiter einen Halter (1632) umfasst, der zur Erhaltung des Vorsprungs (1630) in der ersten Position konfiguriert ist,
oder optional wobei ein Teil des einen oder der mehreren flexiblen Flügel (1624), die mit dem Träger (200) in dem radial zusammengeklappten Zustand im Kontakt sind, Zähne umfasst, die zur Erhöhung von Reibung zwischen dem einen oder den mehreren Flügeln (1624) und dem Träger (200) konfiguriert sind,
oder optional wobei sich eine Basis des einen oder der mehreren flexiblen Flügel (1624) von dem Stent-Röhrchen (1622) über einen Klappteil (1626) erstreckt,
oder optional wobei der Trennkatheter (1628) ein Lumen umfasst, das zur verschiebbaren Aufnahme von zumindest einem Teil des Stent-Röhrchens (200) darin dimensioniert und geformt ist.

20. System nach Anspruch 1, wobei der Träger (200) weiter einen Körperträgerkatheter (212) umfasst, der Folgendes aufweist: ein distales Ende, das mit der Prothesevorrichtung (120) verbunden ist, ein proximales Ende, das mit dem Griff (900) funktionsfähig verbunden ist, und ein Lumen, das zur verschiebbaren Aufnahme der länglichen Stange (206) konfiguriert ist, wobei der Körperträgerkatheter (212) zur Betätigung über den Griff (900) zur Bewegung in Bezug auf die längliche Stange (206) konfiguriert ist, um eine axiale Position der Prothesevorrichtung (120) in Bezug auf den Träger (200) einzustellen.

## Revendications

1. Système destiné à implanter un dispositif de valve cardiaque thérapeutique (100) au niveau d'une valve cardiaque native du cœur d'un patient, le système comprenant :
un dispositif prothétique (120) configuré pour être implanté au niveau de la valve cardiaque native ;
un support (200) configuré pour maintenir le dispositif prothétique (120) au niveau de la valve cardiaque native, le support (200) comprenant :
un premier coude ajustable (202) configuré pour être actionné afin d'ajuster une position du dispositif prothétique (120) le long d'un premier plan ;
un deuxième coude ajustable (204) proximal par rapport au premier coude ajustable (202), le deuxième coude ajustable (204) configuré pour être actionné afin d'ajuster la position du dispositif prothétique (120) le long d'un deuxième plan ; et
un rail allongé (206) configuré pour être couplé au dispositif prothétique (120) ; et
une poignée (900) couplée de manière fonctionnelle au support (200) et comprenant un ou plusieurs actionneurs (910, 912, 914, 916, 918, 920, 922), les un ou plusieurs actionneurs (912) configurés pour être actionnés pour actionner indépendamment le premier coude ajustable (202) et le deuxième coude ajustable (204).

2. Système selon la revendication 1, dans lequel le rail allongé (206) comprend un premier coude préformé disposé au niveau du premier coude ajustable (202).

3. Système selon la revendication 2, dans lequel le rail allongé (206) comprend une première flexibilité, et dans lequel le support (200) comprend en outre un premier cathéter de mise en forme (208) disposé de manière coulissante sur le rail allongé (206) de sorte que le mouvement du premier cathéter de mise en forme (208) sur le premier coude préformé du rail allongé (206) ajuste un angle du premier coude ajustable (202), le premier cathéter de mise en forme (208) ayant une deuxième flexibilité moins flexible que la première flexibilité.

4. Système selon la revendication 3, dans lequel le premier cathéter de mise en forme (208) comprend une partie distale ayant la deuxième flexibilité, et une partie proximale ayant une troisième flexibilité de sorte que le mouvement de la partie proximale du premier cathéter de mise en forme (208) sur le deuxième coude ajustable (204) n'ajuste pas un angle du deuxième coude ajustable (204),
ou facultativement dans lequel le rail allongé (206) comprend un deuxième coude préformé disposé au niveau du deuxième coude ajustable (204),
et facultativement dans lequel le support (200) comprend en outre un deuxième cathéter de mise en forme (210) disposé de manière coulissante sur le premier cathéter de mise en forme (208) de sorte que le mouvement du deuxième cathéter de mise en forme (210) sur le deuxième coude préformé du rail allongé (206) ajuste un angle du deuxième coude ajustable (204), le deuxième cathéter de mise en forme (210) ayant une troisième flexibilité moins flexible que la deuxième flexibilité.

5. Système selon la revendication 3, dans lequel le support (230) comprend en outre :
un cathéter de fil de traction (231) comprenant une partie médiane flexible (235) disposée sur le deuxième coude ajustable (204), une partie distale rigide (233) distale par rapport à la partie médiane flexible (235), une partie proximale rigide (237) proximale par rapport à la partie médiane flexible (235), et une lumière dimensionnée et formée pour recevoir de manière coulissante le premier cathéter de mise en forme (208) dans celle-ci ; et
un fil de traction (236) couplé à la partie distale rigide (233) et s'étendant le long de la partie médiane flexible (235) et de la partie proximale rigide (237), le fil de traction (236) configuré pour être actionné pour ajuster un angle de la partie distale rigide (233) par rapport à la partie proximale rigide (237) pour ajuster ainsi un angle du deuxième coude ajustable (204),
et facultativement dans lequel une extrémité proximale du fil de traction (236) est couplée de manière fonctionnelle à la poignée (900), de sorte que les un ou plusieurs actionneurs sont configurés pour être actionnés pour actionner le fil de traction (236), et pour déconnecter une partie d'implantation distale du fil de traction (236) d'une partie amovible proximale du fil de traction (236).

6. Système selon la revendication 1, dans lequel le support (230) comprend en outre :
un cathéter de fil de traction (231) comprenant une partie médiane flexible (235) disposée au niveau du deuxième coude ajustable (234), une partie distale rigide (233) distale par rapport à la partie médiane flexible (235), une partie proximale rigide (237) proximale par rapport à la partie médiane flexible (235), et une lumière dimensionnée et formée pour recevoir de manière coulissante le rail allongé dans celle-ci ; et
un premier fil de traction (236) couplé à la partie distale rigide (233) et s'étendant le long de la partie médiane flexible (235) et la partie proximale rigide (237), le premier fil de traction (236) configuré pour être actionné pour ajuster un angle de la partie distale rigide (233) par rapport à la partie proximale rigide (237) pour ajuster ainsi un angle du deuxième coude ajustable (234), et facultativement dans lequel le support (230) comprend en outre :
un deuxième cathéter de fil de traction comprenant une deuxième partie médiane flexible disposée au niveau du premier coude ajustable (232), une deuxième partie distale rigide distale par rapport à la deuxième partie médiane flexible, une deuxième partie proximale rigide proximale par rapport à la deuxième partie médiane flexible, et une lumière dimensionnée et formée pour recevoir le cathéter de fil de traction (231) dans celle-ci ; et
un deuxième fil de traction couplé à la deuxième partie distale rigide et s'étendant le long de la deuxième partie médiane flexible et de la deuxième partie proximale rigide, le deuxième fil de traction configuré pour être actionné pour ajuster un angle de la deuxième partie distale rigide par rapport à la deuxième partie proximale rigide pour ajuster ainsi un angle du premier coude ajustable (232).

7. Système selon la revendication 1, dans lequel le support (2200) comprend en outre :
un cathéter de fil de traction (2201) comprenant une partie distale flexible (2205) disposée au niveau du premier coude ajustable (2202), une partie distale rigide (2203) distale par rapport à la partie distale flexible (2205), une partie médiane rigide (2207) proximale par rapport à la partie distale flexible (2205), une partie proximale flexible (2209) disposée au niveau du deuxième coude ajustable (2204) proximale par rapport à la partie médiane rigide (2207), une partie proximale rigide (2211) proximale par rapport à la partie proximale flexible (2209), et une lumière dimensionnée et formée pour recevoir de manière coulissante le rail allongé à l'intérieur de celle-ci ;
un premier fil de traction (2206) couplé à la partie distale rigide (2203) et s'étendant le long de la partie distale flexible (2205) et la partie médiane rigide (2207), le premier fil de traction (2206) configuré pour être actionné pour ajuster un angle de la partie distale rigide (2203) par rapport à la partie médiane rigide (2207) pour ajuster ainsi un angle du premier coude ajustable (2202) ; et
un deuxième fil de traction (2210) couplé à la partie médiane rigide (2207) et s'étendant le long de la partie proximale flexible (2209) et la partie proximale rigide (2211), le deuxième fil de traction (2210) configuré pour être actionné pour ajuster un angle de la partie médiane rigide (2207) par rapport à la partie proximale rigide (2211) pour ajuster ainsi un angle du deuxième coude ajustable (2204),
et facultativement dans lequel le rail allongé est sollicité vers une configuration droite, linéaire, le rail allongé présentant une raideur suffisante pour amener le rail allongé à se redresser lors de la libération de la tension par au moins l'un des premier ou deuxième fils de traction (2206, 2210).

8. Système selon la revendication 7, dans lequel le cathéter de fil de traction (2201) comprend en outre :
un premier tube de guidage (2214) s'étendant le long du cathéter de fil de traction (2201) et ayant une extrémité distale adjacente à la partie distale rigide (2203) du cathéter de fil de traction (2201), le premier tube de guidage (2214) ayant une lumière configurée pour recevoir le premier fil de traction (2206) à travers celui-ci, de sorte qu'une extrémité distale du premier fil de traction (2206) s'étend au-delà de l'extrémité distale du premier tube de guidage (2214) et est couplée à la partie distale rigide (2203) ; et
un deuxième tube de guidage (2216) s'étendant le long du cathéter de fil de traction (2201) et ayant une extrémité distale adjacente à la partie médiane rigide (2207) du cathéter de fil de traction (2201), le deuxième tube de guidage (2216) ayant une lumière configurée pour recevoir le deuxième fil de traction (2210) à travers celle-ci, de sorte qu'une extrémité distale du deuxième fil de traction (2210) s'étend au-delà de l'extrémité distale du deuxième tube de guidage (2216) et est couplée à la partie médiane rigide (2207),
et facultativement, dans lequel le premier tube de guidage (2214) est interrompu ou flexible au niveau de la partie distale flexible (2205) et la partie proximale flexible (2209), et dans lequel le deuxième tube de guidage (2216) est interrompu ou flexible au niveau de la partie proximale flexible (2209).

9. Système selon la revendication 7, dans lequel le cathéter de fil de traction (2301) comprend en outre un arbre de guidage (2330) disposé de manière fixe au sein de la lumière du cathéter de fil de traction (2301), l'arbre de guidage (2330) comprenant un premier canal (2314) dimensionné et formé pour recevoir de manière coulissante le premier fil de traction (2306) à travers celui-ci, une région proximale du premier canal (2314) comprenant une première pluralité d'encoches (2315), et un deuxième canal (2316) dimensionné et formé pour recevoir de manière coulissante le deuxième fil de traction (2310) à travers celui-ci, une région proximale du deuxième canal (2316) comprenant une deuxième pluralité d'encoches,
dans lequel une région proximale du premier fil de traction (2306) comprend une ou plusieurs premières bosses (2308) configurées pour entrer en prise avec une ou plusieurs encoches de la première pluralité d'encoches (2315) du premier canal (2314), et
dans lequel une région proximale du deuxième fil de traction (2310) comprend une ou plusieurs deuxièmes bosses (2312) configurées pour entrer en prise avec une ou plusieurs encoches de la deuxième pluralité d'encoches du deuxième canal (2316),
et facultativement dans lequel une région proximale du rail allongé (2320) comprend une pluralité d'encoches de rail (2322) configurées pour recevoir au moins une partie des une ou plusieurs premières bosses (2308) du premier fil de traction (2306) et au moins une partie des une ou plusieurs deuxièmes bosses (2312) du deuxième fil de traction (2310) lorsque les une ou plusieurs premières bosses (2308) sont entrées en prise avec les une ou plusieurs encoches de la première pluralité d'encoches (2315) et les une ou plusieurs deuxièmes bosses (2312) sont entrées en prise avec les une ou plusieurs encoches de la deuxième pluralité d'encoches, et
facultativement dans lequel le système comprend en outre un dispositif de verrouillage de fil de traction (2340) configuré pour être actionné pour être déplacé de manière axiale jusqu'à ce que le dispositif de verrouillage de fil de traction (2340) soit disposé sur les régions proximales des premier et deuxième fils de traction (2306, 2310) pour maintenir ainsi une position axiale relative entre les premier et deuxième fils de traction (2306, 2310), le rail allongé (2320), le cathéter de fil de traction (2301), et l'arbre de guidage (2330) par ajustement serré.

10. Système selon la revendication 7, comprenant en outre une endoprothèse (110) configurée pour ancrer le support (2400) à un vaisseau sanguin couplé au cœur, l'endoprothèse (110) comprenant un tube d'endoprothèse (216) ayant une lumière dimensionnée et formée pour recevoir de manière coulissante le support (200) dans celle-ci, et dans lequel facultativement le système comprend en outre :
un moyeu de serrage (2408) couplé à une partie proximale du cathéter de fil de traction (2401) et disposé de manière coulissante dans la lumière du tube d'endoprothèse (216), le moyeu de serrage (2408) ayant une lumière comprenant une partie distale effilée (2410) et une partie proximale effilée (2412), le moyeu de serrage (2408) comprenant une ou plusieurs nervures d'écrasement (2414) s'étendant de manière radiale vers l'extérieur à partir d'une surface extérieure du moyeu de serrage (2408) ;
une enclume (2416) disposée de manière coulissante au sein de la lumière du tube d'endoprothèse (216) proximale par rapport au moyeu de serrage (2408), l'enclume (2416) comprenant une partie distale effilée (2418) configurée pour être au moins partiellement reçue par la partie proximale effilée (2412) de la lumière du moyeu de serrage (2408) ;
un collet (2402) disposé de manière coulissante au sein de la lumière du moyeu de serrage (2408), le collet (2402) ayant une lumière dimensionnée et formée pour recevoir de manière coulissante le rail allongé à travers celle-ci, le collet (2402) comprenant une partie distale effilée (2404) configurée pour être au moins partiellement reçue par la partie distale effilée (2410) de la lumière du moyeu de serrage (2408) ;
un poussoir de collet (2422) disposé au sein d'une lumière de l'enclume (2416) et au moins une partie de la lumière du moyeu de serrage (2408) proximale par rapport au collet (2402), le poussoir de collet (2422) configuré pour être actionné pour déplacer le collet (2402) de manière distale par rapport au moyeu de serrage (2408) ; et
un poussoir d'enclume (2424) disposé de manière coulissante au sein de la lumière du tube d'endoprothèse (216) proximal par rapport à l'enclume (2416), le poussoir d'enclume (2424) configuré pour être actionné pour déplacer l'enclume (2416) de manière distale par rapport au moyeu de serrage (2408),
dans lequel les premier et deuxième fils de traction (2406) s'étendent entre une surface externe de la partie distale effilée (2418) de l'enclume (2416) et une surface interne de la partie proximale effilée (2412) de la lumière du moyeu de serrage (2408),
dans lequel, lors d'un mouvement distal du collet (2402) par rapport au moyeu de serrage (2408), la partie distale effilée (2410) de la lumière du moyeu de serrage (2408) amène la partie distale effilée (2404) du collet (2402) à serrer le rail allongé (206) et à fixer une position axiale du rail allongé par rapport au moyeu de serrage (2408), et
dans lequel, lors d'un mouvement distal de l'enclume (2416) par rapport au moyeu de serrage (2408), la partie distale effilée (2418) de l'enclume (2416) serre les premier et deuxième fils de traction (2406) entre la surface externe de la partie distale effilée (2418) de l'enclume (2416) et la surface interne de la partie proximale effilée (2412) de la lumière du moyeu de serrage (2408), et amène les une ou plusieurs nervures d'écrasement (2414) du moyeu de serrage (2408) à se déployer de manière radiale vers l'extérieur et à entrer en prise avec le tube d'endoprothèse (216) pour fixer ainsi une position axiale du moyeu de serrage (2408) par rapport au tube d'endoprothèse (216).

11. Système selon la revendication 7, comprenant en outre :
une endoprothèse (110) configurée pour ancrer le support (2500) à un vaisseau sanguin couplé au cœur, l'endoprothèse (110) comprenant un tube d'endoprothèse (216) ayant une lumière dimensionnée et formée pour recevoir de manière coulissante le support (2500) dans celle-ci, et facultativement, dans lequel le système comprend en outre :
un premier bloc de guidage (2508) disposé au sein de la lumière du cathéter de fil de traction (2501), le premier bloc de guidage (2508) couplé de manière fixe au cathéter de fil de traction (2501) et comprenant une première lumière filetée ;
un premier collier (2514) disposé de manière coulissante au sein de la lumière du cathéter de fil de traction (2501) distal par rapport au premier bloc de guidage (2508) et couplé au premier fil de traction ;
une première vis (2522) ayant une première surface filetée, une extrémité distale couplée au premier collier (2514), et une extrémité proximale configurée pour être couplée de manière libérable à un premier câble de couple (2526) configuré pour transmettre un mouvement rotatif à la première vis (2522), au moins une partie de la première vis (2522) disposée au sein de la première lumière filetée du premier bloc de guidage (2508) ;
un deuxième bloc de guidage (2530) couplé de manière fixe au cathéter de fil de traction (2501) proximal par rapport au premier bloc de guidage (2508), le deuxième bloc de guidage (2530) comprenant une deuxième lumière filetée ;
un deuxième collier (2532) disposé de manière coulissante au sein de la lumière du cathéter de fil de traction (2501) distal par rapport au deuxième bloc de guidage (2530) et couplé au deuxième fil de traction, le deuxième collier (2532) comprenant un coin (2533) s'étendant de manière radiale vers l'extérieur à partir d'une surface extérieure du deuxième collier (2532) ;
une deuxième vis (2540) ayant une deuxième surface filetée, une extrémité distale couplée au deuxième collier (2532), et une extrémité proximale configurée pour être couplée de manière libérable à un deuxième câble de couple (2542) configuré pour transmettre un mouvement rotatif à la deuxième vis (2540), au moins une partie de la deuxième vis (2540) disposée au sein de la deuxième lumière filetée du deuxième bloc de guidage (2530) ; et
un dispositif de verrouillage de tube d'endoprothèse (2550) disposé de manière coulissante entre le tube d'endoprothèse (216) et le cathéter de fil de traction (2501), le dispositif de verrouillage de tube d'endoprothèse (2550) configuré pour être actionné pour se déplacer de manière distale par rapport au cathéter de fil de traction (2501) et recouvrir le coin (2533) du deuxième collier (2532), de sorte que le coin (2533) amène le dispositif de verrouillage de tube d'endoprothèse (2550) à se déployer de manière radiale et à entrer en prise avec le tube d'endoprothèse (216) pour fixer ainsi une position axiale du cathéter de fil de traction (2501) par rapport au tube d'endoprothèse (216).

12. Système selon la revendication 11, dans lequel le deuxième collier (2532) comprend une lumière au moins partiellement définie par le coin (2533), la lumière dimensionnée et formée pour recevoir le rail allongé à travers celle-ci, et dans lequel, lorsque le dispositif de verrouillage de tube d'endoprothèse (2550) recouvre le coin (2533), le coin (2533) se replie de manière radiale vers l'intérieur contre le rail allongé pour fixer ainsi une position axiale du rail allongé par rapport au cathéter de fil de traction (2501) et au tube d'endoprothèse (216),
ou facultativement dans lequel l''extrémité proximale de la première vis (2522) comprend une première connexion de vis (2524) configurée pour s'interverrouiller de manière libérable avec une première connexion de câble de couple (2526) au niveau d'une extrémité distale du premier câble de couple (2526), la première connexion de vis (2524) et la première connexion de câble de couple (2528) configurées pour rester couplées via une tige (2600) disposée à travers la première connexion de vis (2524) et la première connexion de câble de couple (2528) lorsque la première connexion de vis (2524) et la première connexion de câble de couple (2528) sont interverrouillées, et dans lequel la tige (2600) est configurée pour être retirée d'au moins la première connexion de vis (2524) pour permettre le découplage de la première connexion de vis (2524) et de la première connexion de câble de couple (2528),
ou facultativement dans lequel l'extrémité proximale de la deuxième vis (2540) comprend une deuxième connexion de vis configurée pour s'interverrouiller de manière libérable avec une deuxième connexion de câble de couple au niveau d'une extrémité distale du deuxième câble de couple (2542), la deuxième connexion de vis et la deuxième connexion de câble de couple configurées pour rester couplées via une tige (2600) disposée à travers la deuxième connexion de vis et la deuxième connexion de câble de couple lorsque la deuxième connexion de vis et la deuxième connexion de câble de couple sont interverrouillées, et dans lequel la tige (2600) est configurée pour être retirée d'au moins la deuxième connexion de vis afin de permettre le découplage de la deuxième connexion de vis et de la deuxième connexion de câble de couple.

13. Système selon la revendication 2, dans lequel le rail allongé (220) comprend une flexibilité variable le long de sa longueur.

14. Système selon la revendication 13, dans lequel au moins une partie du rail allongé (220) comprend une première aire de section transversale, et dans lequel au moins une partie du premier coude préformé (222) comprend une deuxième aire de section transversale plus petite que la première aire de section transversale, augmentant ainsi la flexibilité au niveau du premier coude préformé (222),
ou facultativement dans lequel le rail allongé (220) comprend un deuxième coude préformé (224) disposé au niveau du deuxième coude ajustable (204), dans lequel au moins une partie du rail allongé (220) comprend une première aire de section transversale, et dans lequel au moins une partie du deuxième coude préformé (224) comprend une deuxième aire de section transversale plus petite que la première aire de section transversale, augmentant ainsi la flexibilité au niveau du deuxième coude préformé (224),
ou facultativement dans lequel au moins une partie du rail allongé (220) comprend une première aire de section transversale, et dans lequel au moins une partie du rail allongé (220) distale par rapport au premier coude préformé (222) comprend une deuxième aire de section transversale plus petite que la première aire de section transversale, augmentant ainsi la flexibilité au niveau de la partie du rail allongé (220) distale par rapport au premier coude préformé (222).

15. Système selon la revendication 13, dans lequel au moins une partie du rail allongé (220) comprend une aire de section transversale circulaire ayant une première flexibilité, et dans lequel au moins une autre partie du rail allongé (220) comprend une aire de section transversale non circulaire ayant une deuxième flexibilité plus flexible que la première flexibilité,
et facultativement dans lequel le premier coude ajustable (202) comprend l'au moins une autre partie du rail allongé (220) comprenant l'aire de section transversale non circulaire, de sorte que la rigidité du rail allongé (220) est maintenue dans un plan perpendiculaire au premier plan, tandis que la flexibilité du rail allongé (220) est augmentée le long du premier plan,
ou facultativement dans lequel le deuxième coude ajustable (204) comprend l'au moins une autre partie du rail allongé (220) comprenant l'aire de section transversale non circulaire, de sorte que la rigidité du rail allongé (220) est maintenue dans un plan perpendiculaire au deuxième plan, tandis que la flexibilité du rail allongé (220) est augmentée le long du deuxième plan.

16. Système selon la revendication 1, dans lequel une orientation du premier plan est dépendante d'un angle du deuxième coude ajustable (204) au sein du deuxième plan.

17. Système selon la revendication 1, comprenant en outre une endoprothèse (110) configurée pour ancrer le support (200) à un vaisseau sanguin couplé au cœur.

18. Système selon la revendication 17, dans lequel l'endoprothèse (110) comprend un tube d'endoprothèse (1622) ayant une lumière dimensionnée et formée pour recevoir de manière coulissante le support (200) dans celle-ci, le tube d'endoprothèse (1622) ayant une ou plusieurs feuilles flexibles (1624) configurées pour effectuer une transition entre un état replié de manière radiale à un état déployé de manière radiale, les une ou plusieurs feuilles flexibles (1624) sollicitées vers l'état replié de manière radiale, le système comprenant en outre :
un cathéter de déconnexion (1628) ayant une languette (1630) configurée pour être actionnée de manière à se déplacer d'une première position où la languette (1630) entre en prise avec les une ou plusieurs feuilles flexibles (1624) et maintient celles-ci dans l'état déployé de manière radiale et d'une deuxième position où la languette (1630) se désengage des une ou plusieurs feuilles flexibles (1624) pour permettre aux une ou plusieurs feuilles flexibles (1624) d'effectuer une transition vers l'état replié de manière radiale pour serrer le support (200).

19. Système selon la revendication 18, dans lequel le tube d'endoprothèse (1622) comprend en outre un élément de retenue (1632) configuré pour maintenir la languette (1630) dans la première position,
ou facultativement dans lequel une partie des une ou plusieurs feuilles flexibles (1624) qui entrent en contact avec le support (200) dans l'état replié de manière radiale comprend des dents configurées pour augmenter le frottement entre les une ou plusieurs feuilles flexibles (1624) et le support (200),
ou facultativement dans lequel une base des une ou plusieurs feuilles flexibles (1624) s'étend depuis le tube d'endoprothèse (1622) via une partie articulée (1626),
ou facultativement dans lequel le cathéter de déconnexion (1628) comprend une lumière dimensionnée et formée pour recevoir de manière coulissante au moins une partie du tube d'endoprothèse (200) dans celle-ci.

20. Système selon la revendication 1, dans lequel le support (200) comprend en outre un cathéter de support corporel (212) ayant une extrémité distale couplée au dispositif prothétique (120), une extrémité proximale couplée de manière fonctionnelle à la poignée (900), et une lumière configurée pour recevoir de manière coulissante le rail allongé (206), le cathéter de support corporel (212) configuré pour être actionné via la poignée (900) pour se déplacer par rapport au rail allongé (206) afin d'ajuster une position axiale du dispositif prothétique (120) par rapport au support (200).
